# EUROPEAN PATENT APPLICATION

(11) **EP 2 198 863 A1**
(43) Date of publication of application: **23.06.2010**
(21) Application number: 10003362.0
(22) Date of filing: 27.02.2007
(51) Int. Cl.: A61K 31/38, A61P 35/00

(54) **Cancer treatment with gamma-secretase inhibitors**

(30) Priority: 27.02.2006 US 777110 P; 27.03.2006 US 786312 P
(62) Divisional of application: 07752086.4
(71) Applicant: The Johns Hopkins University, Baltimore, MD 21218 (US)
(72) Inventor: Eberhart, Charles, Baltimore, MD 21093 (US); Fan, Xing, Lutherville, MD 02193 (US); Maitra, Anirban, MD, 20895 (US)
(74) Representative: Adam, Holger

(57) **Abstract**

Provided are methods for treating cancer in a patient, comprising administering to a patient in need thereof a therapeutically effective regimen, the regimen comprising administering a gamma-secretase inhibitor, wherein the regimen results in a reduction in the cancer cell population in the patient. In some embodiments of the methods, the therapeutically effective regimen stabilizes, reduces or eliminates the cancer stem cell population. Also provided are compounds of the formula (I) or a pharmaceutically acceptable salt thereof, wherein R¹, R², and X are as herein described.

## Description

### 1. CROSS-REFERENCES TO RELATED APRLICATIONS

This application claims the benefit of U.S. Provisional Application No. 60/777,110, filed February 27, 2006; and U.S. Provisional Application No. 60/786,312, filed March 27, 2006; the disclosures both of which are hereby incorporated by reference in their entireties.

### 2. FIELD OF THE INVENTION

The present invention generally relates to methods for preventing, treating, and/or managing cancer, comprising administration of a prophylactically or therapeutically effective regimen of gamma-secretase inhibitors. In some embodiments of the methods, the prophylactically or therapeutically effective regimen stabilizes, reduces or eliminates the cancer stem cell population that produces progeny cells which may form a tumor and/or metastasize. In some embodiments of the methods, the therapeutically effective regimen stabilizes, reduces or eliminates the cancer cell population which may form a tumor and/or metastasize: The prophylactically or therapeutically effective regimen of the invention in some embodiments, includes monitoring the cancer stem cell population in a patient receiving gamma-secretase inhibitors.

### 3. BACKGROUND OF THE INVENTION

### 3.1. CANCER THERAPY

Cancer is one of the most significant chronic conditions. The American Cancer Society's *Cancer Facts and Figures, 2003,* predicts over 1.3 million Americans will receive a cancer diagnosis this year. In the United States, cancer is second only to heart disease in mortality accounting for one of four deaths. In 2002, the National Institutes of Health estimated total costs of cancer totalled $171.6 billion, with $61 billion in direct expenditures. Incidence of cancer is widely expected to increase as the U.S. population ages, further augmenting the impact of this condition. The current treatment regimens for cancer essentially established in the 1970s and 1980s, have not changed dramatically. These treatments, which include chemotherapy, radiation and other modalities, have limited utility in most advanced stage cancers since, among other things, they are relatively non-specific, affecting processes in both normal and cancer cells.

Conventional cancer diagnosis and therapies to date have attempted to selectively detect and eradicate neoplastic cells that are largely fast-growing (*i*.*e*., cells that form the tumor bulk). Standard oncology regimens have often been largely designed to administer the highest dose of irradiation or a compound without undue toxicity, *i.e.,* often referred to as the "maximum tolerated dose" (MTD) or "no observed adverse effect level" (NOAEL). Many conventional cancer chemotherapies (*e*.*g.,* alkylating agents such as cyclophosphamide, antimetabolites such as 5-Fluorouracil, plant alkaloids such as vincristine) and conventional irradiation therapies often exert their toxic effects on cancer cells largely by interfering with numerous cellular mechanisms involved in cell growth and DNA replication. Chemotherapy protocols also often involve administration of a combination of chemotherapeutic agents to increase the efficacy of treatment. Despite the availability of a variety of chemotherapeutic agents, these therapies have many drawbacks (*see, e.g.,* Stockdale, 1998, "Principles Of Cancer Patient Management" in Scientific American Medicine, vol. 3, Rubenstein and Federman, eds., ch. 12, sect. X). For example, almost all chemotherapeutic agents are toxic; chemotherapy also causes significant, and often dangerous, side effects, including severe nausea, bone marrow depression, immunosuppression, *etc*.

Other types of traditional cancer therapies include surgery, hormonal therapy, immunotherapy, anti-angiogenesis therapy, targeted therapy (*e*.*g*., therapy directed to a cancer target such as Gleevec® and other tyrosine kinase inhibitors, Velcade®, Sutent®), and radiation treatment to eradicate neoplastic cells in a patient (see, *e.g.,* Stockdale, 1998, "Principles of Cancer Patient Management," in Scientific American: Medicine, vol. 3, Rubenstein and Federman, eds., ch. 12, sect. IV). All of these approaches can pose significant drawbacks for the patient including a lack of efficacy (in terms of long term outcome) and toxicity. Accordingly, new therapies for improving the long term prospect of cancer patients are needed.

### 3.2. CANCER STEM CELL THERAPY

Cancer stem cells comprise a unique subpopulation (often 0.1-10% or so) of a tumor that, in contrast to the remaining 90% or so of the tumor (i. e., the tumor bulk), are relatively more tumorigenic and resistant to traditional therapy (due, in part, to their relatively more slow-growing or quiescent nature). Given that conventional therapies and regimens have, in large part, been designed to attack rapidly proliferating cells (*i*.*e*., those cancer cells that comprise the tumor bulk), slower growing cancer stem cells may be relatively more resistant than faster growing tumor bulk to conventional therapies and regimens. This would explain another reason for the failure of standard oncology treatment regimens to ensure long-term benefit in most patients with advanced stage cancers. In a specific embodiment, a cancer stem cell(s) is the founder cell of a tumor (*i*.*e*., it is the progenitor of cancer cells). In some embodiments, a cancer stem cell(s) has one or more or all of the following characteristics or properties: (i) the ability to initiate a tumor and to perpetuate tumor growth, (ii) is generally less mutated than the bulk of a tumor, (iii) has many features of a normal stem cell(s) (*e*.*g*., similar cell surface antigen expression profile, self-renewal programs, multi-drug resistance, effective methods to prevent and/or manage DNA damage (e.g. via effective repair mechanisms), an immature phenotype, etc. characteristic of normal stem cells) and may be derived from a normal stem cell(s), (iv) is potentially responsive to its microenvironment (*e*.*g*., the cancer stem cells may be capable of being induced to differentiate and/or divide asymmetrically), and (v) is the source of metastases.

Cancer stem cells have been identified in a large variety of cancer types. For instance, Bonnet *et al.* using flow cytometry were able to isolate the leukemia cells bearing the specific cell surface markers, CD34⁺ CD38⁻, and subsequently prove that it is only these cells (comprising <1% of a given leukemia), unlike the remaining 99+% of the leukemia bulk, that are uniquely able to fully and repeatedly recapitulate the entire leukemia from which it was derived. See, *e*.*g*., "Human acute myeloid leukemia is organized as a hierarchy that originates from a primitive hematopoietic cell," Nat Med 3:730-737 (1997). That is, these cancer stem cells were found as <1 in 10,000 leukemia cells yet enough to transfer and serially transfer the human leukemia to severe combined immunodeficiency/-non-obese diabetic (NOD/SCID) mice with the same histologic phenotype as in the original tumor.

Cox *et al.* identified small subfractions of human acute lymphoblastic leukemia (ALL) cells which had the phenotypes CD34⁺/CD10⁻ and CD34⁺/CD19⁻, and were capable of engrafting ALL tumors in immunocompromised mice ― *i.e.,* the cancer stem cells. In contrast, no engraftment of the mice was observed using the ALL bulk, despite, in some cases, injecting 10-fold more cells. See Cox et al., "Characterization of acute lymphoblastic leukemia progenitor cells," Blood 104(19): 2919-2925 (2004).

Multiple myeloma was found to contain small subpopulations of cells that were CD 138⁻ and, relative to the large bulk population of CD138⁺ myeloma cells had greater clonogenic and tumorigenic potential. Matsui et al., "Characterization of clonogenic multiple myeloma cells," Blood 103(6): 2332. The authors concluded that the CD138⁻ subpopulation of multiple myeloma was the cancer stem cell population.

Kondo *et al.* recently isolated a small population of cells from a Co-glioma cell line, which they identified as the cancer stem cell population. Kondo et al., "Persistence of a small population of cancer stem-like cells in the C6 glioma cell line," Proc. Natl. Acad. Sci. USA 101:781-786 (2004). These cancer stem cells were able to self-renew and recapitulate gliomas in immunocompromised mice.

Breast cancers were shown to contain a small population of cells with stem cell characteristics (bearing surface markers CD44⁺CD24^{low lin-}). Al-Hajj et al., "Prospective identification of tumorigenic breast cancer cells," Proc. Natl. Acad. Sci. USA 100:3993-3988 (2003). As few as 200 of these cells, corresponding to 1-10 % of the total tumor cell population, are able to form tumors in NOD/SCID mice. In contrast, increasing the number of implanted cells to 20,000 lacking these cell surface markers (*i*.*e.,* the tumor bulk) proves unable to re-grow the tumor.

A subpopulation of cells derived from human prostate tumors was found to be enriched for a phenotype that was consistent with a prostate cancer stem cell phenotype. This subpopulation of cells was able to self-renew and to recapitulate the phenotype of the prostate tumor from which they were derived. Collins et al., "Prospective Identification of Tumorigenic Prostate Cancer Stem Cells," Cancer Res 65(23): 10946-10951 (2005).

Fang *et al.* isolated a subpopulation of cells from melanoma with cancer stem cell properties. In particular, this subpopulation of cells could differentiate and self-renew. In culture, the subpopulation formed spheres whereas the more differentiated cell fraction from the lesions were more adherent. The subpopulation containing sphere-like cells were more tumorigenic than the adherent cells when grafted into mice. Fang et al., "A Tumorigenic Subpopulation with Stem Cell Properties in Melanomas," Cancer Res 65(20): 9328-9337 (2005).

Singh *et al*. have recently identified brain tumor stem cells. When isolated and transplanted into nude mice, the CD133+ cancer stem cells form tumors that can be serially transplanted. Singh et al., "Identification of human brain tumor initiating cells," Nature 432:396-401 (2004); Singh et al., "Cancer stem cells in nervous system tumors," Oncogene 23:7267-7273 (2004); Singh et al., "Identification of a cancer stem cell in human brain tumors," Cancer Res. 63:5821-5828 (2003).

Since conventional cancer therapies target rapidly proliferating cells (*i*.*e.*, cells that form the tumor bulk) they may be relatively ineffective in targeting cancer stem cells. In fact, cancer stem cells, including the leukemia stem cells, have indeed been shown to be relatively resistant to conventional chemotherapeutic therapies (*e*.*g*., Ara-C, daunorubicin) as well as newer targeted therapies (*e*.*g*., Gleevec®). Leukemic stem cells divide rarely (*i*.*e*., are relatively slow-growing, or quiescent), express multi-drug resistance genes, employ methods to prevent and/or manage DNA damage (e.g. via effective repair mechanisms), and utilize other anti-apoptotic mechanisms. Jordan et al., "Targeting the most critical cells: approaching leukemia therapy as a problem in stem cell biology", Nat Clin Pract Oncol. 2: 224-225 (2005); Bao et al, "Glioma stem cells promote radioresistance by preferential activation of the DNA damage response", Nature 444: 756-760 (2006); Phillips et al, "The response of CD24-/low/CD44+ breast cancer-initiating cells to radiation", J Natl Cancer Inst 98: 1777-1785 (2006). Further, cancer stem cells may not only contribute to treatment failure, but may also persist in a patient after clinical remission and subsequently contribute to relapse at a later date. Behbood et al., "Will cancer stem cells provide new therapeutic targets?" Carcinogenesis 26(4): 703-711 (2004). Therefore, targeting cancer stem cells even more so than (or in combination with) targeting the rapidly proliferating tumor bulk, is expected to provide for improved long-term outcomes for cancer patients. Accordingly, new therapeutic agents and/or regimens designed to target cancer stem cells are needed to reach this goal.

### 3.3. GAMMA-SECRETASE

Gamma-secretase activity is associated with a protein complex composed of presenilins (PS1 or PS2), nicastrin (Nct), PEN-2, APH-1a, and APH-1b (Yu, G. et al., J. Biol Chem., 273, 16470-16475, 1998; Capell, A. et al., J. Biol. Chem., 273, 3205-3211, 1998). The expression of these complex components is coordinately regulated. The catalytic activity of gamma-secretase is most likely contributed by the presenilins. The presenilins are polytopic transmembrane proteins, which together with signal peptide peptidases and type-4 prepilin peptidases may belong to a novel family of aspartyl proteases.

The presenilins, therefore, are essential components of an intramembranous proteolytic activity known as gamma-secretase (Wolfe, M. S. et al., J. Biol. Chem., 276, 5413-5416,2001). Several type-I integral membrane proteins have been identified as substrates for gamma-secretase, including the Notch receptor (Notch) and APP (De Strooper, B. et al., Nature (London), 398, 518-522, 1999). Notch is a signaling molecule that regulates cell-fate determination during development (Artavanis-Tsakonas, S. et al., Science, 284, 770-776 1999). Signaling through Notch is triggered by the binding of ligands, such as Delta and Jagged, which induce cleavage of Notch by TACE (Brou, C. et al., Mol. Cell, 5, 207-216, 2000). Subsequent cleavage by gamma-secretase releases the Notch intracellular domain, which binds to transcription factors and translocates to the nucleus, where it regulates transcription of Enhancer of Split complex genes (Greenwald, I., Genes Dev., 12, 1751-1762, 1998). Notch has multiple isoforms, and as used herein, the term Notch refers collectively to each of the Notch isoforms.

### 4. SUMMARY OF THE INVENTION

The invention provides a method of preventing, treating, and/or managing cancer in a patient, the method comprising administering to a patient in need thereof a prophylactically effective regimen or a therapeutically effective regimen, the regimen comprising administering a gamma-secretase inhibitor, or a pharmaceutically acceptable salt thereof. In one embodiment, the gamma-secretase inhibitor is a compound or class of compounds of the invention.

In one aspect, the invention provides a method of preventing, treating, and/or managing cancer to a patient in need thereof, the method comprising administering a prophylactically effective regimen or a therapeutically effective regimen, the regimen comprising administering to the patient a compound of the invention, or a pharmaceutically acceptable salt thereof, wherein the patient has been diagnosed with cancer, and wherein said cancer is a hematologic cancer. In some embodiments, the patient received a therapy for the treatment and/or management of the cancer before the administration of the therapeutically effective regimen of the compound of the invention, or a pharmaceutically acceptable salt thereof. Non-limiting examples of such a therapy include chemotherapy, radioimmunotherapy, toxin therapy, prodrug-activating enzyme therapy, antibody therapy, surgical therapy, immunotherapy, radiation therapy, targeted therapy (i.e. therapy directed toward a specific target or pathway, e.g. tyrosine kinase, etc.), and any combination thereof. In some embodiments, the patient has not previously received a therapy for the treatment and/or management of the cancer. In a specific embodiment, the hematologic cancer is leukemia, lymphoma, or myeloma.

In another aspect, the invention provides a method of preventing, treating, and/or managing a solid tumor in a patient, the method comprising administering to a patient in need thereof a prophylactically effective regimen or a therapeutically effective regimen, the regimen comprising administering to the patient a compound of the invention, or a pharmaceutically acceptable salt thereof, wherein the patient has been diagnosed with a solid tumor, and wherein the patient has undergone primary therapy. In some embodiments, the primary therapy is, for example, chemotherapy, radioimmunotherapy, toxin therapy, prodrug-activating enzyme therapy, antibody therapy, surgical therapy, immunotherapy, radiation therapy, targeted therapy (i.e. therapy directed toward a specific target or pathway, e.g. tyrosine kinase, etc.), or any combination thereof.

In particular embodiments of this aspect, the solid tumor is fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, colorectal cancer, kidney cancer, pancreatic cancer, bone cancer, breast cancer, ovarian cancer, prostate cancer, esophageal cancer, stomach cancer, oral cancer, nasal cancer, throat cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, uterine cancer, testicular cancer, small cell lung carcinoma, bladder carcinoma, lung cancer, epithelial carcinoma, glioma, glioblastoma multiforme, astrocytoma, medulloblastoma, craniopharyngioma, ependymoma, pinealoma, hemangioblastoma, acoustic neuroma, oligodendroglioma, meningioma, skin cancer, melanoma, neuroblastoma, or retinoblastoma.

In another aspect, the invention provides a method of preventing, treating, and/or managing cancer, the method comprising administering to a patient in need thereof a prophylactically effective regimen or a therapeutically effective regimen, the regimen comprising administering to the patient a compound of the invention, or a pharmaceutically acceptable salt thereof, wherein the patient received another therapy. In some embodiments, the prior therapy is, for example, chemotherapy, radioimmunotherapy, toxin therapy, prodrug-activating enzyme therapy, antibody therapy, surgical therapy, immunotherapy, radiation therapy, targeted therapy (i.e. therapy directed toward a specific target or pathway, e.g. tyrosine kinase, etc.), or any combination thereof.

In another aspect, the invention provides a method of treating cancer in a patient, the method comprising administering to a patient in need thereof a prophylactically effective regimen, the regimen comprising administering to the patient a compound of the invention, or a pharmaceutically acceptable salt thereof, wherein the patient is in remission for the cancer.

In a specific aspect, the invention provides a method of preventing, treating, and/or managing cancer in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the compound is administered at a dose lower than the maximum tolerated dose (MTD) over a period of 1 day to 36 months, 1 to 24 months, 3 to 6 months, or greater than 24 months. In one embodiment, the dose of the compound of the invention is administered to the patient is from 0.1 to 50 mg/m².

In a specific aspect, the invention provides a method of preventing, treating, and/or managing cancer in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the compound is administered at a dose lower than the human equivalent dose (HED) of the no observed adverse effect level (NOAEL) over a period of 1 day to 36 months, 1 to 24 months, 3 to 6 months, or greater than 24 months. In one embodiment, a compound of the invention is administered at a dose lower than the human equivalent dose (HED) of the no observed adverse effect level (NOAEL) for a period longer than 6 months, including for the remaining lifetime of the patient. In one embodiment, the dose of the compound of the invention administered to the patient is from 0.1 to 50 mg/m².

In a specific aspect, the invention provides a method of preventing, treating, and/or managing kidney cancer in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the patient has been diagnosed with kidney cancer.

In a specific aspect, the invention provides a method of preventing, treating, and/or managing pancreatic cancer in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the patient has been diagnosed with pancreatic cancer.

In a specific aspect, the invention provides a method of preventing, treating, and/or managing bone cancer in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the patient has been diagnosed with bone cancer.

In a specific aspect, the invention provides a method of preventing, treating, and/or managing breast cancer in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the patient has been diagnosed with breast cancer.

In a specific aspect, the invention provides a method of preventing, treating, and/or managing ovarian cancer in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the patient has been diagnosed with ovarian cancer.

In a specific aspect, the invention provides a method of preventing, treating, and/or managing cervical cancer in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the patient has been diagnosed with cervical cancer.

In a specific aspect, the invention provides a method of preventing, treating, and/or managing uterine cancer in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the patient has been diagnosed with uterine cancer.

In a specific aspect, the invention provides a method of preventing, treating, and/or managing testicular cancer in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the patient has been diagnosed with testicular cancer.

In a specific aspect, the invention provides a method ofpreventing, treating, and/or managing bladder cancer in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the patient has been diagnosed with bladder cancer.

In a specific aspect, the invention provides a method of preventing, treating, and/or managing skin cancer in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the patient has been diagnosed with skin cancer.

In a specific aspect, the invention provides a method of preventing, treating, and/or managing melanoma in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the patient has been diagnosed with melanoma.

In a specific aspect, the invention provides a method of preventing, treating, and/or managing neuroblastoma in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the patient has been diagnosed with neuroblastoma.

In a specific aspect, the invention provides a method of preventing, treating, and/or managing retinoblastoma in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the patient has been diagnosed with retinoblastoma.

In a specific aspect, the invention provides a method of preventing, treating, and/or managing medulloblastoma in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the patient has been diagnosed with medulloblastoma.

In a specific aspect, the invention provides a method of preventing, treating, and/or managing a glioma in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the patient has been diagnosed with a glioma.

In a specific aspect, the invention provides a method of preventing, treating, and/or managing a brain tumor in a patient, the method comprising administering to a patient in need thereof, a prophylactically effective regimen or a therapeutically effective regimen, the regimen comprising administering to the patient a compound of the invention, or a pharmaceutically acceptable salt thereof, wherein the patient has been diagnosed with a brain tumor, including, but not limited to, an embryonal brain tumor (*e*.*g.,* medulloblastoma or other primitive neuroectodermal tumor (PNET)), astrocytomas (*e*.*g.,* glioblastoma), oligodendroglioma, ependymoma, choroid plexus tumors, or meningioma.

In some embodiments of the above-described aspects, the regimens comprise the administration of the compound of the invention over a period of 1 day to 36 months, 1 to 24 months, 3 to 6 months or greater than 24 months.

In some embodiments of the above-descnbed aspects, the regimens result in a reduction in the cancer cell population. In a specific embodiment, the regimens result in a 5% to 40%, preferably a 10% to 60%, and more preferably, a 20% to 98% reduction in the cancer cell population.

In certain embodiments of the above-described aspects, the regimens further comprise monitoring the cancer cell population in the patient. In specific embodiments, the monitoring comprises detecting in a specimen from said patient the number or amount of cancer cells in said specimen. In some embodiments, the specimen is a blood specimen or tissue specimen. The regimens, in specific embodiments, comprise administering a second effective amount of the compound of the invention to the patient.

In some embodiments of the above-described aspects, the regimens result in a reduction in the cancer stem cell population. In a specific embodiment, the regimens result in a 5% to 40%, preferably a 10% to 60%, and more preferably, a 20% to 98% reduction in the cancer stem cell population.

In certain embodiments of the above-described aspects, the regimens further comprise monitoring the cancer stem cell population in the patient In specific embodiments, the monitoring comprises detecting in a specimen from said patient the number or amount of cancer stem cells in said specimen. In some embodiments, the specimen is a blood specimen or tissue specimen. The regimens, in specific embodiments, comprise administering a second effective amount of the compound of the invention to the patient.

In some embodiments of the above-described aspects, the regimens comprise intravenous or subcutaneous administration of the compound of the invention. In one embodiment, the regimen comprises intravenous administration of the compound of the invention in a dose of 50 mg/kg or less. In another embodiment, the regimens comprise subcutaneous administration of the compound of the invention in a dose of 50 mg/kg or less. In another embodiment, the regimens comprise intrathecal administration of the compound of the invention in a dose of 50 mg/kg or less. In another embodiment, the regimens comprise oral administration of the compound of the invention in a dose of 50 mg/kg or less.

In certain embodiments of the above-described aspects, the regimens further comprise the administration of an additional therapy to the patient, wherein the compound of the invention and the additional therapy are administered separately, concurrently, or sequentially. The additional therapy can be, for example, chemotherapy, radioimmunotherapy, toxin therapy, prodrug-activating enzyme therapy, surgical therapy, immunotherapy (*e*.*g*., antibody therapy, vaccine therapy, adjuvant administration therapy), radiation therapy, targeted therapy or any combination thereof. In a specific embodiment, the additional therapy is chemotherapy, wherein the regimen comprises the administration of the compound of the invention in combination with an additional therapy. The compound of the invention and the additional therapy can be administered separately, concurrently, or sequentially.

In certain embodiments of the above-described aspects, the compound of the invention used in the above-described regimens is a compound of the formula 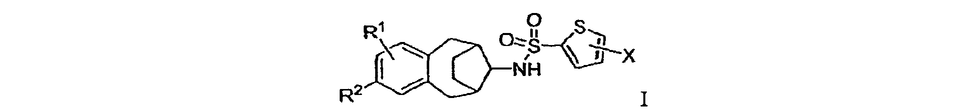 or a pharmaceutically acceptable salt thereof,
wherein:
X is halo;
R¹ is hydrogen, halogen, hydroxy, C₁₋₆alkyl, or C₁₋₄alkoxy;
R² is a radical of the following structure: 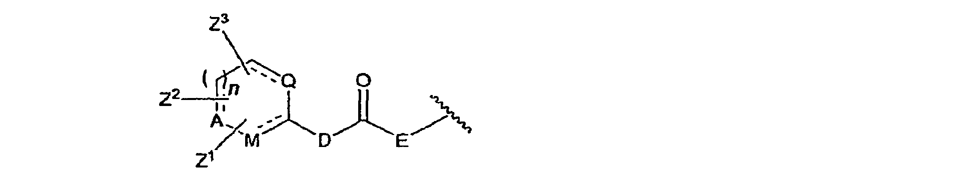
wherein:
E is CH₂ or NH;
D is (CH₂)ₘ, O(CH₂)ₘ, HN(CH₂)ₙ, or CH=CH;
   wherein *m* is 0, 1 or 2;
A and Q are independently N, NCH₃ or C;
M is C or C=O;
*n* is 1 or 2;
Z¹ and Z² are independently H, halo, halo(C₁-₄)alkyl, phenyl, or
Z¹ and Z², when attached to carbon atoms, form a 6-membered aryl ring; and
Z³ is H, halo, halo(C₁-₄)alkyl, or phenyl.

### 4.1. DEFINITIONS

As used herein, the term "agent" refers to any molecule, compound, and/or substance for use in the prevention, treatment, management and/or diagnosis of cancer.

As used herein, the terms "about" or "approximately", unless otherwise indicated, refer to a value that is no more than 10% above or below the value being modified by the term.

As used herein, the term, "alkenyl" means a linear or branched aliphatic hydrocarbon group containing a carbon-carbon double bond having a single radical and 2-10 carbon atoms. A branched" alkenyl means that one or more alkyl groups such as methyl, ethyl or propyl replace one or both hydrogens in a -CH₂- or -CH= linear alkenyl chain. Exemplary alkenyl groups include ethenyl, 1- and 2- propenyl, 1-, 2- and 3- butenyl, 3-methylbut-2-enyl, 2-propenyl, heptenyl, octenyl and decenyl.

As used herein, the term "alkyl" means a linear or branched saturated aliphatic hydrocarbon group having a single radical and 1-10 carbon atoms. Examples of alkyl groups include methyl, propyl, isopropyl, butyl, n-butyl, isobutyl, sec-butyl, tert-butyl, and pentyl. A branched alkyl means that one or more alkyl groups such as methyl, ethyl or propyl, replace one or both hydrogens in a -CH₂- group of a linear alkyl chain. The term "lower alkyl" means an alkyl of 1-3 carbon atoms.

As used herein, the term "antibodies" refer to molecules that contain an antigen binding site, *e*.*g*., immunoglobulins. Immunoglobulin molecules can be of any type (*e*.*g*., IgG, IgE, IgM, IgD, IgA and IgY), class (*e*.*g*., IgG1, IgG2, IgG3, IgG4, IgA1 and IgA2) or subclass. Antibodies include, but are not limited to, monoclonal antibodies, multispecific antibodies, human antibodies, humanized antibodies, camelised antibodies, chimeric antibodies, single domain antibodies, single chain Fvs (scFv), single chain antibodies, Fab fragments, F(ab') fragments, disulfide-linked Fvs (sdFv), and anti-idiotopic (anti-Id) antibodies (including, *e.g.,* anti-Id antibodies to antibodies of the invention), and epitope-binding fragments of any of the above.

As used herein, the terms "antibody conjugate(s)" and "antibody fragment conjugate(s)" refer to a conjugate(s) of an antibody or antibody fragment that is prepared by way of a synthetic chemical reaction(s) or as a recombinant fusion protein(s).

As used herein, the term "aryl" means a carbocyclic aromatic ring system containing one, two or three rings which may be attached together in a pendent manner or fused, and containing a single radical. Exemplary aryl groups include phenyl and naphthyl.

As used herein, the term "cancer" refers to a neoplasm or tumor resulting from abnormal uncontrolled growth of cells. Non-limiting examples include those cancers described in Section 6.3.3, *infra.* The term "cancer" encompasses a disease involving both pre-malignant and malignant cancer cells. In some embodiments, cancer refers to a localized overgrowth of cells that has not spread to other parts of a subject, *i*.*e*., a benign tumor. In other embodiments, cancer refers to a malignant tumor, which has invaded and destroyed neighboring body structures and spread to distant sites. In yet other embodiments, the cancer is associated with a specific cancer antigen.

As used herein, the terms "cancer cell" and "cancer cells" encompass both pre-malignant and malignant cancer cells.

As used herein, the term "cancer stem cell(s)" refers to a cell that can be a progenitor of a highly proliferative cancer cell. A cancer stem cell has the ability to re-grow a tumor as demonstrated by its ability to form tumors in immunocompromised mice, and typically to form tumors upon subsequent serial transplantation in immunocompromised mice. The term "cancer stem cell(s)" also includes cells defined by their gene expression profile and/or cell surface phenotype (e.g. CD34⁺/CD38⁻ for leukemia, CD133⁺ for brain cancer, etc.) and it is noted that such phenotypes may be further refined from time to time. Cancer stem cells can also be slow-growing relative to the remaining bulk of a tumor, that is cancer stem cells are generally quiescent. In certain embodiments, but not all, the cancer stem cell may represent approximately 0.1 to 10% of a tumor.

As used herein, the term "chiral center" refers to a carbon atom to which four different groups are attached.

As used herein, the term "cycloalkenyl" means a non-aromatic monocyclic or multicyclic hydrocarbon ring system containing a carbon-carbon double bond having a single radical and 3 to 12 carbon atoms. Exemplary monocyclic cycloalkenyl rings include cyclopropenyl, cyclopentenyl, cyclohexenyl or cycloheptenyl. An exemplary multi cyclic cycloalkenyl ring is norbornenyl.

As used herein, the term "derivative" in the context of proteinaceous agent (*e*.*g.,* proteins, polypeptides, peptides, and antibodies) refers to a proteinaceous agent that comprises an amino acid sequence which has been altered by the introduction of amino acid residue substitutions, deletions, and/or additions. The term "derivative" as used herein also refers to a proteinaceous agent which has been modified, *i*.*e.,* by the covalent attachment of any type of molecule to the proteinaceous agent. For example, but not by way of limitation, an antibody may be modified, *e*.*g.,* by glycosylation, acetylation, pegylation, phosphorylation, amidation, derivatization by known protecting/blocking groups, proteolytic cleavage, linkage to a cellular ligand or other protein, etc. A derivative of a proteinaceous agent may be produced by chemical modifications using techniques known to those of skill in the art, including, but not limited to specific chemical cleavage, acetylation, formylation, metabolic synthesis in the presence of tunicamycin, etc. Further, a derivative of a proteinaceous agent may contain one or more non-classical amino acids. A derivative of a proteinaceous agent possesses a similar or identical function as the proteinaceous agent from which it was derived. The term "derivative" in the context of a proteinaceous agent also refers to a proteinaceous agent that possesses a similar or identical function as a second proteinaceous agent (*i*.*e*., the proteinaceaous agent from which the derivative was derived) but does not necessarily comprise a similar or identical amino acid sequence of the second proteinaceous agent, or possess a similar or identical structure of the second proteinaceous agent. A proteinaceous agent that has a similar amino acid sequence refers to a second proteinaceous agent that satisfies at least one of the following: (a) a proteinaceous agent having an amino acid sequence that is at least 30%, at least 35%, at least 40%, at least 45%, at least 50%, at least 55%, at least 60%, at least 65%, at least 70%, at least 75%, at least 80%, at least 85%, at least 90%, at least 95% or at least 99% identical to the amino acid sequence of a second proteinaceous agent; (b) a proteinaceous agent encoded by a nucleotide sequence that hybridizes under stringent conditions to a nucleotide sequence encoding a second proteinaceous agent of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least 80 contiguous amino acid residues, at least 90 contiguous amino acid residues, at least 100 contiguous amino acid residues, at least 125 contiguous amino acid residues, or at least 150 contiguous amino acid residues; and (c) a proteinaceous agent encoded by a nucleotide sequence that is at least 30%, at least 35%, at least 40%, at least 45%, at least 50%, at least 55%, at least 60%, at least 65%, at least 70%, at least 75%, at least 80%, at least 85%, at least 90%, at least 95% or at least 99% identical to the nucleotide sequence encoding a second proteinaceous agent. A proteinaceous agent with similar structure to a second proteinaceous agent refers to a proteinaceous agent that has a similar secondary, tertiary or quaternary structure to the second proteinaceous agent. The structure of a proteinaceous agent can be determined by methods known to those skilled in the art, including but not limited to, peptide sequencing, X-ray crystallography, nuclear magnetic resonance, circular dichroism, and crystallographic electron microscopy. In a specific embodiment, a derivative is a functionally active derivative.

As used herein, the phrase "diagnostic agent" refers to any molecule, compound, and/or substance that is used for the purpose of diagnosing cancer. Non-limiting examples of diagnostic agents include antibodies, including those conjugated to a detectable agent. As used herein, the term "detectable agents" refer to any molecule, compound and/or substance that is detectable by any methodology available to one of skill in the art. Non-limiting examples of detectable agents include dyes, gases, metals, or radioisotopes.

As used herein, the term "effective amount" refers to the amount of a therapy that is sufficient to result in the prevention of the development, recurrence, or onset of cancer and one or more symptoms thereof, to enhance or improve the prophylactic effect(s) of another therapy, reduce the severity, the duration of cancer, ameliorate one or more symptoms of cancer, prevent the advancement of cancer, cause regression of cancer, and/or enhance or improve the therapeutic effect(s) of another therapy.

As used herein, the phrase "elderly human" refers to a human between 65 years old or older, preferably 70 years old or older.

As used herein, the term "enantiomer" or "enantiomeric" refers to a molecule that is non-superimposable on its mirror image and hence, is optically active wherein the enantiomer rotates the plane of polarized light in one direction and its mirror image rotates the plane of polarized light in the opposite direction.

As used herein, the phrase "human adult" refers to a human 18 years of age or older.

As used herein, the phrase "human child" refers to a human between 24 months of age and 18 years of age".

As used herein, the term "human infant" refers to a human less than 24 months of age, preferably less than 12 months of age, less than 6 months of age, less than 3 months of age, less than 2 months of age, or less than 1 month of age.

As used herein, the term "immunospecifically binds to an antigen" and analogous terms refer to peptides, polypeptides, proteins, fusion proteins and antibodies or fragments thereof that specifically bind to an antigen or a fragment and do not specifically bind to other antigens. A peptide, polypeptide, protein, or antibody that immunospecifically binds to an antigen may bind to other peptides, polypeptides, or proteins with lower affinity as determined by, *e.g.,* immunoassays, BIAcore, or other assays known in the art.

As used herein, the term "in combination" in the context of the administration of a therapy to a subject refers to the use of more than one therapy (*e.g.,* prophylactic and/or therapeutic). The use of the term "in combination" does not restrict the order in which the therapies (*e*.*g*., a first and second anticancer therapeutic) are administered to a subject. A therapy can be administered prior to (*e*.*g*., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e*.*g*., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, 12 weeks or 24 weeks after) the administration of a second therapy to a subject which had, has, or is susceptible to cancer. In a particular embodiment, the therapies are administered to a subject in a sequence and within a time interval such that they provide an increased benefit than if they were administered otherwise. Any additional therapy can be administered in any order with the other additional therapy.

As used herein, the terms "manage," "managing," and "management" in the context of the administration of therapy to a subject refer to the beneficial effects that a subject derives from a therapy (*e*.*g*., a prophylactic or therapeutic agent) or a combination of therapies, while not resulting in a cure of cancer. In certain embodiments, a subject is administered one or more therapies (*e*.*g*., one or more prophylactic or therapeutic agents) to "manage " cancer so as to prevent the progression or worsening of the condition.

As used herein, the term "marker" in the context of a tissue (e.g. a cell or tumor) means any antigen, molecule or other chemical or biological entity that is specifically found in a tissue that it is desired to be identified in a particular tissue affected by a disease or disorder. In specific embodiments, the marker is a cell surface antigen that is differentially or preferentially expressed by specific cell types. For example, in leukemia, a cancer stem cell differentially expresses CD123 relative to normal stem cells. In another embodiment, a brain cancer stem cell may differentially express CD133 relative to the tumor bulk. In another specific embodiment, the marker is an intracellular protein that is expressed in the cancer stem cells relative to other cell types. For example, brain cancer stem cells may express nestin or aldehyde dehydrogenase at higher levels than cells of the tumor bulk.

As used herein, the term "marker phenotype" in the context of a tissue (*e*.*g*., a cell or a tumor) means any combination of antigens (*e*.*g.,* receptors and ligands), molecules or other chemical or biological entities that are specifically found in a tissue that it is desired to identify, in particular, tissue affected by a disease or disorder (*e*.*g.,* a metastases). Where ant antibody is used as the tracer, a marker is an antigen. In specific embodiments, the marker phenotype is a cell surface phenotype. In other embodiments, the marker may be intracellular proteins. In accordance with this embodiment, the cell surface phenotype may be determined by detecting the expression of a combination of cell surface antigens. Non-limiting examples of cell surface phenotypes of cancer stem cells include CD34⁺/CD38⁻, CD34⁺/CD38⁻/CD123⁺, CD44⁺/CD24⁻, CD133⁺, CD34⁺/CD10⁻ /CD19⁻, CD138⁻7CD34⁻/CD19⁺, CD133⁺/RC2⁺, CD44⁺/α₂β₁^{hi}/CD133⁺ and other cell surface phenotypes mentioned herein, as well as those that are known in the art.

As used herein, the phrase "pharmaceutically acceptable" means approved by a regulatory agency of the federal or a state government, or listed in the U.S. Pharmacopeia, European Pharmacopeia, or other generally recognized pharmacopeia for use in animals, and more particularly, in humans.

As used herein, the terms "prevent", "preventing" and "prevention" in the context of the administration of a therapy to a subject refer to the prevention or inhibition of the recurrence (*e*.*g*., relapse), onset, and/or development of a cancer or a symptom thereof in a subject resulting from the administration of a therapy (*e*.*g*., a prophylactic or therapeutic agent), or a combination of therapies (*e*.*g*., a combination of prophylactic or therapeutic agents). In some embodiments, such terms refer to one, two, three or more results following the administration of one or more therapies: (1) a stabilization, reduction or elimination of the cancer stem cell population, (2) a stabilization, reduction or elimination of the cancer cell population, (3) an increase in the length of remission, (4) a decrease in the recurrence rate of cancer, (5) an increase in the time to recurrence of cancer, and (6) an increase in the survival of the patient. In specific embodiments, such terms refer to a stabilization, reduction or elimination of the cancer stem cell population.

As used herein, the term "predetermined reference range" refers to a reference range for the particular biological entity *e*.*g*., cancer stem cell, for a subject or a population of subjects. Each laboratory may establish its own reference range for each particular assay, or a standard reference range for each assay may be made available and used locally, regionally, nationally, or worldwide. In one specific embodiment, the term refers to a reference range for the number or amount of cancer stem cells in a specimen from a patient. In another specific embodiment, the term refers to a reference range for the number or amount of cancer cells in a specimen from a patient.

As used herein, the phrase "prophylactic agent" refers to any molecule, compound, and/or substance that is used for the purpose of preventing cancer. Examples of prophylactic agents include, but are not limited to, proteins, immunoglobulins (*e*.*g*., multi-specific Igs, single chain Igs, Ig fragments, polyclonal antibodies and their fragments, monoclonal antibodies and their fragments), antibody conjugates or antibody fragment conjugates; peptides (*e*.*g*., peptide receptors, selectins), binding proteins, chemospecific agents, chemotoxic agents (*e*.*g*., anti-cancer agents), and small molecule drugs.

As used herein, the term "prophylactically effective regimen" refers to a regimen for dosing, timing, frequency and duration of the administration of one or more therapies for the prevention of cancer or a symptom thereof.

As used herein, the term "racemic" refers to a mixture of equal parts of enantiomers and which is optically inactive.

As used herein, the term "resolution" refers to the separation or concentration or depletion of one of the two enantiomeric forms of a molecule.

As used herein, the term "small reduction", in the context of a particular cell population (*e*.*g*., circulating endothelial cells and/or circulating endothelial progenitors) refers to less than a 30% reduction in the cell population (*e*.*g*., the circulating endothelial cell population and/or the circulating endothelial progenitor population).

As used herein, the term "stabilizing" and analogous terms, when used in the context of a cancer stem cell population or cancer cell population, refer to the prevention of an increase in the cancer stem cell population or cancer cell population, respectively. In other words, the percentage of cancer stem cells or the percentage of cancer cells that a cancer is composed of is maintained, and does not increase, or increases by less than 20%, preferably less than 10%.

As used herein, the term "stereoisomers" is a general term for all isomers of individual molecules that differ only in the orientation of their atoms in space. It includes enantiomers and isomers of compounds with more than one chiral center that are not mirror images of one another (diastereomers).

As used herein, the term "synergistic" refers to a combination of therapies which is more effective than the additive effects of any two or more single therapies. A synergistic effect of a combination of therapies permits the use of lower dosages of one or more of therapies and/or less frequent administration of said therapies to a subject. The ability to utilize lower dosages of therapies and/or to administer said therapies less frequently reduces the toxicity associated with the administration of said therapies to a subject without reducing the efficacy of said therapies in the prevention, treatment, and/or management of cancer. In addition, a synergistic effect can result in improved efficacy of therapeutic modalities in the prevention or treatment of cancer. Finally, the synergistic effect of a combination of therapies may avoid or reduce adverse or unwanted side effects associated with the use of any single therapy.

As used herein, the terms "subject" and "patient" are used interchangeably. As used herein, the term "subject" refers to an animal, preferably a mammal such as a non-primate (*e*.*g*., cows, pigs, horses, cats, dogs, rats etc.) and a primate (*e*.*g*., monkey and human), and most preferably a human. In some embodiments, the subject is a non-human animal such as a farm animal (*e*.*g*., a horse, pig, or cow) and a pet (*e*.*g*., a dog or cat). In a specific embodiment, the subject is an elderly human. In another embodiment, the subject is a human adult. In another embodiment, the subject is a human child. In yet another embodiment, the subject is a human infant.

As used herein, the term "therapeutically effective regimen" refers to a regimen for dosing, timing, frequency and duration of the administration of one or more therapies for the treatment, and/or management of cancer or a symptom thereof.

As used herein, the term "therapeutic agent" refers to any molecule, compound, and/or substance that is used for the purpose of treating and/or managing cancer. Examples of therapeutic agents include, but are not limited to, proteins, immunoglobulins (*e.g.,* multi-specific Igs, single chain Igs, Ig fragments, polyclonal antibodies and their fragments, monoclonal antibodies and their fragments), antibody conjugates or antibody fragment conjugates, peptides (*e*.*g*., peptide receptors, selectins), binding proteins, chemospecific agents, chemotoxic agents (*e*.*g*., anti-cancer agents), and small molecule drugs.

As used herein, the terms "therapies" and "therapy" can refer to any method(s), composition(s), and/or agent(s) that can be used in the prevention, treatment and/or management of a cancer or one or more symptoms thereof. In certain embodiments, the terms "therapy" and "therapies" refer to chemotherapy, radiation therapy, surgery, hormonal therapy, biological therapy, immunotherapy and/or other therapies useful in the prevention, management and/or treatment of a cancer or one or more symptoms thereof.

As used herein, the terms "treat", "treatment", and "treating" in the context of the administration of a therapy to a subject refer to the reduction or inhibition of the progression and/or duration of cancer, the reduction or amelioration of the severity of cancer, and/or the amelioration of one or more symptoms thereof resulting from the administration of one or more therapies. In specific embodiments, such terms refer to one, two or three or more results following the administration of one, two, three or more therapies: (1) a reduction or elimination in the cancer stem cell population; (2) a reduction or elimination in the cancer cell population; (3) a reduction in the growth of a tumor or neoplasm; (4) a decrease in tumor size; (5) a reduction in the formation of a tumor; (6) eradication, removal, or control of primary, regional and/or metastatic cancer; (7) a decrease in the number or size of metastases; (8) a reduction in mortality; (9) an increase in survival rate, progression free survival, overall survival, disease free survival, relapse free survival, time to progression; (10) an increase in the number of patients in remission; (11) a decrease in hospitalization rate, (12) a decrease in hospitalization lengths, or (13) the size of the tumor is maintained and does not increase or increases by less than 20%, preferably less than 10%. In certain embodiments, such terms refer to a reduction in the cancer stem cell population. In some embodiments, such terms refer to a reduction in the growth of cancerous cells. In some embodiments, such terms refer to a reduction in the cancer stem cell population and a reduction in the cancer cell population. In some embodiments, such terms refer to a reduction in the growth and/or formation of a tumor. In some embodiments, such terms refer to the eradication, removal or control of primary, regional or metastatic cancer (*e.g*., the minimization or delay of the spread of cancer). In some embodiments, such terms refer to a reduction in mortality and/or an increase in survival rate of a patient population. In further embodiments, such terms refer to an increase in the number of patients in remission. In some embodiments, such terms refer to a decrease in hospitalization rate of a patient population and/or a decrease in hospitalization length for a patient population.

Concentrations, amounts, cell counts, percentages and other numerical values may be presented herein in a range format. It is to be understood that such range format is used merely for convenience and brevity and should be interpreted flexibly to include not only the numerical values explicitly recited as the limits of the range but also to include all the individual numerical values or sub-ranges encompassed within that range as if each numerical value and sub-range is explicitly recited.

### 5. BRIEF DESCRIPTION OF THE DRAWINGS

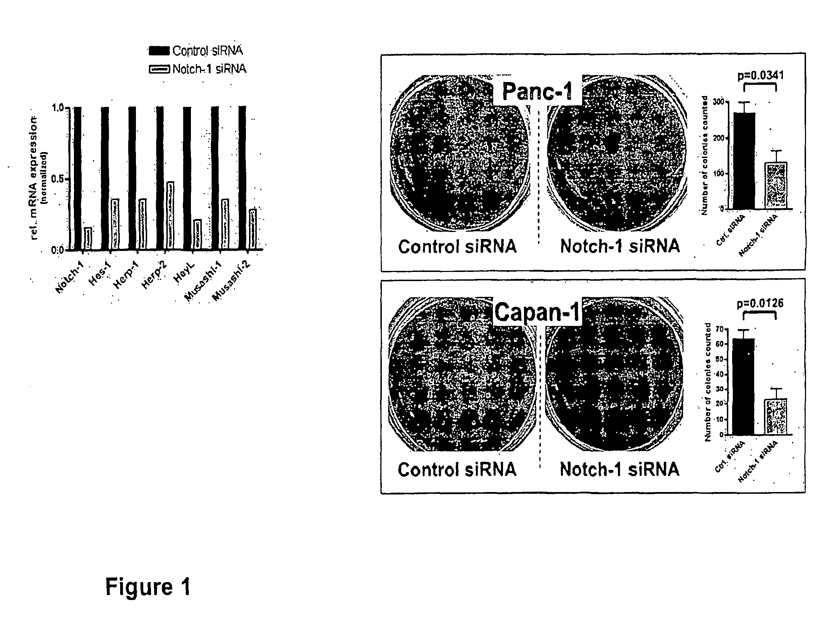

Figure 1 shows that NOTCH1 siRNA inhibits the clonogenicity of pancreatic cancer cell lines. (Left) Downregulation of Notch1 transcripts by RNA-mediated interference downregulates multiple Notch gene targets, including Hes1, Herp1, Herp2, HeyL, Musashil, and Musashi2. Specificity of the RNA interference is confirmed by downregulation of Notch1 transcript levels with the specific siRA, but not with the scrambled controls. (Right) In two pancreatic cancer cell lines, Panc-1 and Capan-1, Notch1 siRNA significantly inhibits clonogenicity in soft agar. Bar graphs represent colony counts at two weeks, with mean and standard deviations calculated from triplicate experiments.

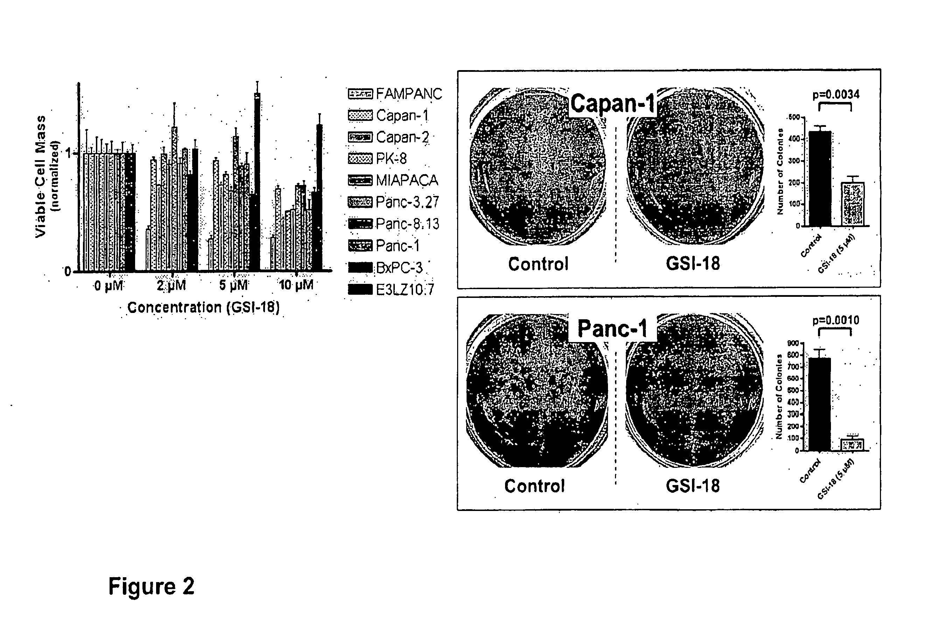

Figure 2 shows that gamma secretase inhibitor GSI-18 has a modest to minimal effect of *in vitro* growth, but profound effects on pancreatic cancer clonogenicity. (Left) GSI-18 has only modest to minimal effects on growth as measured by MTT assays at 96 hours, in a panel of 10 human pancreatic cancer cell lines, at dosages of 2, 5, and 10µM. (Right) In contrast, GSI-18 demonstrates significant inhibition of clonogencity as assessed by colony formation in soft agar in two pancreatic cancer cell lines, Panc-1 and Capan-1. Bar graphs represent colony counts at two weeks, with mean and standard deviations calculated from triplicate experiments..

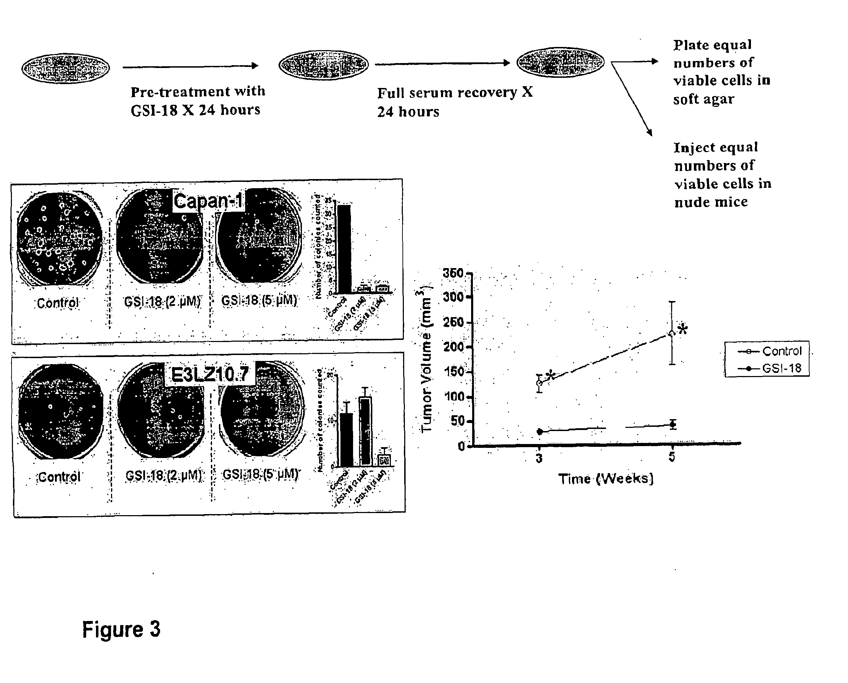

Figure 3 shows that pre-treatment with the gamma secretase inhibitor GSI-18 significantly inhibits the clonogenicity of pancreatic cancer *in vitro*, and tumorigenicity *in vivo*. (Top) Experimental scheme illustrating pre-treatment of pancreatic cancer cell lines with GSI-18, prior to plating for soft agar assays and subcutaneous injection in athymic mice. Treatment for 24 hours with GSI-18 or vehicle is followed by recovery in full serum for a further 24 hours minus the drug. Equal numbers of viable cells from the control and treated groups are then plated for colony assays in soft agar, or injected in nude mice. Of note, during the *in vitro* or *in vivo* assays, no further drug is administered. (Right) Significant inhibition of colony formation is seen following pre-treatment with GSI-18 for both Capan-1 and E3LZ10.7 cell lines. The dosage required for significant inhibition of clonogenicity varies between 2µM (Capan-1) to 5 µM (E3LZ10.7). Bar graphs represent colony counts at two weeks, with mean and standard deviations calculated from triplicate experiments. (Left) Pre-treatment with GSI-18 inhibits tumorigenicity of Panc-1 pancreatic cancer cell line in athymic mice. At five weeks post-injection, vehicle-treated mice demonstrate robust tumor formation, while minimal tumor volumes are observed in the GSI-18 treated cells. Error bars represent standard deviation of five mice per arm.

Figure 4A shows flow cytometric analysis in the adherent MB/primitive neuroectodermal tumor (PNET) cell line PFSK, and the D425Med cell line, which grows in suspension.

Figure 4B shows flow cytometric analysis in the adherent MB/primitive neuroectodermal tumor (PNET) cell lines DAOY and PFSK; and the D283Med and D425Med lines, which grow in suspension.

Figure 4C shows a histogram with the number of colonies formed with CD133-enriched DAOY cultures versus the number of colonies formed in CD133-depleted cell cultures after 4 weeks growth in soft agar.

Figure 4D is a histogram showing the concentrations of, Hes1 mRNA, a marker of Notch pathway activity, present in the CD133-enriched and the CD133-depleted fractions of DAOY, and in the CD133-enriched and the CD133-depleted fractions of PFSK.

Figure 4E shows both mRNA and protein levels of the Notch pathway target Hes1 after 48 hours by various concentration of GSI-18.

Figure 5A shows the ability of DAOY and DAOY:NICD2 cell cultures to excrete Hoechst dye in the absence of GSI-18 and verapamil, in the presence of veraparmil, and in the presence of 2 µM GSI-18.

Figure 5B is a histogram showing the % side population of DAOY, DAOY: NICD2, D283, and D425 cell cultures in the presence and absence of 2 µM GSI-18.

Figures 6A and 6B shows Nestin staining in DAOY cells and in MB2 cells.

Figure 6C is a histogram showing the % nestin-positive cells in DAOY cell culture, DAOY-NIC2D cell culture, and MB2 cell culture in the presence and absence of 2 µM GSI-18.

Figure 6D is a histogram showing the % Ki67, a marker of proliferating cells, in nestin-positive and nestin-negative cells in the presence or absence of 2 µM GSI-18.

Figure 6E is a histogram showing the % change in G1/G0, S, and G2/M fractions in DAOY and PFSK cell cycles upon treatment with 2 µM GSI-18.

Figure 6F is a histogram showing the % caspase-positive fraction of nestin-negative and nestin-positive cells.in the absence and presence of 2 µM GSI-18.

Figure 7A is a histogram showing gamma-secretase inhibitor dose-dependent mRNA change of TuJ1 and GABRA6 in DAOY cell culture.

Figure 7B is a graph showing viable cell mass plotted against time MB cell cultures.

Figure 7C shows panels of viable cell mass at 0 µM, 0.4 µM, and 2 µM GSI-18.

Figure 7D is a histogram showing the viable cell mass in DAOY and DAOY:NIC2D cell cultures at 0 µM, 0.4 µM, and 2 µM GSI-18.

Figure 7E is a histogram showing rowth in the number of live cells in DAOY, D283Med, D425Med and PFSK cultures exposed to 2 µM GSI-18.

Figure 7F shows the phosphorylation of at various concentrations of GSI-18.

Figure 7G is a histogram showing the basal apoptotic rate in MB cells stably transfected with constitutively active Akt and in parental or vector transfected cells in the absence or presence of 2 µM GSI-18.

Figure 7H is a histogram showing colony-forming ability of vehicle-treated and 2 µM GSI-18-treated DAOY cultures, and in vehicle-treated and 2 µM GSI-18-treated DAOY:NIC2D cultures.

Figure 8A shows xenograft formation in athymic nude mice after sub-cutaneous injection of vehicle treated DAOY cells.

Figure 8B shows xenograft formation in athymic nude mice after sub-cutaneous injection of 2 µM GSI-18 treated DAOY cells.

Figure 8C is a histogram showing xenograft volume following injection after sub-cutaneous injection of DAOY and DAOY:NIC2D cells.

Figure 8D is a graph plotting xenograft volume against number of days following injection of DAOY and DAOY:NIC2D cells; and S-phase proliferation rates as determined by flow cytometric analysis of S-phase fraction (inset).

Figure 8E shows xenograft formation in athymic nude mice after sub-cutaneous injection of vehicle treated D425 Med cells, but not on the contralateral side (arrow) injected with cells previously treated with GSI-18 for 48 hours.

Figure 8F shows xenograft formation in an athymic nude mouse treated for 5 consecutive days with 0.5 mg i.p. of GSI-18 after sub-cutaneous injection of DAOY cells.

Figure 8G is a graph plotting xenograft volume against number of days following injection of DAOY and DAOY:NIC2D cells in athymic nude mice, which were either treated with vehicle or 0.5 mg i.p. of GSI-18 for 5 consecutive days following subcutaneous tumor injection.

Figures 9A and 9B are panels showing proliferation in nestin-positive DAOY cells following treatment with 2 µM GSI-18 or vehicle as measured by Ki67 antibodies.

Figures 9C and 9D are panels showing apoptosis in nestin-positive DAOY cells following treatment with 2 µM GSI-18 or vehicle as measured by cleaved caspase-3 antibodies (using cleaved caspase-3 immunofluoresence).

Figure 9E is a histogram showing % apoptotic induction in DAOY cells as measured by cleaved caspase 3 immunofluorescence and flow cytometric analysis of Annexin staining.

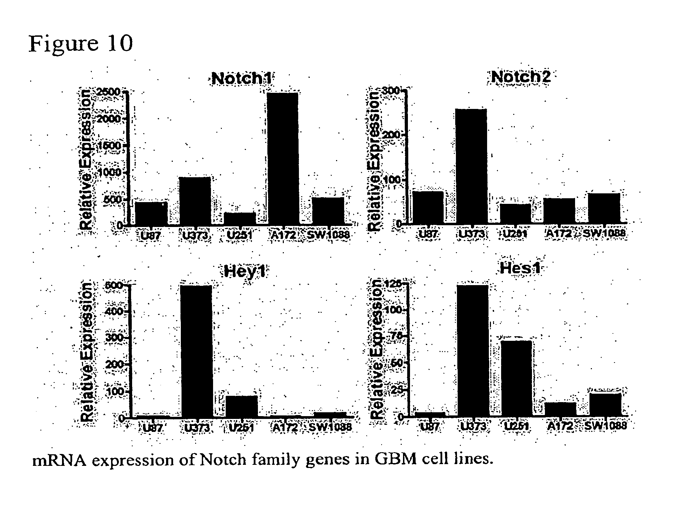

Figure 10 shows the expression of Notch family genes in GBM cells. mRNA expression of the Notch1 and Notch2 receptors, and pathway targets Hes1 and Heyl were quantified by real-time RT-PCR in GBM cell lines U87, U373, A172 and SW1088 (normalized to beta-Actin).

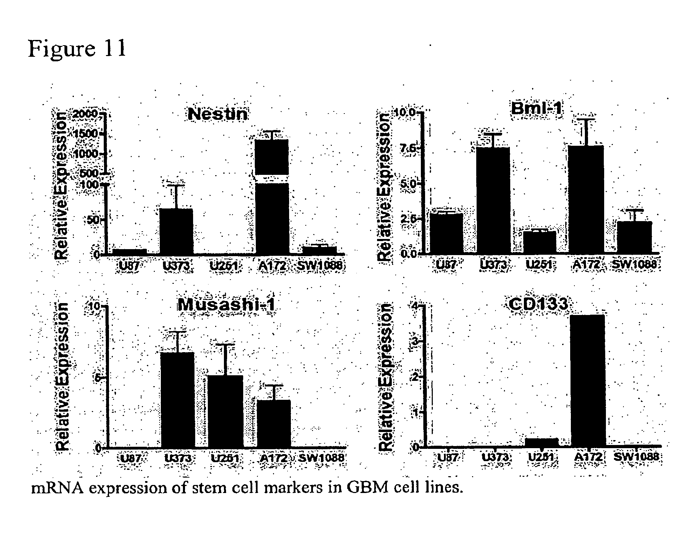

Figure 11 show the expression of neural stem cell markers in GBM cells. mRNA expression of the stem cell markers Nestin, Bmi1, Musashi-1, and CD133 was quantified by real-time RT-PCR in GBM cell lines U87, U373, A172 and SW1088 (normalized to beta-Actin).

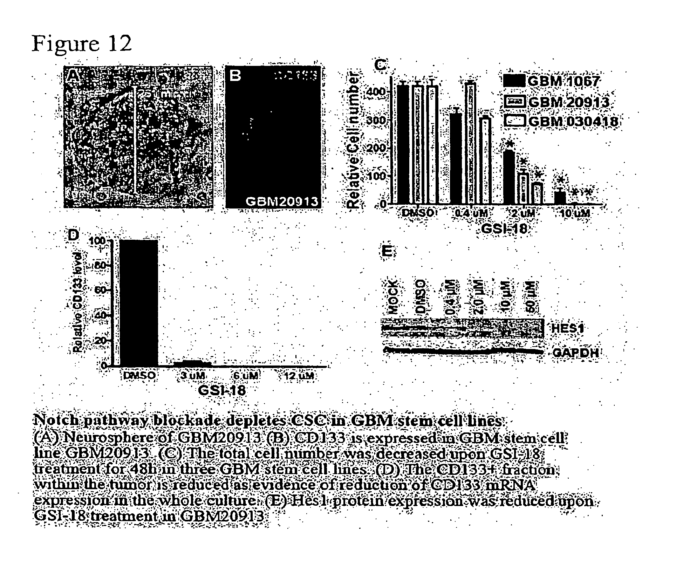

Figure 12 shows that Notch pathway blockade depletes CSCs in GBM neurosphere lines and inhibits tumor growth *in vitro*. (A, B) CD133 is expressed in GBM neurosphere line HSR-GBM1a. (C) The total cell number was decreased upon GSI-18 treatment for 48h in this line and in the subclones of the line. (D) CD133 RNA expression in the whole culture is significantly reduced following Notch Blockade, suggesting that CSCs have been depleted. (E) Protein expression of Hes1, a Notch pathway target gene, was reduced upon GSI-18 treatment in GBM neurosphere cells.

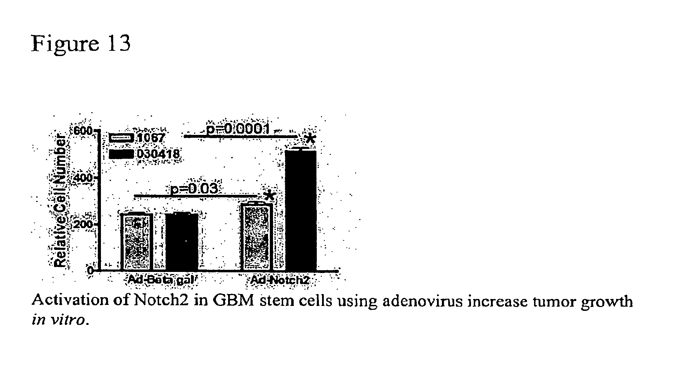

Figure 13 shows that activation of Notch2 in GBM neurospheres increases tumor growth *in vitro.* (A) The active form of Notch2 receptor (NICD2) was stably transfected into HSR-GBMla cells. mRNA expression ofNICD2 was significantly increased, as measured by real-time RT-PCR. (B) The Notch target gene Hes1 was also increased in NICD2 transfected GBM neurospheres. (C) Cell growth was significantly increased in NICD2 transfected cells. (D) Transient expression of NICD2 in GBM neurosphere lines HSR-GBM1b and HSR-GBM1c using adenovirus vectors also increased cell growth.

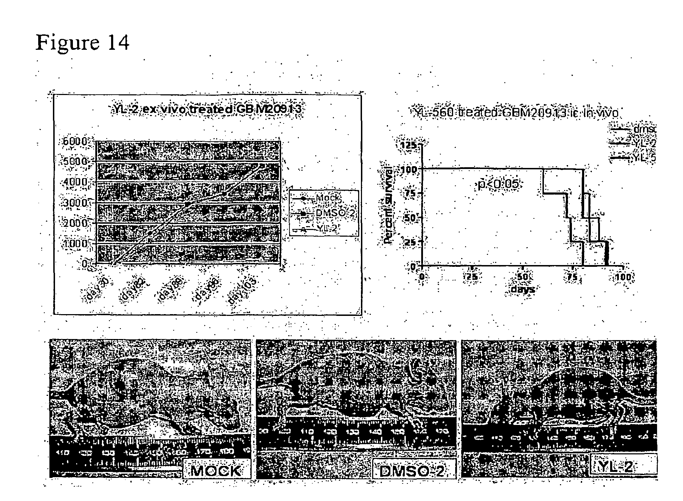

Figure 14 shows that Notch inhibition reduces GBM neurosphere subcutaneous engraftment and prolongs the survival of mice bearing intracranial xenografts. (A,B,C) Large xenografts were formed at all eight sites (asterisk) injected with cells in which Notch signaling was active, including non-treated (n=4, A) and vehicle (DMSO) treated (n=4) cells. Mice injected with equal number of cells previously treated with 2 µM GSI-18 (referred to in Figure 14 as YL-560) (n=6) were unable to efficiently form tumors, with small-lesions developing at only three of 6 injection sites. (D) Tumor volume was measured in mock, DMSO and 2 µM GSI-18 treated cells. (E) Mice bearing intracranial xenografts from GSI-18 (referred to in Figure 14 as YL-560) treated GBM20913 have prolonged survival compare to mice with vehicle treated xenografts.

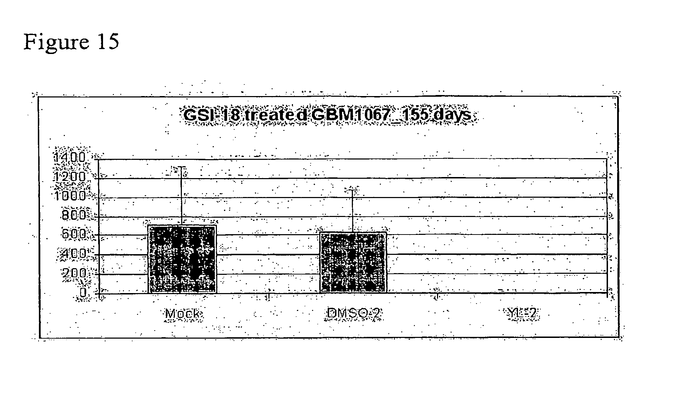

Figure 15 shows that Notch inhibition reduces GBM neurosphere subcutaneous engraftment in another GBM neurosphere subline GBM10627. Neurosphere cells experienced Notch inhibition were unable to form subcutaneous xenografts in nude mice.

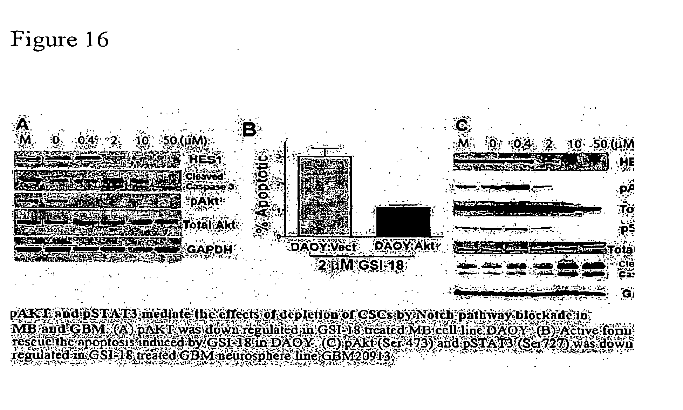

Figure 16 shows that Akt-dependant apoptosis also contributes to depletion of CSCs by Notch pathway blockade. (A) Increase of cleaved Caspase 3 and loss of Akt phosphorylation is induced by 0.4 uMor higher levels of GSI-18, while overall levels of Akt and GAPDH are unchanged in DAOY medulloblastoma cells. (B) Cell death in 2 uM GSI-18 is reduced in a representative subclone of DAOY expressing activated Akt as compare to vector transfected cells. (C) Loss of Akt phosphorylation and activation of Caspase3 cleavage is induced by 2 µM or higher levels of GSI-18, while overall levels of Akt and GAPDH are unchanged in GBM neurosphere cells.

### 6. DETAILED DESCRIPTION OF THE INVENTION

The present invention generally relates to compounds and methods for preventing, treating, and/or managing cancer, the methods comprising administering to a patient in need thereof a prophylactically effective regimen or a therapeutically effective regimen, the regimen comprising administering to the patient a compound of the invention, or a pharmaceutically acceptable salt thereof (as described in Section 6.1 *infra*). In some embodiments, the regimen results in a reduction of the cancer stem cell population. While not being bound by any specific theory, the inventors believe a reduction in the cancer stem cell population or the elimination of cancer stem cells in the patient ultimately improves prospects for disease-free survival, remission-free survival, progression-free survival, and/or overall survival. In specific embodiments, the compounds of the invention demonstrate selective cytotoxicity toward cancer stem cells in comparison with stem cells obtained from healthy subjects.

For clarity of disclosure, and not by way of limitation, the detailed description of the invention is divided into the subsections which follow.

### 6.1 COMPOUNDS OF THE INVENTION

### 6.1.1 SYNTHESES OF THE COMPOUNDS OF THE INVENTION

### 6.2 PHARMACEUTICAL COMPOSITIONS AND ROUTES OF ADMINISTRATION 6.3 PROPHYLACTIC AND THERAPEUTIC USES

### 6.3.1 DOSAGE REGIMENS

### 6.3.2 OTHER THERAPIES

### 6.3.3 TARGET CANCERS

### 6.3.4 PATIENT POPULATION

### 6.4 METHODS OF MONITORING CANCER STEM CELLS

### 6.5 METHODS OF MONITORING CANCER CELLS

### 6.6 METHODS OF MONITORING LYMPHOCYTE CELL COUNT & NEUTROPHIL CELL COUNT

### 6.7 BIOLOGICAL ASSAYS

### 6.7.1 IN VITRO ASSAYS

### 6.7.2 IN VIVO ASSAYS

### 6.7.3 ASSESSING TOXICITY

### 6.8 ARTICLES OF MANUFACTURE

### 6.1. COMPOUNDS OF THE INVENTION

Compounds of the invention are gamma-secretase inhibitors. Examples of suitable gamma-secretase inhibitors are disclosed, for instance, in U.S. Patent Application Publication Nos. 2004/0029862, 2004/0049038, 2004/0186147, 2005/0215602, 2005/0182111,2005/0182109, 2005/0143369, and 2005/0119293, the disclosures, each of which is hereby incorporated by reference in its entirety. In addition, suitable gamma-secretase inhibitors are disclosed in International Publication Nos. WO 2001/70677, WO 2002/049038, WO 2004/186147, WO 2003/093253, WO 2003/093251, WO 2003/093252, WO 2003/093264, WO 2005/030731, WO 2005/014553, WO 2004/039800, and WO 2004/039370, the disclosures, each of which is hereby incorporated by reference in its entirety.

In some embodiments, the compounds of the invention inhibit the Notch pathway in addition to inhibiting gamma-secretase.

In some embodiments, the compounds of the invention are compounds of the formula I, having the following formula: 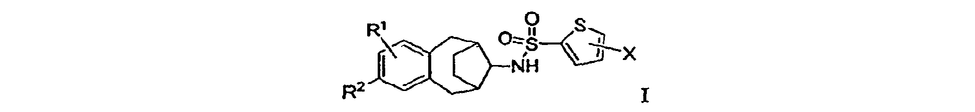 wherein:
X is halo;
R¹ is hydrogen, halogen, hydroxy, C₁₋₆alkyl, or C₁₋₄alkoxy;
R² is a radical of the following structure: 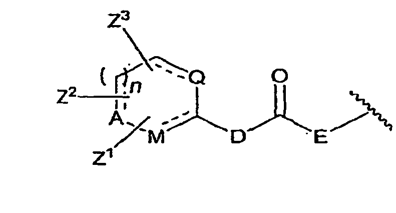
wherein:
E is CH₂ or NH;
D is (CH₂)*ₘ*, O(CH₂)*ₘ*, HN(CH₂)*ₙ*, or CH=CH;
   wherein *m* is 0, 1 or 2;
A and Q are independently N, NCH₃ or C;
M is C or C=O;
*n* is 1 or 2;
Z¹ and Z² are independently H, halo, halo(C₁-₄)alkyl, phenyl, or Z¹ and Z², when attached to carbon atoms, form a 6-membered aryl
ring; and
Z³ is H, halo, halo(C₁-₄)alkyl, or phenyl.

In certain embodiments of the compound of the formula I, *n* is 1; and A, M, and Q are C. For instance, R² can be a radical of the following structure: 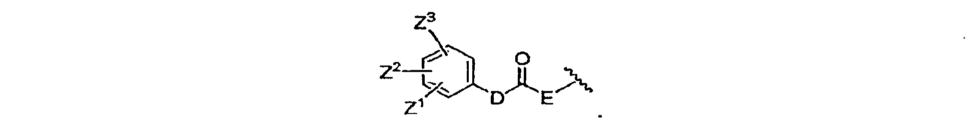 In specific embodiments wherein R² has this structure, D is (CH₂)*ₘ*, wherein *m* is 0, 1, or 2. For example, in a specific embodiment, D is (CH₂)*ₘ* and *m* is 0. In another specific embodiment, D is (CH₂)*ₘ* and *m* is 1. In another specific embodiment, D is (CH₂)*ₘ* and *m* is 2.

In other specific embodiments of the compound of the formula I, wherein R² is a radical of the structure: 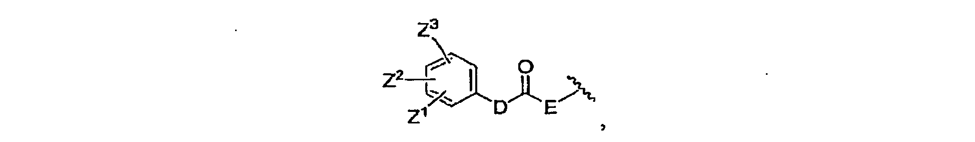 D is O(CH₂)*ₘ*, wherein *m* is 2.. In other specific embodiments wherein R² has this structure, D is CH=CH. The double bond of D can be, for example, in the trans configuration.

In some embodiments of the compound of the formula I, wherein R² is a radical of the structure: 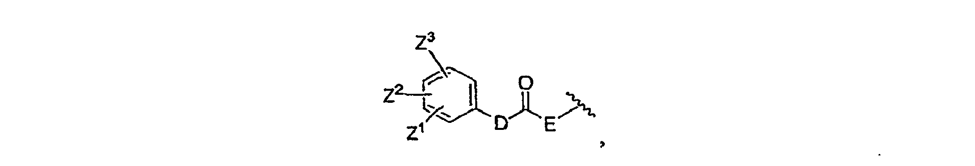 Z¹, Z² and Z³ are independently hydrogen, halo, or halo(C₁-₄)alkyl. For instance, Z¹, Z² and Z³ independently can be hydrogen, fluoro, or perfluoro(C₁-₄)alkyl.

In some embodiments of the compound of the formula I, wherein R² is a radical of the structure: 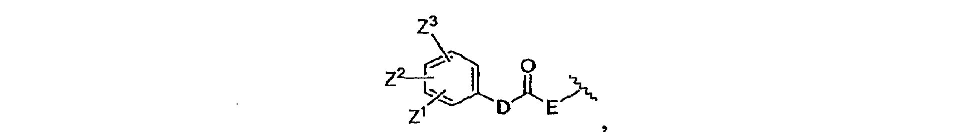 E is NH.

In other certain embodiments of the compound of the formula I, *n* is 2; and A is NCH₃, Q is N, and M is C=O. For instance, R² can be a radical of the following structure: 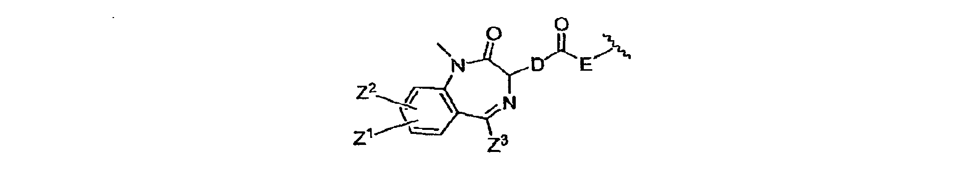 In specific embodiments, wherein R² is a radical of this benzodiazepinone structure, Z³ is phenyl. In a particular embodiment, wherein R² is a radical of this benzodiazepinone structure, Z³ is phenyl, D is HN(CH₂)ₙ and *n* is 1.

In specific embodiments, wherein R² is a radical of this benzodiazepinone structure, E is NH.

In some embodiments of the compound of the formula I, X is chloro. For instance, in some embodiments the compound of the formula I, the compound has the following formula:

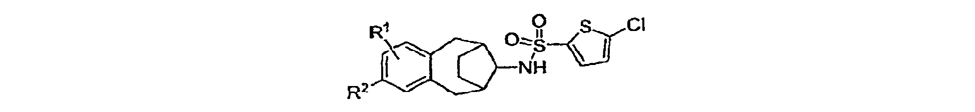

In some embodiments of the compound of the formula I, R¹ is hydrogen.

In specific embodiments of the compound of formula I, the compound has one of the following formula: 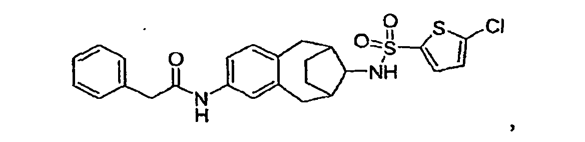 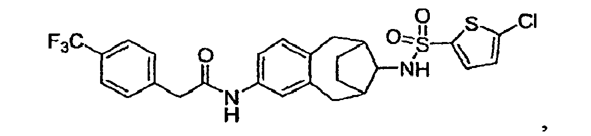 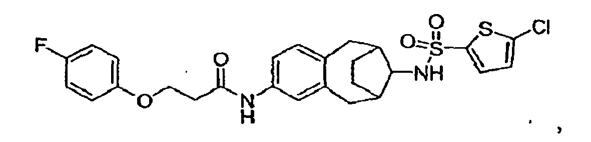 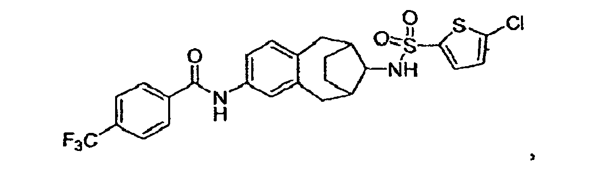 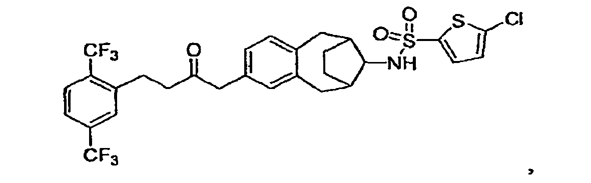 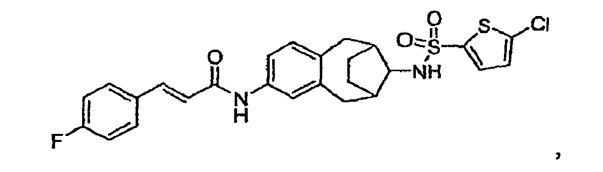 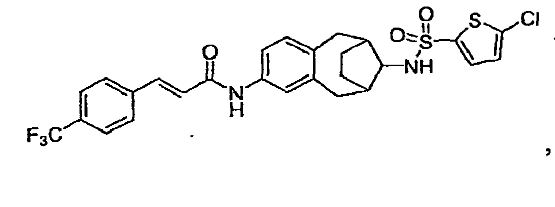 or

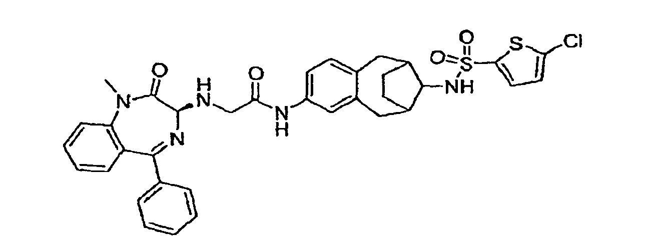

Any of the above-described compounds of the invention, including a compound of the formula I can be used in the prophylactic and therapeutic methods described in Section 6.3.

Some of the compounds disclosed herein may contain one or more asymmetric centers, and may thus give rise to enantiomers, diastereomers, and other stereoisomeric forms. The present invention is meant to encompass all such possible forms as well as their racemic and resolved forms and mixtures thereof (*e.g*., an enantiomeric mixture enriched for one enantiomer). A compound of the invention having one or more asymmetric centers can be in the form of an optical isomer or a diastereomer. Purified optical isomers can be isolated by known techniques such as chiral chromatography, or by formation of diastereomeric salts from an optically active acid or base. In other embodiments, optically pure isomers can be obtained by synthesis from optically pure starting materials.

The invention disclosed herein is meant to encompass all pharmaceutically acceptable salts thereof of the disclosed compounds. The pharmaceutically acceptable salts include, but are not limited to, metal salts such as sodium salt, potassium salt, cesium salt and the like; alkaline earth metals such as calcium salt, magnesium salt and the like; organic amine salts such as triethylamine salt, pyridine salt, picoline salt, ethanolamine salt, triethanolamine salt, dicyclohexylamine salt, N,N'-dibenzylethylenediamine salt and the like; inorganic acid salts such as hydrochloride, hydrobromide, sulfate, phosphate and the like; organic acid salts such as formate, acetate, trifluoroacetate, maleate, fumarate, tartrate and the like; sulfonates such as methanesulfonate, benzenesulfonate p-toluenesulfonate, and the like; amino acid salts such as arginate, asparginate, glutamate and the like.

The invention disclosed herein is also meant to encompass all prodrugs of the disclosed compounds. Prodrugs are considered to be any covalently bonded carriers which release the active parent drug *in vivo.*

The invention disclosed herein is also meant to encompass the *in vivo* metabolic products of the disclosed compounds. Such products may result, for example, from the oxidation, reduction, hydrolysis, amidation, esterification and the like of the administered compound, primarily due to enzymatic processes. Accordingly, the invention includes compounds produced by a process comprising contacting a compound of this invention with a mammal for a period of time sufficient to yield a metabolic product thereof. Such products typically are identified by preparing a radiolabelled compound of the invention, administering it parenterally in a detectable dose to an animal such as rat, mouse, guinea pig, monkey, or to man, allowing sufficient time for metabolism to occur and isolating its conversion products from the urine, blood or other biological samples.

The invention disclosed herein is also meant to encompass the disclosed compounds being isotopically-labelled by having one or more atoms replaced by an atom having a different atomic mass or mass number. Examples of isotopes that can be incorporated into the disclosed compounds include isotopes of hydrogen, carbon, nitrogen, oxygen, phosphorous, fluorine and chlorine, such as ²H ³H, ¹³C, ¹⁴C, ¹⁵N, ¹⁸O, ¹⁷O, ³¹P, ³²P, ³⁵S, ¹⁸F, and ³⁶Cl, respectively.

In certain embodiments, the compounds of the invention may be covalently bound to antibodies or other agents that target cancer stem cells. For instance, the compounds of the invention can be covalently bound to an antibody that binds to a cell surface antigen present on a cancer stem cell. In particular embodiments, the cell surface antigen to which the antibody binds is CD123, CD44, CD34, CD133, CD19, CD38, CD20, CD 123, CLL-1, RC2 and α₂β₁.

The compounds of the invention can be covalently bound to an agent, *e.g*., an antibody, using known conjugation techniques. For example, one end of a cross-linking agent can be bound to a chemical moiety (*e.g*., a carboxylic acid moiety) on the compound of the invention, while the other end of the cross-linking agent is bound to a site on an antibody. Such bifunctional cross-linking agents and their use in preparing conjugated antibodies are known and discussed in Hermanson, G.T. Bioconjugate Techniques, Academic, San Diego, CA, 1996; pp. 494-527.

### 6.1.1. SYNTHESES OF THE COMPOUNDS OF THE INVENTION

Compounds of the invention can be synthesized from known starting materials using known synthetic reactions. In the case of compounds of the formula I, certain of the compounds of the invention can be obtained by sulfonylation of the amino intermediate A-1 with a sulfonyl chloride of the formula B-I as shown in Scheme I below. Typically, such sulfonylations are conducted in an organic solvent, *e.g.*, CH₂Cl₂, in the presence of a base, *e.g*., pyridine.

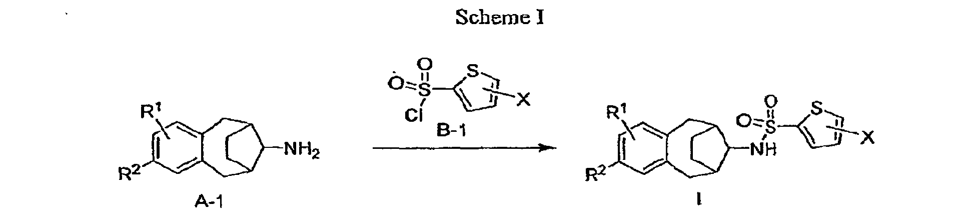

Compounds of the formula I, wherein E is NH, can be formed by the methods shown in Scheme II below. The sulphonamide intermediate A-2 is coupled for example with a carboxylic acid intermediate B-2 using a carbodiimide coupling agent (*e.g*., carbonyl diimidazole) in an organic solvent, *e.g*., tetrahydrofuran, to afford compounds of the formula IA-2.

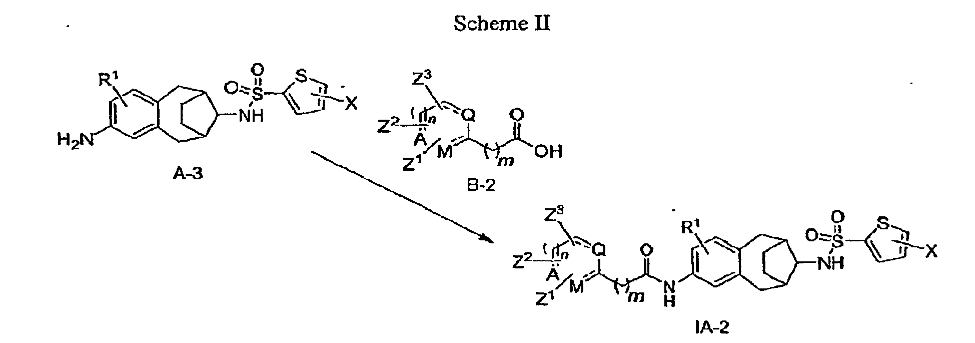

Other methods for synthesizing the compounds of the formula I, can be found, for instance in S.J. Lewis et al., Biorg. Med. Chem. Lett., 15 (2005), 373-378.

In some cases other compounds of the invention can be formed by the methods described in in U.S. Patent Application Publication Nos. 2004/0029862, 2004/0049038, 2004/0186147, 2005/0215602, 2005/0182111, 2005/0182109, 2005/0143369, and 2005/0119293, the disclosures, each of which is hereby incorporated by reference in its entirety. In addition, other synthetic methods are disclosed in International Publication Nos. WO 2001/70677, WO 2002/049038, WO 2004/186147, WO 2003/093253, WO 2003/093251, WO 2003/093252, WO 2003/093264, WO 2005/030731, WO 2005/014553, WO 2004/039800, and WO 2004/039370, the disclosures, each of which is hereby incorporated by reference in its entirety.

### 6.2. PHARMACEUTICAL COMPOSITIONS AND ROUTES OF ADMINISTRATION

The present invention provides compositions comprising a compound of the invention In particular, the invention provides a pharmaceutical composition comprising an effective amount of a compound of the invention and a pharmaceutically acceptable carrier or vehicle. In a specific embodiment, a pharmaceutical composition comprises an effective amount of a compound of the invention and a pharmaceutically acceptable carrier or vehicle. The pharmaceutical compositions are suitable for veterinary and/or human administration.

The pharmaceutical compositions of the present invention can be in any form that allows for the composition to be administered to a subject, said subject preferably being an animal, including, but not limited to a human, mammal, or non-human animal, such as a cow, horse, sheep, pig, fowl, cat, dog, mouse, rat, rabbit, guinea pig, etc., and is more preferably a mammal, and most preferably a human.

The compositions of the invention can be in the form of a solid, liquid or gas (aerosol). Typical routes of administration may include, without limitation, oral, topical, parenteral, sublingual, rectal, vaginal, ocular, intradermal, intratumoral, intracerebral, intrathecal, and intranasal. Parenteral administration includes subcutaneous injections, intravenous, intramuscular, intraperitoneal, intrapleural, intrasternal injection or infusion techniques. In a specific embodiment, the compositions are administered parenterally. In a more specific embodiment, the compositions are administered intravenously. Pharmaceutical compositions of the invention can be formulated so as to allow a compound of the invention to be bioavailable upon administration of the composition to a subject. Compositions can take the form of one or more dosage units, where, for example, a tablet can be a single dosage unit, and a container of a compound of the invention in aerosol form can hold a plurality of dosage units.

Materials used in preparing the pharmaceutical compositions can be non-toxic in the amounts used. It will be evident to those of ordinary skill in the art that the optimal dosage of the active ingredient(s) in the pharmaceutical composition will depend on a variety of factors. Relevant factors include, without limitation, the type of subject (*e.g*., human), the overall health of the subject, the type of cancer the subject is in need of treatment of, the use of the composition as part of a multi-drug regimen, the particular form of the compound of the invention, the manner of administration, and the composition employed.

The pharmaceutically acceptable carrier or vehicle may be particulate, so that the compositions are, for example, in tablet or powder form. The carrier(s) can be liquid, with the compositions being, for example, an oral syrup or injectable liquid. In addition, the carrier(s) can be gaseous, so as to provide an aerosol composition useful in, *e*.*g*., inhalatory administration.

The term "carrier" refers to a diluent, adjuvant or excipient, with which a compound of the invention is administered. Such pharmaceutical carriers can be liquids, such as water and oils, including those of petroleum, animal, vegetable or synthetic origin, such as peanut oil, soybean oil, mineral oil, sesame oil and the like. The carriers can be saline, gum acacia, gelatin, starch paste, talc, keratin, colloidal silica, urea, and the like. In addition, auxiliary, stabilizing, thickening, lubricating and coloring agents can be used. In one embodiment, when administered to a subject, the compounds of the invention and pharmaceutically acceptable carriers are sterile. Water is a preferred carrier when the compound of the invention is administered intravenously. Saline solutions and aqueous dextrose and glycerol solutions can also be employed as liquid carriers, particularly for injectable solutions. Suitable pharmaceutical carriers also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The present compositions, if desired, can also contain minor amounts of wetting or emulsifying agents, or pH buffering agents..

The composition may be intended for oral administration, and if so, the composition is preferably in solid or liquid form, where semi-solid, semi-liquid, suspension and gel forms are included within the forms considered herein as either solid or liquid.

As a solid composition for oral administration, the composition can be formulated into a powder, granule, compressed tablet, pill, capsule, chewing gum, wafer or the like form. Such a solid composition typically contains one or more inert diluents. In addition, one or more of the following can be present: binders such as ethyl cellulose, carboxymethylcellulose, microcrystalline cellulose, or gelatin; excipients such as starch, lactose or dextrins, disintegrating agents such as alginic acid, sodium alginate, Primogel, corn starch and the like; lubricants such as magnesium stearate or Sterotex; glidants such as colloidal silicon dioxide; sweetening agents such as sucrose or saccharin, a flavoring agent such as peppermint, methyl salicylate or orange flavoring, and a coloring agent.

When the pharmaceutical composition is in the form of a capsule, *e*.*g*., a gelatin capsule, it can contain, in addition to materials of the above type, a liquid carrier such as polyethylene glycol, cyclodextrin or a fatty oil.

The pharmaceutical composition can be in the form of a liquid, *e.g.*, an elixir, syrup, solution, emulsion or suspension. The liquid can be useful for oral administration or for delivery by injection. When intended for oral administration, a composition can comprise one or more of a sweetening agent, preservatives, dye/colorant and flavor enhancer. In a composition for administration by injection, one or more of a surfactant, preservative, wetting agent, dispersing agent, suspending agent, buffer, stabilizer and isotonic agent can also be included.

The liquid compositions of the invention, whether they are solutions, suspensions or other like form, can also include one or more of the following: sterile diluents such as water for injection, saline solution, preferably physiological saline, Ringer's solution, isotonic sodium chloride, fixed oils such as synthetic mono or digylcerides which can serve as the solvent or suspending medium, polyethylene glycols, glycerin, cyclodextrin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. A parenteral composition can be enclosed in an ampoule, a disposable syringe or a multiple-dose vial made of glass, plastic or other material. Physiological saline is a preferred adjuvant. An injectable composition is preferably sterile.

The pharmaceutical compositions comprise an effective amount of a compound of the invention such that a suitable dosage will be obtained (*see* Section 6.3.1, *infra*, for suitable dosages). Typically, this amount is at least 0.01% of a compound of the invention by weight of the composition. When intended for oral administration, this amount can be varied to be between 0.1% and 80% by weight of the composition. Preferred oral compositions can comprise from between 4% and 50% of the compound of the invention by weight of the composition. Preferred compositions of the present invention are prepared so that a parenteral dosage unit contains from between 0.01% and 2% by weight of the compound of the invention.

The compounds of the invention can be administered by any convenient route, for example, by infusion or bolus injection, by absorption through epithelial or mucocutaneous linings (*e.g*., oral mucosa, rectal and intestinal mucosa, etc.). Administration can be systemic or local. Various delivery systems are known, *e.g*., microparticles, microcapsules, capsules, etc., and may be useful for administering a compound of the invention. In certain embodiments, more than one compound of the invention is administered to a subject. Methods of administration may include, but are not limited to, oral administration and parenteral administration; parenteral administration including, but not limited to, intradermal, intramuscular, intraperitoneal, intravenous, subcutaneous; intranasal, epidural, sublingual, intranasal, intracerebral, intraventricular, intrathecal, intravaginal, transdermal, rectally, by inhalation, or topically to the ears, nose, eyes, or skin. The preferred mode of administration is left to the discretion of the practitioner, and will depend in-part upon the site of the medical condition (such as the site of cancer, a cancerous tumor or a pre-cancerous condition).

In one embodiment, the compounds of the invention are administered parenterally. In a specific embodiment, the compounds of the invention are administered intravenously.

In specific embodiments, it can be desirable to administer one or more compounds of the invention locally to the area in need of treatment. This can be achieved, for example, and not by way of limitation, by local infusion during surgery; topical application, *e.g.,* in conjunction with a wound dressing after surgery; by injection; by means of a catheter, by means of a suppository; or by means of an implant, the implant being of a porous, non-porous, or gelatinous material, including membranes, such as sialastic membranes, or fibers. In one embodiment, administration can be by direct injection at the site (or former site) of a cancer, tumor, or precancerous tissue. In certain embodiments, it can be desirable to introduce one or more compounds of the invention into the central nervous system by any suitable route, including intraventricular and intrathecal injection. Intraventricular injection can be facilitated by an intraventricular catheter, for example, attached to a reservoir, such as an Ommaya reservoir.

Pulmonary administration can also be employed, *e.g*., by use of an inhaler or nebulizer, and formulation with an aerosolizing agent, or via perfusion in a fluorocarbon or synthetic pulmonary surfactant. In certain embodiments, the compounds of the invention can be formulated as a suppository, with traditional binders and carriers such as triglycerides.

In yet another embodiment, the compounds of the invention can be delivered in a controlled release system. In one embodiment, a pump can be used (*see* Sefton, CRC Crit. Ref. Biomed. Eng. 1987, 14, 201; Buchwald et al., Surgery 1980, 88: 507; Saudek et al., N. Engl. J. Med. 1989, 321: 574). In another embodiment, polymeric materials can be used (*see* Medical Applications of Controlled Release, Langer and Wise (eds.). CRC Pres., Boca Raton, FL, 1974; Controlled Drug Bioavailability, Drug Product Design and Performance, Smolen and Ball (eds.), Wiley, New York, 1984; Ranger and Peppas, J. Macromol. Sci. Rev. Macromol. Chem. 1983, 23, 61; *see also* Levy et al., Science 1985, 228, 190; During et al., Ann. Neurol., 1989, 25, 351; Howard et al., J. Neurosurg., 1989, 71, 105). In yet another embodiment, a controlled-release system can be placed in proximity of the target of the compounds of the invention, *e.g*., the brain, thus requiring only a fraction of the systemic dose (*see*, *e.g.,* Goodson, in Medical Applications of Controlled Release, supra, vol. 2, 1984, pp. 115-138). Other controlled-release systems discussed in the review by Langer (Science 1990, 249, 1527-1533) can be used.

In another embodiment, polymeric materials can be used to achieve controlled or sustained release of the compounds of the invention (*see*, *e.g*., U.S. Pat. No. 5,679,377; U.S. Pat. No. 5,916,59.7; U.S. Pat. No. 5,912,015; U.S. Pat. No. 5,989,463; U.S. Pat. No. 5,128,326; PCT Publication No. WO 99/15154; and PCT Publication No. WO 99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. In a preferred embodiment, the polymer used in a sustained release formulation is inert, free of leachable impurities, stable on storage, sterile, and biodegradable.

The present compositions can take the form of solutions, suspensions, emulsion, tablets, pills, pellets, capsules, capsules containing liquids, powders, sustained-release formulations, suppositories, emulsions, aerosols, sprays, suspensions, or any other form suitable for use. In one embodiment, the pharmaceutically acceptable carrier is a capsule (*see e*.*g*., U.S. Patent No. 5,698,155). Other examples of suitable pharmaceutical carriers are described in Remingion's Pharmaceutical Sciences by E.W. Martin.

Sustained or directed release compositions that can be formulated include, but are not limited to, compounds of the invention protected with differentially degradable coatings, *e*.*g*., by microencapsulation, multiple coatings, etc. It is also possible to freeze-dry the compositions and use the lyophilizates obtained, for example, for the preparation of products for injection.

In a preferred embodiment, the compounds of the invention are formulated in accordance with routine procedures as a pharmaceutical composition adapted for intravenous administration to animals, particularly human beings. Typically, the carriers or vehicles for intravenous administration are sterile isotonic aqueous buffer solutions. Where necessary, the compositions can also include a solubilizing agent. Compositions for intravenous administration can optionally comprise a local anaesthetics such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachet indicating the quantity of active agent. Where a compound of the invention is to be administered by infusion, it can be dispensed, for example, with an infusion bottle containing sterile pharmaceutical grade water or saline. Where the compound of the invention is administered by injection, an ampoule of sterile water for injection or saline can be provided so that the ingredients can be mixed prior to administration.

Compositions for oral delivery can be in the form of tablets, lozenges, aqueous or oily suspensions, granules, powders, emulsions, capsules, syrups, or elixirs, for example. Orally administered compositions can contain one or more optional agents, for example, sweetening agents such as fructose, aspartame or saccharin; flavoring agents such as peppermint, oil of wintergreen, or cherry; coloring agents; and preserving agents, to provide a pharmaceutically palatable preparation. Moreover, where in tablet or pill form, the compositions can be coated to delay disintegration and absorption in the gastrointestinal tract thereby providing a sustained action over an extended period of time. Selectively permeable membranes surrounding an osmotically active driving complex are also suitable for orally administered compositions of the invention. In these later platforms, fluid from the environment surrounding the capsule is imbibed by the driving complex, which swells to displace the agent or agent composition through an aperture, These delivery platforms can provide an essentially zero order delivery profile as opposed to the spiked profiles of immediate release formulations. A time-delay material such as glycerol monostearate or glycerol stearate can also be used. Oral compositions can include standard carriers such as mannitol, lactose, starch, magnesium stearate, sodium saccharine, cellulose, magnesium carbonate, etc. Such carriers are preferably of pharmaceutical grade.

The pharmaceutical compositions of the invention can be intended for topical administration, in which case the carrier can be in the form of a solution, emulsion, ointment or gel base. The base, for example, can comprise one or more of the following: petrolatum, lanolin, polyethylene glycols, beeswax, mineral oil, diluents such as water and alcohol, and emulsifiers and stabilizers. Thickening agents can be present in a composition for topical administration. If intended for transdermal administration, the composition can be in the form of a transdermal patch or an iontophoresis device. Topical formulations can comprise a concentration of a compound of the invention of from between 0.01% and 10% w/v (weight per unit volume of composition).

The compositions can include various materials that modify the physical form of a solid or liquid dosage unit. For example, the composition can include materials that form a coating shell around the active ingredients. The materials that form the coating shell are typically inert, and can be selected from, for example, sugar, shellac, and other enteric coating agents. Alternatively, the active ingredients can be encased in a gelatin capsule.

The compositions can consist of gaseous dosage units, *e.g*., it can be in the form of an aerosol. The term aerosol is used to denote a variety of systems ranging from those of colloidal nature to systems consisting of pressurized packages. Delivery can be by a liquefied or compressed gas or by a suitable pump system that dispenses the active ingredients. Aerosols of the compositions can be delivered in single phase, bi-phasic, or tri- phasic systems in order to deliver the composition. Delivery of the aerosol includes the necessary container, activators, valves, subcontainers, spacers and the like, which together can form a kit. Preferred aerosols can be determined by one skilled in the art, without undue experimentation.

Whether in solid, liquid or gaseous form, the compositions of the present invention can comprise an additional active agent selected from among those including, but not limited to, an additional prophylactic agent, an additional therapeutic agent, an antiemetic agent, a hematopoietic colony stimulating factor, an adjuvant therapy, a vaccine or other immune stimulating agent, an antibody/antibody fragment-based agent, an anti-depressant and an analgesic agent. For instance in a particular embodiment, the pharmaceutical composition comprises a compound of the invention, an additional agent, and a pharmaceutically acceptable acceptable carrier or vehicle.

The pharmaceutical compositions can be prepared using methodology well known in the pharmaceutical art. For example, a composition intended to be administered by injection can be prepared by combining a compound of the invention with water so as to form a solution. A surfactant can be added to facilitate the formation of a homogeneous solution or suspension. Surfactants are complexes that can non-covalently interact with a compound of the invention so as to facilitate dissolution or homogeneous suspension of the compound of the invention in the aqueous delivery system.

In one embodiment, the pharmaceutical compositions of the present invention may comprise one or more known therapeutically active agents.

### 6.3. PROPHYLACTIC AND THERAPEUTIC USES

Cancer or a neoplastic disease, including, but not limited to, neoplasms, tumors, metastases, or any disease or disorder characterized by uncontrolled cell growth, can be treated, suppressed, delayed, managed, inhibited or prevented by administering to a subject in need thereof a prophylactically effective regimen or a therapeutically effective regimen, the regimen comprising administering to the patient a compound of the invention. The invention as it applies to cancer encompasses the treatment, suppression, delaying, management, inhibiting of growth and/or progression, and prevention of cancer or neoplastic disease as described herein.

In one embodiment, the compounds of the invention are administered as monotherapy for the prevention, treatment, and/or management of cancer.

One aspect of the invention relates to a method of preventing, treating, and/or managing cancer in a patient (*e.g*., a human patient), the method comprising administering to the patient a prophylactically effective regimen or a therapeutically effective regimen, the regimen comprising administering to the patient a compound of the invention or a composition of the invention, wherein the patient has been diagnosed with cancer.

In one embodiment, the cancer is a hematologic cancer. For instance, the cancer can be leukemia, lymphoma or myeloma- In another embodiment, the cancer is a solid turnor.

In one embodiment of this aspect, the patient has received or is receiving another therapy. In another embodiment of this aspect, the patient has not previously received a therapy for the prevention, treatment, and/or management of the cancer.

The medical practitioner can diagnose the patient using any of the conventional cancer screening methods including, but not limited to physical examination (*e.g*., prostate examination, breast examination, lymph nodes examination, abdominal examination, skin surveillance), visual methods (*e.g*., colonoscopy, bronchoscopy, endoscopy), PAP smear analyses (cervical cancer), stool guaiac analyses, blood tests (*e.g*., complete blood count (CBC) test), blood chemistries including liver function tests, prostate specific antigen (PSA) test, carcinoembryonic antigen (CEA) test, cancer antigen (CA)-125 test, alpha-fetoprotein (AFP)), karyotyping analyses, bone marrow analyses (*e.g*., in cases of hematological malignancies), histology, cytology, a sputum analysis and imaging methods (*e.g*., computed tomography (CT), magnetic resonance imaging (MRI), ultrasound, X-ray imaging, mammograph imaging, bone scans).

Another aspect of the invention relates to a method of preventing, treating, and/or managing a solid tumor in a patient (*e.g*., a human patient), the method comprising administering to a patient in need thereof a prophylactically effective regimen or a therapeutically effective regimen, the regimen comprising administering to the patient a compound or composition of the invention wherein the patient has been diagnosed with a solid tumor, and wherein the patient has undergone a primary therapy to reduce the bulk of the tumor. The primary therapy to reduce the tumor bulk size is preferably a therapy other than a compound or composition of the invention. In specific embodiment of this aspect, the solid tumor is fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, colorectal cancer, kidney cancer, pancreatic cancer, bone cancer, breast cancer, ovarian cancer, prostate cancer, esophageal cancer, stomach cancer, oral cancer, nasal cancer, throat cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, uterine cancer, testicular cancer, small cell lung carcinoma, bladder carcinoma, lung cancer, epithelial carcinoma, glioma, glioblastoma multiforme, astrocytoma, medulloblastoma, craniopharyngioma, ependymoma, pinealoma, hemangioblastoma, acoustic neuroma, oligodendroglioma, meningioma, skin cancer, melanoma, neuroblastoma, retinoblastoma, embryonal brain tumor, PNET, or choroid plexus tumor.

Another aspect of the invention relates to a method of preventing, treating, and/or managing cancer, the method comprising administering to a patient in need thereof a prophylactically effective regimen or a therapeutically effective regimen, the regimen comprising administering to the patient a compound of the invention (as described above), or a pharmaceutically acceptable salt thereof wherein the patient received another therapy. In some embodiments, the prior therapy is, for example, chemotherapy, radioimmunotherapy, toxin therapy, prodrug-activating enzyme therapy, antibody therapy, surgical therapy, immunotherapy, radiation therapy, targeted therapy or any combination thereof.

In some embodiments, the prior therapy has failed in the patient. In some embodiments, the therapeutically effective regimen comprising administration of a compound of the invention is administered to the patient immediately after patient has undergone the prior therapy. For instance, in certain embodiments, the outcome of the prior therapy may be unknown before the patient is administered a compound of the invention.

Another aspect of the invention relates to a method of preventing cancer in a patient (*e*.*g*., a human patient), the method comprising administering to a patient in need thereof a prophylactically effective regimen or a therapeutically effective regimen, the regimen comprising administering to the patient a compound or composition of the invention, wherein the cancer in the patient has entered remission. In some embodiments of this aspect, through administration of a prophylactically effective regimen or a therapeutically effective regimen, the medical practitioner can effectively cure the cancer, or prevent its recurrence.

Another aspect of the invention relates to a method of preventing, treating, and/or managing a solid tumor in a patient (*e*.*g*., a human patient), the method comprising administering to a patient in need thereof a prophylactically effective regimen or a therapeutically effective regimen, the regimen comprising administering to the patient a compound or composition of the invention, wherein the compound or composition of the invention is administered at a dose that is lower than the maximum tolerated dose (MTD) over a period of three months, four months, six months, nine months, 1 year, 2 years, 3 years, 4 years or more.

Another aspect of the invention relates to a method of preventing, treating, and/or managing a solid tumor in a patient (*e.g.*, a human patient), the method comprising administering to a patient in need thereof a prophylactically effective regimen or a therapeutically effective regimen, the regimen comprising administering to the patient a compound or composition of the invention, wherein the compound or composition of the invention is administered at a dose that is lower than the human equivalent dosage (HED) of the no observed adverse effect level (NOAEL) over a period of three months, four months, six months, nine months, 1 year, 2 years, 3 years, 4 years or more. The NOAEL, as determined in animal studies, is useful in determining the maximum recommended starting dose for human clinical trials. For instance, the NOAELs can be extrapolated to determine human equivalent dosages. Typically, such extrapolations between species are conducted based on the doses that are normalized to body surface area (*i*.*e*., mg/m²). In specific embodiments, the NOAELs are determined in mice, hamsters, rats, ferrets, guinea pigs, rabbits, dogs, primates, primates (monkeys, marmosets, squirrel monkeys, baboons), micropigs or minipigs. For a discussion on the use of NOAELs and their extrapolation to determine human equivalent doses, *see* Guidance for Industry Estimating the Maximum Safe Starting Dose in Initial Clinical Trials for Therapeutics in Adult Healthy Volunteers, U.S. Department of Health and Human Services Food and Drug Administration Center for Drug Evaluation and Research (CDER), Pharmacology and Toxicology, July 2005.

Cancer stem cells may be more sensitive than tumor bulk to Notch pathway inhibition. Therefore, in one embodiment, the dose of Notch pathway inhibitor that is administered to the patient may be effective to stabilize, reduce, impair, or eliminate the cancer stem cells with resulting tumor shrinkage occuring less acutely than had the administered dose been higher. In this embodiment, the effect of the drug on the cancer stem cells will ultimately lead to reduction in tumor size over time, since the tumor bulk cells (which are the progeny cells of the cancer stem cells and have a finite lifespan) will gradually die. In this embodiment, the tumor shrinkage is a result of the drug's impairment on the cancer stem cells and their ability to replenish the tumor bulk cells. In contrast, a higher dose of Notch pathway inhibitor would cause a more acute tumor shrinkage due to a direct effect on the tumor bulk. However, given that a lower dose may be less toxic, a prolonged low dose regimen may ultimately be more effective at targeting cancer stem cells and thus providing greater long term benefit.

In certain embodiments of these aspects, the regimens comprise administering a prophylactically effective regimen and/or a therapeutically effective regimen, wherein the regimen results in a reduction in the cancer stem cell population in the patient. In one embodiment, the patient undergoing the regimen is monitored to determine whether the regimen has resulted in a reduction in the cancer stem cell population in the patient.

Typically, the monitoring of the cancer stem cell population is conducted by detecting the number, amount, portion or percentage of cancer stem cells in a specimen extracted from the patient. Methods of detecting the number or amount of cancer stem cells in a specimen are described *infra* in Section 6.4. This monitoring step is typically performed at least 1, 2, 4, 6, 8,10,12, 14,15,16,18,20, or 30 days after the patient begins receiving the regimen.

In some embodiments, the specimen may be a blood specimen, wherein the number or amount of cancer stem cells per unit of volume (*e.g*., 1 mL) or other measured unit (*e*.*g*., per unit field in the case of a histological analysis) is quantitated. In certain embodiments, the cancer stem cell population is determined as a portion (*e*.*g*., a percentage) of the cancer cells present in the blood specimen, as a subset of the cancer cells present in the blood specimen, or as a subset of a subset of the cancer cells present in the blood specimen. The cancer stem cell population, in other embodiments, can be determined as a percentage of the total blood cells.

In other embodiments, the specimen extracted from the patient is a tissue specimen (*e*.*g*., a biopsy extracted from suspected cancerous tissue), where the number or amount of cancer stem cells can be measured, for example, on the basis of the number or amount of cancer stem cells per unit area, volume, or weight of the tissue. In certain embodiments, the cancer stem cell population is determined as a portion (*e*.*g*., a percentage) of the cancer cells present in the tissue, as a subset of the cancer cells present in the tissue, or as a subset of a subset of the cancer cells present in the tissue.

The number or amount of cancer stem cells in the extracted specimen can be compared with the numbers or amounts of cancer stem cells measured in reference samples to assess the efficacy of the regimen, and the amelioration of the cancer under therapy. In one embodiment, the reference sample is a specimen extracted from the patient undergoing therapy, wherein the specimen is extracted from the patient at an earlier time point (*e.g*., prior to receiving the regimen, as a baseline reference sample, or at an earlier time point while receiving the therapy). In another embodiment, the reference sample is extracted from a healthy, noncancer-afflicted patient.

In other embodiments the cancer stem cell population in the extracted specimen can be compared with a predetermined reference range. In a specific embodiment, the predetermined reference range is based on the number or amount of cancer stem cells obtained from a population(s) of patients suffering from the same type of cancer as the patient undergoing the therapy.

If the reduction in the cancer stem cell population is determined to be too small upon comparing the cancer stem cell population in the specimen extracted from the patient undergoing the regimen with the reference specimen, then the medical practitioner has a number of options to adjust the regimen. For instance, the medical practitioner can then increase either the dosage of the compound or composition of the invention administered, the frequency of the administration, the duration of administration, or any combination thereof. In a specific embodiment, after the determination is made, a second effective amount of a compound or composition of the invention can be administered to the patient.

In other embodiments, the regimens comprise administering a prophylactically effective regimen and/or a therapeutically effective regimen, wherein the regimen results in a reduction in the cancer cell population in the patient. In one embodiment, the patient undergoing the regimen is monitored to determine whether the regimen has resulted in a reduction in the cancer cell population in the patient.

Typically, the monitoring of the cancer cell population is conducted by detecting the number or amount of cancer cells in a specimen extracted from the patient. Methods of detecting the number or amount of cancer cells in a specimen are described *infra* in Section 6.5. This monitoring step is typically performed at least 1, 2, 4, 6, 8, 10, 12, 14, 15, 16, 18, 20, or 30 days after the patient begins receiving the regimen.

In some embodiments, the specimen may be a blood specimen, wherein the number or amount of cancer cells per unit of volume (*e.g.*, 1 mL) or other measured unit (*e*.*g*., per unit field in the case of a histological analysis) is quantitated. The cancer cell population, in certain embodiments, can be determined as a percentage of the total blood cells.

In other embodiments, the specimen extracted from the patient is a tissue specimen (*e.g.,* a biopsy extracted from suspected cancerous tissue), where the number or amount of cancer cells can be measured, for example, on the basis of the number or amount of cancer cells per unit weight of the tissue.

The number or amount of cancer cells in the extracted specimen can be compared with the numbers or amounts of cancer cells measured in reference samples to assess the efficacy of the regimen and amelioration of the cancer under therapy. In one embodiment, the reference sample is a specimen extracted from the patient undergoing therapy, wherein the specimen from the patient is extracted at an earlier time point (*e*.*g*., prior to receiving the regimen, as a baseline reference sample, or at an earlier time point while receiving the therapy). In another embodiment, the reference sample is extracted from a healthy, noncancer-afflicted patient.

In other embodiments the cancer cell population in the extracted specimen can be compared with a predetermined reference range. In a specific embodiment, the predetermined reference range is based on the number or amount of cancer cells obtained from a population(s) of patients suffering from the same type of cancer as the patient undergoing the therapy.

If the reduction in the cancer cell population is judged too small upon comparing the number, amount, or percentage of cancer cells in the specimen extracted from the patients undergoing therapy with the reference specimen, then the medical practitioner has a number of options to adjust the therapeutic regimen. For instance, the medical practitioner can then either increase the dosage of the compound or composition of the invention administered, the frequency of the administration, the duration of administration, or any combination thereof. In a specific embodiment, after the determination is made, a second effective amount of a compound or composition of the invention can be administered to the patient.

In other embodiments, the regimens comprise administering a compound or composition of the invention, wherein the regimen results in a reduction in the number, amount, or percentage of cancer stem cells and a reduction in the number, amount, or percentage of cancer cells in the patient.

### 6.3.1. DOSAGE REGIMENS

The amount of a compound or pharmaceutical composition of the invention used in the prophylactic and/or therapeutic regimens which will be effective in the prevention, treatment, and/or management of cancer can be determined by methods disclosed herein. The frequency and dosage will vary also according to factors specific for each patient depending on the specific compounds administered, the severity of the cancerous condition, the route of administration, as well as age, body, weight, response, and the past medical history of the patient. For example, the dosage of a compound of the invention which will be effective in the treatment, prevention, and/or management of cancer can be determined by administering the compound to an animal model such as, *e.g.,* the animal models disclosed herein or known to those skilled in the art. *See* Section 6.7.2, *infra.* In addition, *in vitro* assays may optionally be employed to help identify optimal dosage ranges. *See* Section 6.7.1, *infra.*

In some embodiments, the prophylactic and/or therapeutic regimens comprise titrating the dosages administered to the patient so as to achieve a specified measure of therapeutic efficacy. Such measures include a reduction in the cancer stem cell population in the patient and/or a reduction in the cancer cell population in the patient.

In some embodiments, the prophylactic and/or therapeutic regimens comprise administering dosages of a compound or pharmaceutical composition of the invention that are effective to reduce in the cancer stem cell population. Methods that can be used to determine the reduction in the cancer stem cell population in a patient undergoing therapy are discussed *infra* in Section 6.4.

In certain embodiments, the dosage of the compound of the invention in the prophylactic and/or therapeutic regimen is adjusted so as to achieve a reduction in the number or amount of cancer stem cells found in a test specimen extracted from a patient after undergoing the therapeutic regimen, as compared with a reference sample. Here, the reference sample is a specimen extracted from the patient undergoing therapy, wherein the specimen is extracted from the patient at an earlier time point. In one embodiment, the reference sample is a specimen extracted from the same patient, prior to receiving the prophylactic or therapeutic regimen. In specific embodiments, the number or amount of cancer stem cells in the test specimen is at least 2%, 5%, 10%, 15%, 20%, 30%, 40%, 5%, 60%, 70%, 80%, 90%, 95% or 99% lower than in the reference sample.

In other embodiments, the dosage of the compound of the invention in the prophylactic and/or therapeutic regimen is adjusted so as to achieve a reduction in the number or amount of cancer stem cells found in a test specimen extracted from a patient after undergoing the prophylactic and/or therapeutic regimen, as compared with a reference sample,
wherein the reference sample specimen is extracted from a healthy, noncancer-afflicted patient. In specific embodiments, the number or amount of cancer stem cells in the test specimen is at least within 60%, 50%, 40%, 30%, 20%, 15%, 10%, 5%, or 2% of the number or amount of cancer stem cells in the reference sample.

In some embodiments, the dosage of the compound of the invention in the prophylactic and/or therapeutic regimen is adjusted so as to achieve a number or amount of cancer stem cells that falls within a predetermined reference range. In these embodiments, the number or amount of cancer stem cells in a test specimen is compared with a predetermined reference range. In a specific embodiment, the predetermined reference range is based on the number or amount of cancer stem cells obtained from a population(s) of patients suffering from the same type of cancer as the patient undergoing the therapy.

In some embodiments, the dosage of the compound of the invention is adjusted so as to achieve suppression of Notch pathway signalling by 20%,30%, 40%, 50%, 60%, 70%, 80%, 90%, or 100% in either the entire tumor cell population, or in the cancer stem cell fraction.

In some embodiments, the prophylactic and/or therapeutic regimens comprise administering dosages of a compound or pharmaceutical composition of the invention that are effective to reduce the cancer cell population. Methods that can be used to determine the the cancer cell population in a patient undergoing treatment are discussed *infra* in Section 6.5.

In certain embodiments, the dosage of the compound of the invention in the prophylactic and/or therapeutic regimen is adjusted so as to achieve a reduction in the number or amount of cancer cells found in a test specimen extracted from a patient after undergoing the prophylactic and/or therapeutic regimen, as compared with a reference sample Here, the reference sample is a specimen extracted from the patient undergoing therapy, wherein the specimen is extracted from the patient at an earlier time point. In one embodiment, the reference sample is a specimen extracted from the same patient, prior to receiving the prophylactic and/or therapeutic regimen. In specific embodiments, the number or amount of cancer cells in the test specimen is at least 2%, 5%, 10%, 15%, 20%, 30%, 40%, 50%, 60%, 70%, 80%, 90%, 95% or 99% lower than in the reference sample.

In some embodiments, the dosage of the compound of the invention in the prophylactic and/or therapeutic regimen is adjusted so as to achieve a number or amount of cancer cells that falls within a predetermined reference range. In these embodiments, the number or amount of cancer cells in a test specimen is compared with a predetermined reference range.

In other embodiments, the dosage of the compound of the invention in prophylactic and/or therapeutic regimen is adjusted so as to achieve a reduction in the number or amount of cancer cells found in a test specimen extracted from a patient after undergoing the prophylactic and/or therapeutic regimen, as compared with a reference sample, wherein the reference sample is a specimen is extracted from a healthy, noncancer-afflicted patient. In specific embodiments, the number or amount of cancer cells in the test specimen is at least within 60%, 50%, 40%, 30%, 20%, 15%, 10%, 5%, or 2% of the number or amount of cancer cells in the reference sample.

In treating certain human patients having solid tumors, extracting multiple tissue specimens from a suspected tumor site may prove impracticable. In these embodiments, the dosage of the compounds of the invention in the prophylactic and/or therapeutic regimen for a human patient is extrapolated from doses in animal models that are effective to reduce the cancer stem cell population in those animal models. In the animal models, the prophylactic and/or therapeutic regimens are adjusted so as to achieve a reduction in the number or amount of cancer stem cells found in a test specimen extracted from an animal after undergoing the prophylactic and/or therapeutic regimen, as compared with a reference sample. The reference sample can be a specimen extracted from the same animal, prior to receiving the prophylactic and/or therapeutic regimen. In specific embodiments, the number or amount of cancer stem cells in the test specimen is at least 2%, 5%, 10%, 15%, 20%, 30%, 40%, 50% or 60% lower than in the reference sample. The doses effective in reducing the number or amount of cancer stem cells in the animals can be normalized to body surface area (*e.g.,* mg/m²) to provide an equivalent human dose.

The prophylactic and/or therapeutic regimens disclosed herein comprise administration of compounds of the invention or pharmaceutical compositions thereof to the patient in a single dose or in multiple doses (*e.g.,* 1, 2, 3,4,5, 6, 7, 8, 10, 15, 20, or more doses).

In one embodiment, the prophylactic and/or therapeutic regimens comprise administration of the compounds of the invention or pharmaceutical compositions thereof in multiple doses. When administered in multiple doses, the compounds or pharmaceutical compositions are administered with a frequency and in an amount sufficient to prevent, treat, and/or manage the condition. In one embodiment, the frequency of administration ranges from once a day up to about once every eight weeks. In another embodiment, the frequency of administration ranges from about once a week up to about once every six weeks. In another embodiment, the frequency of administration ranges from about once every three weeks up to about once every four weeks.

In some embodiments of the invention, the dosage of a compound of the invention or pharmaceutical composition thereof administered is at least 1.5, 1.6, 1.8, 2, 2.5, 3, 4, 5, 6, 7, 8, 10, 20, 30, 40, 50, 60, 70, 80, 90 or 100 times lower than the maximum tolerated dose (MTD) over a period of three months, four months, six months, nine months, 1 year, 2 years, 3 years, 4 years or more.

In some embodiments of the invention, the dosage of a compound of the invention or pharmaceutical composition thereof administered is at least 1.1, 1.2, 1.3, 1.4, 1.5, 1.6,1.8, 2, 2.5, 3, 4, 5, 6, 7, 8, 10, 20, 30, 40, 50, 60, 70, 80, 90 or 100 times lower than the human equivalent dose (HED) of the no observed adverse effect level (NDAEL) over a period of three months, four months, six months, nine months, 1 year, 2 years, 3 years, 4 years or more. In specific embodiments, the NOAELs are determined in mice, hamsters, rats, ferrets, guinea pigs, rabbits, dogs, primates, primates (monkeys, marmosets, squirrel monkeys, baboons), micropigs or minipigs *See* the discussion regarding the NOAELs and HEDs in Section 6.3, *supra.*

Generally, the dosage of a compound of the invention administered to a subject to prevent, treat, and/or manage cancer is in the range of 0.01 to 500 mg/kg, and more typically, in the range of 0.1 mg/kg to 100 mg/kg, of the subject's body weight. In one embodiment, the dosage administered to a subject is in the range of 0.1 mg/kg to 50 mg/kg, or 1 mg/kg to 50 mg/kg, of the subject's body weight, more preferably in the range of 0.1 mg/kg to 25 mg/kg, or 1 mg/kg to 25 mg/kg, of the patient's body weight.

In a specific embodiment, the dosage of a compound of the invention administered to a subject to prevent, treat, and/or manage cancer in a patient is 500 mg/kg or less, preferably 250 mg/kg or less, 100 mg/kg or less, 95 mg/kg or less, 90 mg/kg or less, 85 mg/kg or less, 80 mg/kg or less, 75 mg/kg or less, 70 mg/kg or less, 65 mg/kg or less, 60 mg/kg or less, 55 mg/kg or less, 50 mg/kg or less, 45 mg/kg or less, 40 mg/kg or less, 35 mg/kg or less, 30 mg/kg or less, 25 mg/kg or less, 20 mg/kg or less, 15 mg/kg or less, 10 mg/kg or less, 5 mg/kg or less, 2.5 mg/kg or less, 2 mg/kg or less, 1.5 mg/kg or less, or 1 mg/kg or less of a patient's body weight.

In another specific embodiment, the dosage of a compound of the invention administered to a subject to prevent, treat, and/or manage cancer in a patient is a unit dose of 0.1 to 50 mg, 0.1 mg to 20 mg, 0.1 mg to 15 mg, 0.1 mg to 12 mg, 0.1 mg to 10 mg, 0.1 mg to 8 mg, 0.1 mg to 7 mg, 0.1 mg to 5 mg, 0.1 to 2.5 mg, 0.25 mg to 20 mg, 0.25 to 15 mg, 0.25 to 12 mg, 0.25 to 10 mg, 0.25 to 8 mg, 0.25 mg to 7 m g, 0.25 mg to 5 mg, 0.5 mg to 2.5 mg, 1 mg to 20 mg, 1 mg to 15 mg, 1 mg to 12 mg, 1 mg to 10 mg, 1 mg to 8 mg, 1 mg to 7 mg, 1 mg to 5 mg, or 1 mg to 2.5 mg.

In a specific embodiment, the dosage of a compound of the invention administered to a subject to prevent, treat, and/or manage cancer in a patient is in the range of 0.01 to 10 g/m², and more typically, in the range of 0.1 g/m² to 7.5 g/m², of the subject's body weight. In one embodiment, the dosage administered to a subject is in the range of 0.5 g/m² to 5 g/m², or 1 g/m² to 5 g/m² of the subject's body's surface area.

In other embodiments, the prophylactic and/or therapeutic regimen comprises administering to a patient one or more doses of an effective amount of a compound of the invention, wherein the dose of an effective amount achieves a plasma level of at least 0.1 µg/mL, at least 0.5 µg/mL, at least 1 µg/mL, at least 2 µg/mL, at least 5 µg/mL, at least 6 µg/mL, at least 10 µg/mL, at least 15 µg/mL, at least 20 µg/mL, at least 25 µg/mL, at least 50 µg/mL, at least 100 µg/mL, at least 125 µg/mL, at least 150 µg/mL, at least 175 µg/mL, at least 200 µg/mL, at least 225 µg/mL, at least 250 µg/mL, at least 275 µg/mL, at least 300 µg/mL, at least 325 µg/mL, at least 350 µg/mL, at least 375 µg/mL, or at least 400 µg/mL of the compound of the invention.

In other embodiments, the prophylactic and/or therapeutic regimen comprises administering to a human patient in need thereof a therapeutically effective regimen, the regimen comprising administering a gamma-secretase inhibitor for at least 1 day, 1 month, 2 months, 3 months, 4 months, 5 months, 6 months, 7 months, 8 months, 9 months, 10 months, 11 months, 12 months, 15 months, 18 months, 24 months or 36 months or from 1 day to 36 months, from 1 month to 24 months or from 1 month to 12 months, wherein the regimen results in a reduction in the cancer cell population in the patient.

In other embodiments, the prophylactic and/or therapeutic regimen comprises administering to a patient a plurality of doses of an effective amount of a compound of the invention, wherein the plurality of doses maintains a plasma level of at least 0.1 µg/mL, at least 0.5 µg/mL, at least 1 µg/mL, at least 2 µg/mL, at least 5 µg/mL, at least 6 µg/mL, at least 10 µg/mL, at least 15 µg/mL, at least 20 µg/mL, at least 25 µg/mL, at least 50 µg/mL, at least 100 µg/mL, at least 125 µg/mL, at least 150 µg/mL, at least 175 µg/mL, at least 200 µg/mL, at least 225 µg/mL, at least 250 µg/mL, at least 275 µg/mL, at least 300 µg/mL, at least 325 µg/mL, at least 350 µg/mL, at least 375 µg/mL, or at least 400 µg/mL of the compound of the invention for at least 1 day, 1 month, 2 months, 3 months, 4 months, 5 months, 6 months, 7 months, 8 months, 9 months, 10 months, 11 months, 12 months, 15 months, 18 months, 24 months or 36 months.

In some embodiments, the prophylactic and/or therapeutic regimen comprises administration of a compound of the invention in combination with one or more additional anticancer therapeutics. See Section 6.3.2. Preferably, the dosages of the one or more additional anticancer therapeutics used in the combination therapy is lower than those which have been or are currently being used to prevent, treat, and/or manage cancer. The recommended dosages of the one or more additional anticancer therapeutics currently used for the prevention, treatment, and/or management of cancer can be obtained from any reference in the art including, but not limited to, Hardman et al., eds., Goo&nan & Gilman's The Pharmacological Basis Of Basis Of Therapeutics, 10th ed., Mc-Graw-Hill, New York, 2001; Physician's Desk Reference (60th ed., 2006), which is incorporated herein by reference in its entirety.

The compound of the invention and the one or more additional anticancer therapeutics can be administered separately, simultaneously, or sequentially. In various embodiments, the compound of the invention and the additional anticancer therapeutic are administered less than 5 minutes apart, less than 30 minutes apart, less than 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In preferred embodiments, two or more anticancer therapeutics are administered within the same patient visit.

In certain embodiments, the compound of the invention and the additional anticancer therapeutic are cyclically administered. Cycling therapy involves the administration of one anticancer therapeutic for a period of time, followed by the administration of a second anticancer therapeutic for a period of time and repeating this sequential administration, *i*.*e*., the cycle, in order to reduce the development of resistance to one or both of the anticancer therapeutics, to avoid or reduce the side effects of one or both of the anticancer therapeutics, and/or to improve the efficacy of the therapies.

In a preferred embodiment, the anticancer therapeutics are administered concurrently to a subject in separate compositions. The combination anticancer therapeutics of the invention may be administered to a subject by the same or different routes of administration.

In a specific embodiment, cycling therapy involves the administration of a first anticancer therapeutic for a period of time, followed by the administration of a second anticancer therapeutic for a period of time, optionally, followed by the administration of a third anticancer therapeutic for a period of time and so forth, and repeating this sequential administration, *i*.*e*., the cycle in order to reduce the development of resistance to one of the anticancer therapeutics, to avoid or reduce the side effects of one of the anticancer therapeutics, and/or to improve the efficacy of the anticancer therapeutics.

When a compound of the invention and the additional anticancer therapeutic are administered to a subject concurrently, the term "concurrently" is not limited to the administration of the anticancer therapeutics at exactly the same time, but rather, it is meant that they are administered to a subject in a sequence and within a time interval such that they can act together (*e*.*g*., synergistically to provide an increased benefit than if they were administered otherwise). For example, the anticancer therapeutics may be administered at the same time or sequentially in any order at different points in time; however, if not administered at the same time, they should be administered sufficiently close in time so as to provide the desired therapeutic effect, preferably in a synergistic fashion. The combination anticancer therapeutics of the invention can be administered separately, in any appropriate form and by any suitable route. When the components of the combination anticancer therapeutics are not administered in the same pharmaceutical composition, it is understood that they can be administered in any order to a subject in need thereof. For example, a compound of the invention can be administered prior to (*e*.*g*., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e*.*g*., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of the additional anticancer therapeutic, to a subject in need thereof. In various embodiments, the anticancer therapeutics are administered 1 minute apart, 10 minutes apart, 30 minutes apart, less than 1 hour apart, 1 hour apart, 1 hour to 2 hours apart, 2 hours to 3 hours apart, 3 hours to 4 hours apart, 4 hours to 5 hours apart, 5 hours to 6 hours apart, 6 hours to 7 hours apart, 7 hours to 8 hours apart, 8 hours to 9 hours apart, 9 hours to 10 hours apart, 10 hours to 11 hours apart, 11 hours to 12 hours apart, no more than 24 hours apart or no more than 48 hours apart. In one embodiment, the anticancer therapeutics are administered within the same office visit. In another embodiment, the combination anticancer therapeutics of the invention are administered at 1 minute to 24 hours apart

### 6.3.2. OTHER THERAPIES

The present invention also provides methods for preventing, treating, and/or managing cancer, the methods comprising administering to a patient (*e*.*g*., a human patient) in need thereof, a prophylactically or a therapeutically effective regimen, the regimen comprising administering to the patient a compound of the invention and one or more additional therapies, said additional therapy not being compounds of the invention. The compound of the invention and the additional therapy can be administered separately, concurrently, or sequentially. The combination of agents can act additively or synergistically.

Any therapy (*e*.*g*., therapeutic or prophylactic agent) which is useful, has been used, or is currently being used for the prevention, treatment, and/or management of cancer can be used in compositions and method of the invention. Therapies (*e.g.,* therapeutic or prophylactic agents) include, but are not limited to, peptides, polypeptides, fusion proteins, nucleic acid molecules, small molecules, mimetic agents, synthetic drugs, inorganic molecules, vaccines, antibodies and organic molecules- Non-limiting examples of cancer therapies include chemotherapies, radiation therapies, hormonal therapies, targeted therapies, and/or biological therapies/immunotherapies and surgery. In certain embodiments, a prophylactically and/or therapeutically effective regimen of the invention comprises the administration of a combination of therapies.

Examples of cancer therapies include, but not limited to: acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dirnesylate; bisphosphonates (*e*.*g*., pamidronate (Aredria), sodium clondronate (Bonefos), zoledronic acid (Zometa), alendronate (Fosamax), etidronate, ibandomate, cimadronate, risedromate, and tiludromate); bizelesin; bleomycin sulfate; brequirzar sodium; bropirinrine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; EphA2 inhibitors; elsamitrucin; enloplatin; enpromate; epipropidine; epirubicin hydrochloride; erbulozole; esorobicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flusocitabine; fosquidone; fostriecin sodium; gemcitabine; gemcitabine hydrochloride; hydroxyurea; idarubicin hydrachloride; ifosfamide; ilmofosine; interleukin II (including recombinant interleukin II, or rIL2), interferon alpha-2a; interferon alpha-2b; interferon aipha-n1; interferon alpha-n3; interferon bet-I a; interferon gamma-I b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; anti-CD2 antibodies; megestrol acetate; melengestrol acetate; melphalan; menagaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogietimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur, teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; frimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride.

Other examples of cancer therapies include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; axninolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor, bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospennine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; HMG CoA reductase inhibitors (*e*.*g*., atorvastatin, cerivastatin, fluvastatin, lescol, lupitor, lovastatin, rosuvastatin, and simvastatin); hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4- iroplact; irsogladine; isobengazole; isohomohalicondrin B; itasetron; jasplakinolide; kabalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; LFA-3TIP (Biogen, Cambridge, MA; International Publication No. WO 93/0686 and U.S. Patent No. 6,162,432); liarozole; linear polyamine analogue; lipophilic disaccharide peptide; lipophilic platinum compounds; lissoclinamide 7; lobaplatin; lombricine; Iometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgamostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+-pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; sernustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; 5-fluorouracil; leucovorin; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor, urokinase receptor antagonists; vapreotide; variolin B; vector system, erythrocyte gene therapy; thalidomide; velaresol; veramine; verdins; verteporfin; vinorelbine; vinxaltine; vorozole; zanoterone; zeniplatin; zilascorb; and zinostatin stimalamer.

In some embodiments, the therapy(ies) used in combination with a compound of the invention is an immunomodulatory agent. Non-limiting examples of immunodulatory agents include proteinaceous agents such as cytokines, peptide mimetics, and antibodies (*e*.*g*., human, humanized, chimeric, monoclonal, polyclonal, Fvs, ScFvs, Fab or F(ab)₂ fragments or epitope binding fragments), nucleic acid molecules (*e.g.,* antisense nucleic acid molecules and triple helices), small molecules, organic compounds, and inorganic compounds. In particular, immunomodulatory agents include, but are not limited to, methotrexate, leflunomide, cyclophosphamide, cytoxan, Immuran, cyclosporine A, minocycline, azathioprine, antibiotics (*e.g.,* FK506 (tacrolimus)), methylprednisolone (MP), corticosteroids, steroids, mycophenolate mofetil, rapamycin (sirolimus), mizoribine, deoxyspergualin, brequinar, malononitriloamindes (*e.g.,* leflunamide), T cell receptor modulators, cytokine receptor modulators, and modulators mast cell modulators. Other examples of immunomodulatory agents can be found, *e.g.,* in U.S. Publication No. 2005/0002934 A1 at paragraphs 259-275 which is incorporated herein by reference in its entirety. In one embodiment, the immunomodulatory agent is a chemotherapeutic agent. In an alternative embodiment, the immunomodulatory agent is an immunomodulatory agent other than a chemotherapeutic agent. In some embodiments, the therapy(ies) used in accordance with the invention is not an immunomodulatory agent.

In some embodiments, the therapy(ies) used used in combination with a compound of the invention is an anti-angiogenic agent. Non-limiting examples of anti-angiogenic agents include proteins, polypeptides, peptides, fusion proteins, antibodies (*e*.*g*., human, humanized, chimeric, monoclonal, polyclonal, Fvs, ScFvs, Fab fragments, F(ab)₂ fragments, and antigen-binding fragments thereof) such as antibodies that immunospecifically bind to TNF-α, nucleic acid molecules (*e*.*g*., antisense molecules or triple helices), organic molecules, inorganic molecules, and small molecules that reduce or inhibit angiogenesis. Other examples of anti-angiogenic agents can be found, *e.g.,* in U.S. Publication No. 2005/0002934 A1 at paragraphs 277-282, which is incorporated by reference in its entirety. In other embodiments, the therapy(ies) used in accordance with the invention is not an anti-angiogenic agent.

In some embodiments, the therapy(ies) used used in combination with a compound of the invention is an inflammatory agent Non-limiting examples of anti-inflammatory agents include any anti-inflammatory agent, including agents useful in therapies for inflammatory disorders, well-known to one of skill in the art. Non-limiting examples of anti-inflammatory agents include non-steroidal anti-inflammatory drugs (NSAIDs), steroidal anti-inflammatory drugs, anticholinergics (*e*.*g*., atropine sulfate, atropine methylnitrate, and ipratropium bromide (ATROVENT™), beta2-agonists (*e.g.,* abuterol (VENTOLIN™ and PROVENTIL™), bitolterol (TORNALATE™), levalbuterol (XOPONEX™), metaproterenol (ALUPENT™), pirbuterol (MAXAIR™), terbutlaine (BRETHAIRE™ and BRETHINE™), albuterol (PROVENTTL™, REPETABS™, and VOLMAX™), formoterol (FORADIL AEROLIZER™), and salmeterol (SEREVENT™ and SEREVENT DTSKUS™)), and methylxanthines (*e.g.,* theophylline (UNIPHYL™, THEO-DUR™, SLO-BID™, AND TEHO-42™)). Examples ofNSAIDs include, but are not limited to, aspirin, ibuprofen, celecoxib (CELEBREX™, diclofenac (VOLTAREN™), etodolac (LODINE™), fenoprofen (NALFON™), indomethacin (INDOCIN™), ketoralac (TORADOL™), oxaprozin (DAYPRO™), nabumentone (RELAFEN™), sulindac (CLINORIL™), tolmentin (TOLECTINT™), rofecoxib (VIOXX™), naproxen (ALEVE™, NAPROSYN™), ketoprofen (ACTRON™) and nabumetone (RELAFEN™). Such NSAIDs function by inhibiting a cyclooxgenase enzyme (*e*.*g*., COX-1 and/or COX-2). Examples of steroidal anti-inflammatory drugs include, but are not limited to, glucocorticoids, dexamethasone (DECADRON™), corticosteroids (*e.g.,* methylprednisolone (MEDROL™), cortisone, hydrocortisone, prednisone (PREDMSONE™ and DELTASONE™], prednisolone (PRELONE™ and PEDLAPRED™), triamcinolone, azulfidine, and inhibitors of eicosanoids (*e.g.*, prostaglandins, thromboxanes, and leukotrienes. Other examples of anti-inflammatory agents can be found, *e.g.*, in U.S. Publication No. 005/0002934 A1 at paragraphs 290-294, which is incorporated by reference in its entirety. In other embodiments, the therapy(ies) used in accordance with the invention is not an anti-inflammatory agent.

In certain embodiments, the therapy(ies) used is an alkylating agent, a nitrosourea, an antimetabolite, and anthracyclin, a topoisomerase **II** inhibitor, or a mitotic inhibitor. Alkylating agents include, but are not limited to, busulfan, cisplatin, carboplatin, cholormbucil, cyclophosphamide, ifosfarnide, decarbazine, mechlorethamine, mephalen, and themozolomide. Nitrosoureas include, but are not limited to carmustine (BCNU) and lomustine (CCNU). Antimetabolites includes but are not limited to 5-fluorouracil, capecitabine, methotrexate, gemcitabine, cytarabine, and fludarabine. Anthracyclins include but are not limited to daunorubicin, doxorubicin, epirubicin, idarubicin, and mitoxantrone. Topoisomerase II inhibitors include, but are not limited to, topotecan, irinotecan, etopiside (VP-16), and teniposide. Mitotic inhibitors include, but are not limited to taxanes (paclitaxel, docetaxel), and the vinca alkaloids (vinblastine, vincristine, and vinorelbine).

In some embodiments, the therapy(ies) used in combination with a compound of the invention is an agent that targets cancer stem cells. In certain embodiments, the agent is attached directly or indirectly to a therapeutic moiety other than a compound of the invention. In some embodiments, the agent used is an agent that binds to a marker, e.g., antigen on cancer stem cells. In a specific embodiment, the agent binds to an antigen that is expressed at a greater level on cancer stern cells than on normal stem cells. In another specific embodiment, the agent binds to an antigen that is expressed at the same level on cancer stem cells as on normal stem cells.

In a specific embodiment, the agent binds specifically to a cancer stem cell antigen. In other embodiments, the therapy(ies) used in accordance with the invention is an agent that binds to a marker on cancer stem cells. Non-limiting examples of antigens on cancer stem cells that can be used to target cancer stem cells include CD123, CD44, CLL1, CD133, CD34, CD19, CD20, RC2, and α₂β₁. In one embodiment, the agent that binds to a marker on cancer stem cells is an antibody. In another embodiment, the agent that binds to a marker on cancer stem cells is a ligand (e.g., interleukin 3). In certain embodiments, the antibody or antibody fragment or ligand is attached directly or indirectly to a therapeutic moiety. Non-limiting examples of therapeutic moieties include, but are not limited to, therapeutic enzymes, chemotherapeutic agents, cytokines, toxins, radionuclides, and antimetabolites.

In certain embodiments, antibodies that bind to a marker on cancer stem cells are substantially non-immunogenic in the treated subject. Strategies to prepare non-immunogenic antibodies include, but are not limited to, chimerizing the antibody, humanizing the antibody, and isolating antibodies from the same species as the subject receiving the therapy. See, for example, paragraphs 539-573 of U.S. Publication No. 2005/0002934 A1, which is incorporated by reference in its entirety. Antibodies that bind to markers in cancer stem cells can be produced using techniques known in the art.

In some embodiments, a compound of the invention is used in combination with radiation therapy comprising the use of x-rays, gamma rays and other sources of radiation to destroy cancer stem cells and/or cancer cells. In specific embodiments, the radiation therapy is administered as external beam radiation or teletherapy, wherein the radiation is directed from a remote source. In other embodiments, the radiation therapy is administered as internal therapy or brachytherapy wherein a radioactive source is placed inside the body close to cancer stem cells, cancer cells and/or a tumor mass.

Currently available cancer therapies and their dosages, routes of administration and recommended usage are known in the art and have been described in such literature as the Physician's Desk Reference (60th ed., 2006). In accordance with the present invention, the dosages and frequency of administration of chemotherapeutic agents are described in the Section 6.3.1.

### 6.3.3. TARGET CANCERS

Any type of cancer can be prevented, treated and/or managed in accordance with the invention- Non-limiting examples of cancers that can be prevented, treated and/or managed in accordance with the invention cancers include: leukemias, such as but not limited to, acute leukemia, acute lymphocytic leukemia, acute myelocytic leukemias, such as, myeloblastic, promyelocytic, myelomonocytic, monocytic, and erythroleukemia leukemias and myelodysplastic syndrome; chronic leukemias, such as but not limited to, chronic myelocytic (granulocytic) leukemia, chronic lymphocytic leukemia, hairy cell leukemia; polycythemia vera; lymphomas such as but not limited to Hodgkin's disease, non-Hodgkin's disease; multiple myeloma such as but not limited to smoldering multiple myeloma, nonsecretory myeloma, osteosclerotic myeloma, plasma cell leukemia, solitary plasmacytoma and extramedullary plasmacytoma; Waldenström's macroglobulinemia; monoclonal gammopathy of undetermined significance; benign monoclonal gammopathy; heavy chain disease; bone and connective tissue sarcomas such as but not limited to bone sarcoma, osteosarcoma, chondrosarcoma, Ewing's sarcoma, malignant giant cell tumor, fibrosarcoma of bone, chordoma, periosteal sarcoma, soft-tissue sarcomas, angiosarcoma (hemangiosarcoma), fibrosarcoma, Kaposi's sarcoma, leiomyosarcoma, liposarcoma, lynnphangiosarcoma, neurilemmoma, rhabdomyosarcoma, synovial sarcoma; brain tumors such as but not limited to, glioma, astrocytoma, brain stem glioma, ependymoma, oligodendroglioma, nonglial tumor, acoustic neurinoma, craniopharyngioma, medulloblastoma, meningioma, pineocytoma, pineoblastoma, primary brain lymphoma; breast cancer including but not limited to ductal carcinoma, adenocarcinoma, lobular (small cell) carcinoma, intraductal carcinoma, medullary breast cancer, mucinous breast cancer, tubular breast cancer, papillary breast cancer, Paget's disease, and inflammatory breast cancer, adrenal cancer such as but not limited to pheochromocytom and adrenocortical carcinoma; thyroid cancer such as but not limited to papillary or follicular thyroid cancer, medullary thyroid cancer and anaplastic thyroid cancer; pancreatic cancer such as but not limited to, insulinoma, gastrinoma, glucagonoma, vipoma, somatostatin-secreting tumor, and carcinoid or islet cell tumor; pituitary cancers such as but limited to Cushing's disease, prolactin-secreting tumor, acromegaly, and diabetes insipius; eye cancers such as but not limited to ocular melanoma such as iris melanoma, choroidal melanoma, and cilliary body melanoma, and retinoblastoma; vaginal cancers such as squamous cell carcinoma, adenocarcinoma, and melanoma; vulvar cancer such as squamous cell carcinoma, melanoma, adenocarcinoma, basal cell carcinoma, sarcoma, and Paget's disease; cervical cancers such as but not limited to, squamous cell carcinoma, and adenocarcinoma; uterine cancers such as but not limited to endometrial carcinoma and uterine sarcoma; ovarian cancers such as but not limited to, ovarian epithelial carcinoma, borderline tumor, germ cell tumor, and stromal tumor; esophageal cancers such as but not limited to, squamous cancer, adenocarcinoma, adenoid cystic carcinoma, mucoepidermoid carcinoma, adenosquamous carcinoma, sarcoma, melanoma, plasmacytoma, venmeous carcinoma, and oat cell (small cell) carcinoma; stomach cancers such as but not limited to, adenocarcinoma, fungating (polypoid), ulcerating, superficial spreading, diffusely spreading, malignant lymphoma, liposarcoma, fibrosarcoma, and carcinosarcoma; colon cancers; rectal cancers; liver cancers such as but not limited to hepatocellular carcinoma and bepatoblastoma; gallbladder cancers such as adenocarcinoma; cholangiocarcinomas such as but not limited to papillary, nodular, and diffuse; lung cancers such as non-small cell lung cancer, squamous cell carcinoma (epidermoid carcinoma), adenocarcinoma, large-cell carcinoma and small-cell lung cancer, testicular cancers such as but not limited to germinal tumor, seminoma, anaplastic, classic (typical), spermatocytic, nonseminoma, embryonal carcinoma, teratoma carcinoma, choriocarcinoma (yolk-sac tumor), prostate cancers such as but not limited to, prostatic intraepithelial neoplasia, adenocarcinoma, leiomyosarcoma, and rhabdomyosarcoma; penal cancers; oral cancers such as but not limited to squamous cell carcinoma; basal cancers; salivary gland cancers such as but not limited to adenocarcinoma, mucoepidermoid carcinoma, and adenoidcystic carcinoma; pharynx cancers such as but not limited to squamous cell cancer, and verrucous; skin cancers such as but not limited to, basal cell carcinoma, squamous cell carcinoma and melanoma, superficial spreading melanoma, nodular melanoma, lentigo malignant melanoma, acral lentiginous melanoma; kidney cancers such as but not limited to renal cell carcinoma, adenocarcinoma, hypemephroma, fibrosarcoma, transitional cell cancer (renal pelvis and/ or uterer); Wilms' tumor; bladder cancers such as but not limited to transitional cell carcinoma, squamous cell cancer, adenocarcinoma, carcinosarcoma. In addition, cancers include myxosarcorna, osteogenic sarcoma, endotheliosarcoma, lymphangioendotheliosarcoma, mesothelioma, synovioma, hemangioblastoma, epithelial carcinoma, cystadenocarcinoma, bronchogenic carcinoma, sweat gland carcinoma, sebaceous gland carcinoma, papillary carcinoma and papillary adenocarcinomas (for a review of such disorders, see Fishman et al., 1985, Medicine, 2d Ed., J.B. Lippincott Co., Philadelphia and Murphy et al., 1997, Informed Decisions: The Complete Book of Cancer Diagnosis, Treatment, and Recovery, Viking Penguin, Penguin Books U.S.A., Inc., United States of America).

The prophylactically and/or therapeutically effective regimens are also useful in the treatment, prevention and/or management of a variety of cancers or other abnormal proliferative diseases, including (but not limited to) the following: carcinoma, including that of the bladder, breast, colon, kidney, liver, lung, ovary, pancreas, stomach, cervix, thyroid and skin; including squamous cell carcinoma; hematopoietic tumors of lymphoid lineage, including leukemia, acute lymphocytic leukemia, acute lymphoblastic leukemia, B-cell lymphoma, T cell lymphoma, Burkitt's lymphoma; hematopoietic tumors of myeloid lineage, including acute and chronic myelogenous leukemias and promyelocytic leukemia; tumors of mesenchymal origin, including fibrosarcoma and rhabdomyoscarcoma; other tumors, including inelanoma, sezninoma, tetratocarcinoma, neuroblastoma and glioma; tumors of the central and peripheral nervous system, including astrocytoma, neuroblastoma, glioma, and schwannomas; tumors of mesenchymal origin, including fibrosarcoma, rhabdomyoscarama, and osteosarcoma; and other tumors, including melanoma, xeroderma pigmentosum, keratoactanthoma, seminoma, thyroid follicular cancer and teratocarcinoma. In some embodiments, cancers associated with aberrations in apoptosis are prevented, treated and/or managed in accordance with the methods of the invention. Such cancers may include, but are not limited to, follicular lymphomas, carcinomas with p53 mutations, hormone dependent tumors of the breast, prostate and ovary, and precancerous lesions such as familial adenomatous polyposis, and myelodysplastic syndromes. In specific embodiments, malignancy or dysproliferative changes (such as metaplasias and dysplasias), or hyperproliferative disorders of the skin, lung, liver, bone, brain, stomach, colon, breast, prostate, bladder, kidney, pancreas, ovary, and/or uterus are prevented, treated and/or managed in accordance with the methods of the invention. In other specific embodiments, a sarcoma, or melanoma is prevented, treated and/or managed in accordance with the methods of the invention.

In a specific embodiment, the cancer being prevented, treated, and/or managed in accordance with the invention is leukemia, lymphoma or myeloma (e.g., multiple myeloma).

Non-limiting examples of leukemias and other blood-borne cancers that can be prevented, treated, and/or managed with the methods of the invention include acute lymphoblastic leukemia "ALL", acute lymphoblastic B-cell leukemia, acute lymphoblastic T-cell leukemia, acute myeloblastic leukemia "AML", acute promyelocytic leukemia "APL", acute manoblastic leukemia, acute erythroleukemic leukemia, acute megakaryoblastic leukemia, acute myelomonocytic leukemia, acute nonlymphocyctic leukemia, acute undifferentiated leukemia, chronic myelocytic leukemia "CML", chronic lymphocytic leukemia "CLL", and hairy cell leukemia. In a specific embodiment, the cancer being prevented, treated, and/or managed in accordance with the invention is acute lymphoblastic leukemia "ALL".

Non-limiting examples of lymphomas that can be prevented, treated, and/or managed in accordance with the methods of the invention include Hodgkin's disease, non-Hodgkin's Lymphoma, Multiple myeloma, Waldenström's macroglobulinemia, Heavy chain disease, and Polycythemia vera.

In another embodiment, the cancer being prevented, treated, and/or managed in accordance with the invention is a solid tumor. Examples of solid tumors that can be prevented, treated, and/or managed in accordance with the methods of the invention include, but are not limited to fibrosarcoma, myxosarcoma, liposarcoma, chondrosarcoma, osteogenic sarcoma, chordoma, angiosarcoma, endotheliosarcoma, lymphangiosarcoma, lymphangioendothebosarcoma, synovioma, mesothelioma, Ewing's tumor, leiomyosarcoma, rhabdomyosarcoma, colon cancer, colorectal cancer, kidney cancer, pancreatic cancer, bone cancer, breast cancer, ovarian cancer, prostate cancer, esophageal cancer, stomach cancer, oral cancer, nasal cancer, throat cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, uterine cancer, testicular cancer, small cell lung carcinoma, bladder carcinoma, lung cancer, epithelial carcinoma, glioma, glioblastoma multiforme, astrocytoma, medulloblastoma, craniopharyngioma, ependymoma, pinealoma, hemangioblastoma, acoustic neuroma, oligodendroglioma, meningioma, skin cancer, melanoma, neuroblastoma, and retinoblastoma. In a specific embodiment, the cancer being prevented, treated, and/or managed in accordance with the invention is medulloblastoma. In another specific embodiment, the cancer being prevented, treated, and/or managed in accordance with the invention is pancreatic cancer.

### 6.3.4. PATIENT POPULATION

In accordance with the invention, a prophylactically and/or therapeutically effective regimen of the invention is administered to subjects with or expected to develop cancer (e.g., subjects with a genetic predisposition for a particular type of cancer, subject that have been exposed to a carcinogen, or subjects that are in remission from a particular cancer). In a specific embodiment, the subject has been diagnosed with cancer using techniques known to one of skill in the art including, but not limited to, physical examination (e.g., prostate examination, breast examination, lymph nodes examination, abdominal examination, skin surveillance), visual methods (e.g., colonoscopy, bronchoscopy, endoscopy), PAP smear analyses (cervical cancer), stool guaiac analyses, blood tests (*e.g.,* complete blood count (CBC) test), blood chemistries including liver function tests, prostate specific antigen (PSA) test, carcinoembryonic antigen (CEA) test, cancer antigen (CA)-125 test, alpha-fetoprotein (AFP)), karyotyping analyses, bone marrow analyses (*e.g.,* in cases of hematological malignancies), histology, cytology, a sputum analysis and imaging methods (e.g., computed tomography (CT), magnetic resonance imaging (MRI), ultrasound, X-ray imaging, mammograph imaging, bone scans). Subjects may or may not have been previously treated for cancer.

The prophylactically and/or therapeutically regimens may be used as any line of cancer therapy, e.g., a first line, second line or third line of cancer therapy. In a specific embodiment, the subject to receive or receiving a compound of the invention is receiving or has received other cancer therapies. In another embodiment the subject to receive a compound of the invention is receiving other cancer therapies before any adverse effects or intolerance of these other cancer therapies occurs. In an alternative embodiment, the subject to receive or receiving a compound of the invention has not received or is not receiving other cancer therapies.

In one embodiment, a compound of the invention is administered to a subject that is undergoing or has undergone surgery to remove a tumor or neoplasm. In a specific embodiment, a compound of the invention is administered to a subject concurrently or following surgery to remove a tumor or neoplasm. In another embodiment, a compound of the invention is administered to a subject before surgery to remove a tumor or neoplasm and, in some embodiments, during and/or after surgery.

In one embodiment, a compound of the invention is administered to a subject after a course of therapy with the goal of killing cancer cells. In some embodiments, the course of therapy involves the administration of bolus doses of chemotherapeutic agents and/or bolus doses of radiation therapy. In a specific embodiment, a compound of the invention is administered to a subject after the subject has received a course of therapy involving maximum tolerated doses or no observed adverse effect level doses of one or more chemotherapeutic agents and/or radiation therapy.

In certain embodiments, a compound of the invention is administered to a subject as an alternative to chemotherapy, radiation therapy, hormonal therapy, targeted therapy, and/or biological therapy/immunotherapy where the therapy has proven or may prove too toxic, i.e., results in unacceptable or unbearable side effects, for the subject. In some embodiments, a compound of the invention is administered to a subject that is susceptible to adverse reactions from other cancer therapies. The subject may *e.g.,* have a suppressed immune system (e.g., post-operative patients, chemotherapy patients, and patients with immunodeficiency disease), have an impaired renal or liver function, be elderly, be a child, be an infant, have a neuropsychiatric disorder, take a psychotropic drug, have a history of seizures, or be on medication that would negatively interact with the cancer therapies.

In a specific embodiment, a compound of the invention is administered to subjects that will, are or have undergone radiation therapy. Among these subjects are those that have received chemotherapy, hormonal therapy and/or biological therapy/immunotherapy as well as those who have undergone surgery.

In another embodiment, a compound of the invention is administered to subjects that will, are or have received hormonal therapy, targeted therapy, and/or biological therapy/immunotherapy. Among these subjects are those that have received chemotherapy and/or radiation therapy as well as those who have undergone surgery.

In certain embodiments, a compound of the invention is administered to a subject refractory to one or, more therapies. In one embodiment, that a cancer is refractory to a therapy means that at least some significant portion of the cancer cells are not killed or their cell division arrested. The determination of whether the cancer cells are refractory can be made either in *vivo or in vitro* by any method known in the art for assaying the effect of a therapy on cancer cells, using the art-accepted meanings of "refractory" in such a context. *See, e.g.,* Section 6.5 for non-limiting examples of methods for determining the effect of a therapy on cancer cells. In various embodiments, a cancer is refractory where the number or amount of cancer cells has not been significantly reduced, or has increased. In other embodiments, that a cancer is refractory means that at least some significant portion of cancer stem cells are not killed or their cell division arrested. The determination of whether the cancer stem cells are refractory can be made either in *vivo or in vitro* by any methods known in the art or described herein, including determination of the number, amount, portion, percentage or quantity of cancer stem cells (e.g. as measured by % of tumor) relative to a reference (e.g. pre-treatment value) over time (i.e. versus post-treatment values). See, e.g., Section 6.4 for non-limiting examples methods for determining the effectiveness of a therapy on cancer stem cells.

In some embodiments, a compound of the invention is administered to reverse resistance or reduced sensitivity of cancer cells to certain hormonal, radiation and chemotherapeutic agents thereby resensitizing the cancer cells to one or more of these agents, which can then be administered (or continue to be administered) to treat or manage cancer, including to prevent metastasis. In a specific embodiment, the compound of the invention is administered to patients with increased levels of the cytokine IL-6, which has been associated with the development of cancer cell resistance to different treatment regimens, such as chemotherapy and hormonal therapy.

In some embodiments, a compound of the invention is administered to a subject with a mean absolute lymphocyte count of at least approximately 400 cells/mm³, at 500 cells/mm³, at least approximately 600 cells/mm³, at least approximately 700 cells/mm³, at least approximately 800 cells/mm³, at least approximately 900 cells/mm³, at least approximately 1000 cells/mm³, at least approximately 1100 cells/mm3, at least approximately 1200 cells/mm³. In other embodiments, a prophylactically and/or therapeutically effective regimen of the invention is administered to a subject with a mean absolute lymphocyte count of approximately 400 cells/mm³ to approximately 1200 cells/mm³, approximately 500 cells/mm³ to approximately 1200 cells/mm³, approximately 600 cells/mm³ to approximately 1200 cells/mm³, approximately 700 cells/mm³ to approximately 1200 cells/mm³, approximately 800 cells/mm³ to approximately 1200 cells/mm³, approximately 900 cells/mm³ to approximately 1200 cells/mm³, approximately 1000 cells/mm³ to approximately 12000 cells/mm³ In a more specific embodiment, the regimen results in a mean absolute lymphocyte count of at least approximately 400 cells/mm³. The mean absolute lymphocyte count can be determined by methods set forth in Section 6.6, *infra.* In some embodiments, the regimen comprises monitoring the mean absolute lymphocyte count in the human subject.

In some embodiments, a regimen of the invention is administered to a subject with a mean absolute neutrophil count of at least approximately 1000 cells/mm³, at least approximately 1200 cells/mm³, at least approximately 1500 cells/mm³, or at least approximately 2000 cells/mm³. In another embodiments, a regimen of the invention is administered to a subject with a mean absolute neutrophil count of approximately 1000 ceils/mm³ to approximately 2500 cells/mm³. In a specific embodiment, the mean absolute neutrophil count is determined by the methods described in Section 6.6, *infra.* In some embodiments, the regimen comprises monitoring the absolute neutrophil count.

In some embodiments, a compound of the invention is administered to a subject that is in remission. In a specific embodiment, the subject is cancer-free, *i.e.,* no cancer is detectable using a method described herein or known to one of skill in the art.

### 6.4. METHODS OF MONITORING CANCER STEM CELLS

As part of the prophylactically effective regimens and/or therapeutically effective regimens of the invention, the cancer stem cell population can be monitored to assess the efficacy of a therapy as well as to determine prognosis of a subject with cancer. In certain embodiments of the prophylactically effective regimens and/or therapeutically effective regimens of the invention, the regimens result in a stabilization or reduction in the cancer stem cell population in the patient. In one embodiment, the subject undergoing the regimen is monitored to assess whether the regimen has resulted in a stabilization or reduction in the cancer stem cell population in the subject.

In some embodiments, the number or amount of cancer stem cells in a subject is determined using a technique well-known to one of skill in the art or described below in § 6.5.

In accordance with the invention, cancer stem cells comprise a unique subpopulation (often 0.1-10% or so) of a tumor that, in contrast to the remaining 90% or so of the tumor (*i.e.,* the tumor bulk), are relatively more tumorigenic resistant to traditional therapy (due, in part, to their and relatively more slow-growing or quiescent nature). Given that conventional therapies and regimens have, in large part, been designed to attack rapidly proliferating cells (i.e., those cancer cells that comprise the tumor bulk), slower growing cancer stem cells may be relatively more resistant than faster growing tumor bulk to conventional therapies and regimens. This would explain another reason for the failure of standard oncology treatment regimens to ensure long-term benefit in most patients with advanced stage cancers. In a specific embodiment, a cancer stern cell(s) is the founder cell of a tumor (*i.e.,* it is the progenitor of cancer cells). In some embodiments, a cancer stem cell(s) has one or more or all of the following characteristics or properties: (i) the ability to initiate a tumor and to perpetuate tumor growth, (ii) is generally less mutated than the bulk of a tumor, (iii) has many features of a normal stem cell(s) (*e.g.,* similar cell surface antigen expression profile, self-renewal programs, multi-drug resistance, effective methods to prevent and/or manage DNA damage (e.g. via effective repair mechanisms), an immature phenotype, etc. characteristic of normal stem cells) and may be derived from a normal stem cell(s), (iv) is potentially responsive to its microenvironment (*e.g.,* the cancer stem cells may be capable of being induced to differentiate and/or divide asymmetrically), and (v) is the source of metastases.

In other embodiments, the number or amount of cancer stem cells in a sample from a subject is determined/assessed using a technique described herein or well-known to one of skill in the art. Such samples include, but are not limited to, biological samples and samples derived from a biological sample. In certain embodiments, in addition to the biological sample itself or in addition to material derived from the biological sample such as cells, the sample used in the methods of this invention comprises added water, salts, glycerin, glucose, an antimicrobial agent, paraffin, a chemical stabilizing agent, heparin, an anticoagulant, or a buffering agent. In certain embodiments, the biological sample is blood, serum, urine, bone marrow or interstitial fluid. In another embodiment, the sample is a tissue sample. In a particular embodiment, the tissue sample is breast, brain, skin, colon, lung, liver, ovarian, pancreatic, prostate, renal, bone or skin tissue. In a specific embodiment, the tissue sample is a biopsy. The amount of biological sample taken from the subject will vary according to the type of biological sample and the method of detection to be employed. In a particular embodiment, the biological sample is blood, serum, or urine and the amount of blood, serum, or urine taken from the subject is 0.1 ml, 0.5 ml, 1 ml, 5 ml, 8 ml, 10 ml or more. In another embodiment, the biological sample is a tissue and the amount of tissue taken from the subject is less than 10 milligrams, less than 25 milligrams, less than 50 milligrams, less than 1 gram, less than 5 grams, less than 10 grams, less than 50 grams, or less than 100 grams.

In accordance with the methods of the invention, a sample derived from a biological sample is one in which the biological sample has been subjected to one or more pretreatment steps prior to the detection and/or measurement of the cancer stem cell population in the sample. In certain embodiments, a biological fluid is pretreated by centrifugation, filtration, precipitation, dialysis, or chromatography, or by a combination of such pretreatment steps. In other embodiments, a tissue sample is pretreated by freezing, chemical fixation, paraffin embedding, dehydration, permeablization, or homogenization followed by centrifugation, filtration, precipitation, dialysis, or chromatography, or by a combination of such pretreatment steps. In certain embodiments, the sample is pretreated by removing cells other than stem cells or cancer stem cells from the sample, or removing debris from the sample prior to the determination of the number or amount of cancer stem cells in the sample according to the methods of the invention. The samples for use in the methods of this invention may be taken from any animal subject, preferably mammal, most preferably a human. The subject from which a sample is obtained and utilized in accordance with the methods of this invention includes, without limitation, an asymptomatic subject, a subject manifesting or exhibiting 1, 2, 3, 4 or more symptoms of cancer, a subject clinically diagnosed as having cancer, a subject predisposed to cancer, a subject suspected of having cancer, a subject undergoing therapy for cancer, a subject that has been medically determined to be free of cancer (*e.g.,* following therapy for the cancer), a subject that is managing cancer, or a subject that has not been diagnosed with cancer. In certain embodiments, the term "cancer-free," as used herein, refers to a subject or subjects that are free from cancer. In other embodiments, the term refers to a subject or subjects free from any disorder.

In certain embodiments, the number or amount of cancer stem cells in a subject or a sample from a subject assessed at least 1, 2, 4, 6, 8, 10, 12, 14, 15, 16, 18, 20, or 30, 60, 90 days 6 months, 9 months, 12 months, > 12 months after the subject begins receiving the regimen. In certain embodiments, the number or amount of cancer stem cell is assessed after a number of doses (*e.g.,* after 1, 2, 5, 10, 20, 30 or more doses of a therapy). In other embodiments, the number or amount of cancer stem cells is assessed after 2 weeks, 1 1 month, 2 months, 1 year, 2 years, 3 years, 4 years or more after receiving one or more therapies.

The number, quantity, amount or relative amount of cancer stem cells in a sample can be expressed as the percentage of, *e.g.,* overall cells, overall cancerous cells or overall stem cells in the sample.

In some embodiments, the sample may be a blood sample or bone marrow sample, wherein the number, amount or relative amount of cancer stern cells per unit of volume (*e.g.,* 1 mL) or other measured unit (*e.g.,* per unit field in the case of a histological analysis) is quantitated. In certain embodiments, the cancer stem cell population is determined as a portion (e.g., a percentage) of the cancerous cells present in the blood sample or as a subset of the cancerous cells present in the blood sample. The cancer stem cell population, in other embodiments, can be determined as a portion (e.g., percentage) of the total blood cells. In yet other embodiments, the cancer stem cell population is determined as a portion (e.g., a percentage) of the total stem cells present in the blood sample.

In other embodiments, the sample from the patient is a tissue sample (e.g., a biopsy from a subject with or suspected of having cancerous tissue), where the number, amount, portion, percentage or relative amount of cancer stem cells can be measured, for example, by immunohistochemistry or flow cytometry, or on the basis of the number, amount, portion, percentage or relative amount of cancer stem cells per unit weight of the tissue. In certain embodiments, the cancer stem cell population is determined as a portion (*e.g.,* a percentage) of the cancerous cells present in the tissue sample or as a subset of the cancerous cells present in the tissue sample. In yet other embodiments, the cancerous stem cell population is determined as a portion (e.g., a percentage) of the overall cells or stem cell cells in the tissue sample.

The number or amount of cancer stem cells in a test sample can be compared with the number or amount of cancer stem cells in reference sample(s) to assess the efficacy of the regimen. In one embodiment, the reference sample is a sample obtained from the subject undergoing therapy at an earlier time point (*e.g.,* prior to receiving the regimen as a baseline reference sample, or at an earlier time point while receiving the therapy). In this embodiment, the therapy desirably results in a decrease in the number or amount of cancer stem cells in the test sample as compared with the reference sample. In another embodiment, the reference sample is obtained from a healthy, cancer-free subject, or from a patient that is in remission for the same type of cancer. In this embodiment, the therapy desirably results in the test sample having an equal number or amount of cancer stem cells, or less than the number or amount of cancer stem cells than are detected in the reference sample.

In other embodiments, the cancer stem cell population in a test sample can be compared with a predetermined reference range and/or an earlier number or amount of cancer stem cells determined for the subject to gauge the subject's response to the regimens described herein. In a specific embodiment, a reduction in the number or amount of cancer stern cells relative to a predetermined reference range and/or earlier cancer stem cell number or amount determined for the subject indicates an improvement in the subject's prognosis or a positive response to the regimen, whereas an increase relative to the predetermined reference range and/or earlier cancer stem cell number or amount indicates the same or worse prognosis, or a failure to respond to the regimen. The cancer stem cell amount can be used in conjunction with other measures to assess the prognosis of the subject and/or the efficacy of the regimen. In a specific embodiment, the predetermined reference range is based on the number or amount of cancer stem cells obtained from a population(s) of patients suffering from the same type of cancer as the patient undergoing the therapy.

Generally, since stem cell antigens are often present on both cancer stem cells and normal stem cells, a sample from the cancer-afflicted patient will have a higher stem cell count than a sample from a healthy, cancer-free subject due to the presence of the cancer stem cells. The therapy will desirably result in a cancer stem cell count for the test sample (e.g., the sample from the patient undergoing therapy) that is less than or comparable to the stem cell count in a reference sample that is sample from a healthy, cancer-free subject.

If the reduction in the number or percentage of cancer stem cells is determined to be too small upon comparing the number, amount or relative amount of cancer stem cells in the sample from the subj ect undergoing the regimen with the reference sample, then the medical practitioner has a number of options to adjust the regimen. For instance, the medical practitioner can then increase either the dosage (*e.g.,* for chemotherapeutic therapies) or intensity (*e.g.,* for radiation therapies) of the therapy administered, the frequency of the administration, the duration of administration, combine the therapy with another therapy(ies), change the management altogether including halting therapy, or any combination thereof.

In certain embodiments, the dosage, frequency and/or duration of administration of a therapy is modified as a result of the change in the number, amount or relative amount of cancer stem cells detected in a test sample. For example, if a subject receiving therapy for leukemia has a cancer stem cell measurement of 2.5% of his tumor prior to therapy and 5% after 6 weeks of therapy, then the therapy may be altered or stopped because the increase in the percentage of cancer stem cells indicates that the therapy is not optimal. Alternatively, if another subject with leukemia has a cancer stem cell measurement of 2.5% of his tumor prior to therapy and 1% after 6 weeks of therapy, then the therapy may be continued because the decrease in the percentage of cancer stem cells indicates that the therapy is effective.

The number, amount or relative amount of cancer stem cells can be monitored/assessed using standard techniques known to one of skill in the art. Cancer stem cells can be monitored by, *e.g.,* obtaining a sample, such as a tumor sample, blood sample or a bone marrow sample, from a subject and detecting cancer stern cells in the sample. The number, amount or relative amount of cancer stem cells in a sample (which may be expressed as percentages of, e.g., overall cells or overall cancer cells) can be assessed by detecting the expression of antigens on cancer stem cells. Techniques known to those of skilled in the art can be used for measuring these activities. Antigen expression can be assayed, for example, by immunoassays including, but are not limited to, western blots, immunohistochemistry, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, immunofluorescence, protein A immunoassays, flow cytometry and FACS analysis. In such circumstances, the number or amount of cancer stem cells in a test sample from a subject may be determined by comparing the results to number or amount of stem cells in a reference sample (*e.g.,* a cancer-free subject sample) or to a predetermined reference range.

In a specific embodiment, the cancer stem cell population in a sample from a patient is determined by flow cytometry. This method exploits the differential expression of surface markers on cancer stem cells relative to the bulk of the tumor. Labelled antibodies (e.g., fluorescent antibodies) can be used to react with the cells in the sample, and the cells are subsequently sorted by FACS methods. In some embodiments, a combination of cell surface markers are utilized in order to determine the number, amount or relative amount of cancer stem cells in the sample. For example, both positive and negative cell sorting may be used to assess the number, amount or relative amount of cancer stem cells in the sample. Cancer stem cells for specific tumor types can be determined by assessing the expression of markers on cancer stem cells. In certain embodiments, the tumors harbor cancer stem cells and their associated markers as set forth in Table 1 below.

**Table 1**

| | Cancer Stem Cell Phenotype |
|---|---|
| Tumor | |
| Leukemia (AML) | CD34+/CD38- |
| Breast | CD44+/CD24- |
| Brain | CD133+ |
| Leukemia (ALL) | CD34+/CD 10-/CD19- |
| Ovarian | CD44+/CD24- |
| Multiple Myeloma | CD138-/CD34-/CD19+ |
| Chronic myelogenous leukemia | CD34+/CD38- |
| Melanoma | CD20+ |
| Ependymoma | CD133+/RC2+ |
| Prostate | CD44+/α₂β₁^{hi}/CD133+ |

Additional cancer stem cell markers include CD123, CLL-1, and combinations of SLAMs, *et al.,* and those markers disclosed in U.S. Patent No. 6,004,528 to Bergstein and in U.S. Patent Application Publication No. 2006/0083682, each of which are incorporated herein by reference in its entirety.

In a specific embodiment the cancer stem population in a sample, *e.g.,* a tissue sample, such as a solid tumor biopsy, is determined using immunohistochemistry techniques. This method exploits the differential expression of surface markers on cancer stem cells relative to the bulk of the tumor. Labelled antibodies (*e.g.,* fluorescent antibodies) can be used to react with the cells in the sample, and the tissue is subsequently stained. In some embodiments, a combination of cell surface markers are utilized in order to determine the number, amount or relative amount of cancer stem cells in the sample. Cancer stem cells for specific tumor types can be determined by assessing the expression of markers that are specific to cancer stem cells. In certain embodiments, the tumors harbor cancer stem cells and their associated markers as set forth in Table 1 above.

Suitable cancer stern cell antigens may be identified: (i) through publicly available information, such as published and unpublished expression profiles including cell surface antigens of cancer stem cells of a particular tumor type or adult stem cells for a particular tissue type (e.g. Table 1), and/or (ii) by cloning cancer stem cells or adult stem cells of a particular tumor or tissue type respectively, in order to determine their expression profiles and complement of cell surface antigens. Cloning of normal stem cells is a technique routinely employed in the art (Uchida et al., "Heterogeneity of hematopoeitic stem cells", Curr. Opin. Immunol, 5:177-184 (1993)). In fact, this same technique has been successfully used to identify cancer stem cells as well. Moreover, assumption that a proportion of normal stem cell gene products, *e.g.* cell surface antigens, will also be present on cancer stem cells derived from the same tissue type has proven an effective way to identify cancer stem cell gene products and cancer stem cells. For example, knowledge that the normal hematopoietic stem cell was CD34+/CD38- resulted in the determination that acute myeloid leukemia (AML) stem cells would similarly be CD34+/CD38-. This indeed was confirmed by the aforementioned standard stem cell cloning techniques (Bonnet et al, "Human acute myeloid leukemia is organized as a hierarchy that originates from a primitive hematopoietic cell," Nat Med 3:730-737 (1997)). Brain cancer stem cells were similarly isolated used CD133, a marker of normal brain stem cells, in this case CD133 (Singh SK et al. Identification of human brain tumor initiating cells. Nature. 2004 Nov 18;432(7015):396-401).

In certain embodiments using flow cytometry of a sample, the Hoechst dye protocol can be used to identify cancer stem cells in tumors. Briefly, two Hoechst dyes of different colors (typically red and blue) are incubated with the tumor cells. The cancer stem cells, in comparison with bulk cancer cells, over-express dye efflux pumps on their surface that allow these cells to pump the dye back out of the cell. Bulk tumor cells largely have fewer of these pumps, and are therefore relatively positive for the dye, which can be detected by flow cytometry. Typically a gradient of dye positive ("dye⁺") vs. dye negative ("dye⁻") cells emerges when the entire population of cells is observed. Cancer stem cells are contained in the dye or dye low (dye^{low}) population, which are also called the "side popualtion" cells or SP cells for the characteristic appearance of these cells in a standard dot plot of this type of data. For an example of the use of the Hoechst dye protocol to characterize a stem cell population see Goodell et al., "Isolation and functional properties of murine hematopoietic stem cells that are replicating in vivo," J Exp Med 183:1797-1806 (1996).

In other embodiments using flow cytometry of a sample, the cells in the sample may be treated with a substrate for aldehyde dehydogenase that becomes fluorescent when catalyzed by the enzyme. For instance, the sample can be treated with BODIPY® - aminoacetaldehyde which is commercially available from StemCell Technologies Inc. as Aldefluor®. Cancer stem cells express high levels of aldehyde dehydrogenase relative to bulk cancer cells and therefore become brightly fluorescent upon reaction with the substrate. The cancer stem cells, which are fluorescent, can be detected and counted using a standard flow cytometer.

In other embodiments, a sample (*e.g.,* a tumor sample, blood sample or bone marrow sample) obtained from the patient is cultured in *in vitro* systems to assess the cancer stem cell population. For example, tumor samples can be cultured on soft agar, and the number of cancer stem cells can be correlated to the ability of the sample to generate colonies of cells that can be visually counted. Colony formation is considered a surrogate measure of stem cell content, and thus, can be used to quantitate the number of cancer stem cells. For instance, with hematological cancers, colony-forming assays include colony forming cell (CFC) assays, long-term culture initiating cell (LTC-IC) assays, and suspension culture initiating cell (SC-IC) assays.

Alternatively, the proliferative capacity of the more mature cells of the colony can be used as a surrogate for determining the underlying cancer stem cells that seeded the colony. This capacity can be assessed by measuring the uptake of labels that are incorporated into proliferating cells, *e.g.,* 3H-thymidine or sodium 3'-{1-[(phenylamino)-carbonyl]-3,4-tetrazolium}-bis(4-metboxy-6-nitro) benzene-sulfonic acid hydrate (XTT).

In other embodiments, spheres are measured to determine the amount of cancer stem cells in a sample (e.g., cancer stem cells form three-dimensional clusters of cells, called spheres) in appropriate media that is conducive to forming spheres. Spheres can be quantitated to provide a measure of cancer stem cells. *See* Singh et al., "Identification of a Cancer Stem Cell from Human Brain Tumors," Cancer Res 63: 5821-5828 (2003). Secondary spheres can also be measured. Secondary spheres are generated when the spheres that form from the patient sample are broken apart, and then allowed to reform.

In other embodiments, the number or amount of cancer stem cells in a sample can be determined in a cobblestone assay. Cancer stem cells from certain hematological cancers form "cobblestone areas" (CAs) when added to a culture containing a monolayer of bone marrow stromal cells. For instance, the amount of cancer stem cells from a leukemia sample can be assessed by this technique. The tumor samples are added to the monolayer of bone marrow stromal cells. The leukemia cancer stem cells, more so than the bulk leukemia cells, have the ability to migrate under the stromal layer and seed the formation of a colony of cells which can be seen visually under phase contrast microscopy in approximately 10-14 days as CAs. The number of CAs in the culture is a reflection of the leukemia cancer stem cell content of the tumor sample, and is considered a surrogate measure of the amount of stem cells capable of engrafting the bone marrow of immunodeficient mice. This assay can also be modified so that the CAs can be quantitated using biochemical labels of proliferating cells instead of manual counting, in order to increase the throughput of the assay. Chung et al., "Enforced expression of an Flt3 internal tandem duplication in human CD34+ cells confers properties of self-renewal and enhanced erythropoiesis." Blood 105(1):77-84 (2005).

In other embodiments, a sample (*e.g.,* a tumor sample, blood sample or bone marrow sample) obtained from the patient is analyzed in *in vivo* systems to determine the cancer stem cell population. In certain embodiments, for example, *in* vivo engraftment is used to quantitate the number of cancer stem cells in a sample. *In vivo* engraftment is the formation of tumors in an animal such as in immunocompromised or immunodeficient mice (such as NOD/SCID mice). Typically, the patient sample is cultured or manipulated in vitro and then injected into the mice. In these assays, mice can be injected with decreasing numbers of cells from patient samples, and the frequency of tumor formation can be plotted vs. the number of cells injected to determine the frequency of the cancer stem cells in the sample. Alternatively, the rate of growth of the resulting tumor can be measured, with larger or more rapidly advancing tumors indicating a higher cancer stem cell frequency in the patient sample.

### 6.5. METHODS OF MONITORING CANCER CELLS

As part of the prophylactically effective regimens and/or therapeutically effective regimens of the invention, the number or amount of cancer cells (alone or in combination with the number or amount of cancer stem cells) can be monitored/assessed using standard techniques known to one of skill in the art. In certain embodiments of the prophylactically effective regimens and/or therapeutically effective regimens of the invention, the regimens result in a stabilization or reduction in the number or amount (expressed, e.g., as a percentage) of cancer cells in the subject. In one embodiment, the subject undergoing the regimen is monitored to determine whether the regimen has resulted in a stabilization or reduction in the number or amount (expressed, e.g., as a percentage) of cancer cells in the subject.

In some embodiments, the number or amount of cancer cells is assessed in a subject using techniques described herein or known to one of skill in the art. In other embodiments, the number or amount of cancer cells is detected in a sample. Such samples include, but are not limited to, biological samples and samples derived from a biological sample- In certain embodiments, in addition to the biological sample itself or in addition to material derived from the biological sample such as cells, the sample used in the methods of this invention comprises added water, salts, glycerin, glucose, an antimicrobial agent, paraffin, a chemical stabilizing agent, heparin, an anticoagulant, or a buffering agent. In certain embodiments, the biological sample is blood, serum, urine, bone marrow or interstitial fluid. In another embodiment, the sample is a tissue sample. In a particular embodiment, the tissue sample is breast, colon, lung, liver, ovarian, pancreatic, prostate, renal, bone or skin tissue. In a specific embodiment, the tissue sample is a biopsy, including a tumor biopsy. The amount of biological sample taken from the subject will vary according to the type of biological sample and the method of detection to be employed. In a particular embodiment, the biological sample is blood, serum, or urine and the amount of blood, serum, or urine taken from the subject is 0.1 ml, 0.5 ml, 1 ml, 5 ml, 10 ml or more. In another embodiment, the biological sample is a tissue and the amount of tissue taken from the subject is less than 10 milligrams, less than 25 milligrams, less than 50 milligrams, less than 1 gram, less than 5 grams, less than 10 grams, less than 50 grams, or less than 100 grams.

In accordance with the methods of the invention, a sample derived from a biological sample is one in which the biological sample has been subjected to one or more pretreatment steps prior to the detection and/or measurement of the cancer cell population in the sample. In certain embodiments, a biological fluid is pretreated by centrifugation, filtration, precipitation, dialysis, or chromatography, or by a combination of such pretreatment steps. In other embodiments, a tissue sample is pretreated by freezing, chemical fixation, paraffin embedding, dehydration, permeablization, or homogenization followed by centrifugation, filtration, precipitation, dialysis, or chromatography, or by a combination of such pretreatment steps. In certain embodiments, the sample is pretreated by removing cells other than cancer cells from the sample, or removing debris from the sample prior to the determination of the number, amount or relative amount of cancer cells in the sample according to the methods of the invention.

The samples for use in the methods of this invention may be taken from any animal subject, preferably mammal, most preferably a human. The subject from which a sample is obtained and utilized in accordance with the methods of this invention includes, without limitation, an asymptomatic subject, a subject manifesting or exhibiting 1, 2, 3, 4 or more symptoms of cancer, a subject clinically diagnosed as having cancer, a subject predisposed to cancer, a subject suspected of having cancer, a subject undergoing therapy for cancer, a subject that has been medically determined to be free of cancer (e.g., following therapy for the cancer), a subject that is managing cancer, or a subject that has not been diagnosed with cancer.

In certain embodiments, the number or amount of cancer cells is assessed in a subject or a sample from a subject at least 1, 2, 4, 6, 8, 10, 12, 14, 15, 16, 18, 20, or 30, 60, 90 days 6 months, 9 months, 12 months, > 12 months after the subject begins receiving the regimen. In certain embodiments, the number or amount of cancer cells is assessed after a number of doses (e.g., after 1, 2, 5, 10, 20, 30 or more doses of a therapy). In other embodiments, the number or amount of cancer cells is assessed after 2 weeks, 1 month, 2 months, 1 year, 2 years, 3 years, 4 years or more after receiving one or more therapies.

The number or amount of cancer cells in a sample can be expressed as the percentage of, *e.g.,* overall cells in the sample. In some embodiments, the sample is a blood sample or bone marrow sample, wherein the number or amount of cancer cells per unit of volume (e.g., 1 mL) or other measured unit (*e.g.,* per unit field in the case of a histological analysis) is quantitated. The cancer cell population, in certain embodiments, can be determined as a percentage of the total blood cells.

In other embodiments, the sample from the patient is a tissue sample (*e.g.,* a biopsy from a subject with or suspected or having cancerous tissue), where the number or amount of cancer cells can be measured, for example, by immunohistochemistry or on the basis of the number or amount of cancer cells per unit weight of the tissue.

The number or amount of cancer cells in the test sample can be compared with the number or amount of cancer cells measured in a reference sample(s) to assess the efficacy of the regimen. In one embodiment, the reference sample is a sample from the subject undergoing therapy, at an earlier time point (e.g., prior to receiving the regimen as a baseline reference sample, or at an earlier time point while receiving the therapy). In this embodiment, the therapy desirably results in a decrease in the number or amount of cancer cells in the test sample as compared with the reference sample. In another embodiment, the reference sample is obtained from a healthy, cancer-free subject, or from a patient that is in remission for the same type of cancer. In this embodiment, the therapy desirably results in the test sample having an equal number or amount of cancer cells as detected in the reference sample (*e.g.,* no detectable cancer cells).

If the reduction in the number or amount of cancer cells is judged too small, then the medical practitioner has a number of options to adjust the regimen. For instance, the medical practitioner can then either increase the dosage of the therapy administered, the frequency of the administration, the duration of administration, combine the therapy with another therapy(ies), halt the therapy, or any combination thereof.

The number or amount of cancer cells can be monitored/assessed using standard techniques known to one of skill in the art. Cancer cells can be monitored by, *e.g.,* obtaining a sample, such as a tumor sample, blood sample or bone marrow sample, from a subject and detecting cancer cells in the sample. The number or amount of cancer cells in a sample (which may be expressed as a percentage) can be assessed by detecting the expression of antigens on cancer cells and/or by detecting the proliferation of cancer cells. Techniques known to those of skilled in the art can be used for measuring these activities. For example, cellular proliferation can be assayed by 3H-thymidine incorporation assays and trypan blue cell counts. Antigen expression can be assayed, for example, by immunoassays including, but are not limited to western blots, immunohistochemistry radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, flow cytometry, fluorescence-activated cell sorter (FACS) analysis and immunofluorescence.

The number or amount of cancer cells can be compared to a predetermined reference range and/or an earlier number or amount of cancer cells determined for the subject to gauge the subject's response to the regimens described herein. In a specific embodiment, a reduction in the number or amount of cancer cells relative to a predetermined reference range and/or earlier cancer cell number or amount determined for the subject indicate an improvement in the subject's prognosis or response to a therapy, whereas an increase relative to the predetermined reference range and/or earlier cancer cell numbers indicates the same or worse prognosis, or failure to respond to a therapy. In certain embodiments, the dosage, frequency and/or duration of administration of a therapy is modified as a result of the change in the relative amount of cancer cells.

In some embodiments, the cancer cell population can be monitored/assessed using gross measurements of the cancer cell population. For example, in some embodiments, the cancer cell population is determined using imaging methods such as computed tomography (CT), magnetic resonance imaging (MRI), ultrasound, X-ray imaging, mammograph imaging, radionuclide imaging, PET scan, detection of photons or bone scans.

In embodiments of the invention comprising treatment of solid tumors, the bulk size of the tumor or tumor burden may provide an estimate of the cancer cell population. A number of known methods can be used to assess the bulk size of the tumor or tumor burden of a patient. Non-limiting examples of such methods include imaging methods (e.g., computed tomography (CT), magnetic resonance imaging (MRI), PET scans, ultrasound, X ray imaging, mammograph imaging, bone scans, detection of photons and radioisotope imaging), visual methods (e.g., colonoscopy, bronchoscopy, endoscopy), physical examination *(e.g.,* prostate examination, breast examination, lymph nodes examination, abdominal examination, general palpation), blood tests (e.g., complete blood count (CBC) test, blood chemistries including liver function tests, prostate specific antigen (PSA) test, carcinoembryonic antigen (CEA) test, cancer antigen (CA)-125-test, alpha-fetoprotein (AFP)), bone marrow analyses (e.g., in cases of hematological malignancies), histopathology, cytology and flow cytometry.

In some embodiments, the bulk tumor size or tumor burden can be measured by assessments based on the size of tumor lesions determined from imaging methods. In specific embodiments, the assessments are performed in accordance with the Response Evaluation Criteria In Solid Tumors (RECIST) Guidelines, which are set forth in Therasse, P. et al., "New Guidelines to Evaluate the Response to Treatment in Solid Tumors," J. of the Nat. Canc. Inst. 92(3), 205-216 (2000). For instance, in specific embodiments, lesions in the subject that are representative of bulk tumor size are selected so that they are at least =20 mm in their longest diameter at baseline (prior to treatment) when conventional imaging techniques are used (*e.g.,* conventional CT scan, MRI or x-ray) and lesions that are at least =10 mm in their longest diameter at baseline should be selected when spiral CT scanning is used.

### 6.6. METHODS OF MONITORING LYMPHOCYTE CELL COUNT & NEUTROPHIL CELL COUNT

As part of the prophylactically effective regimens and/or therapeutically effective regimens of the invention, the peripheral blood lymphocyte counts can be monitered/assessed using standard techniques known to one of skill in the art. Peripheral blood lymphocytes counts in a subject can be determined by, e.g., obtaining a sample of peripheral blood from said subject, separating the lymphocytes from other components of peripheral blood such as plasma using e.g., Ficoll-Hypaque (Pharmacia) gradient centrifugation, and counting the lymphocytes using trypan blue. Peripheral blood T-cell counts in subject can be determined by, e.g., separating the lymphocytes from other components of peripheral blood such as plasma using, e.g., a use of Ficoll-Hypaque (Pharmacia) gradient centrifugation. Labeling the T-cells with an antibody directed to a T-cell antigen such as CD3, CD4, and CD8 which is conjugated to a FACS detectable agent, such as FITC or phycoerythrin, and measuring the number of T-cells by FACS. Further, the effect on a particular subset of T cells (e.g., CD2⁺, CD4⁺, CD8⁺, CD4⁺RO⁺, CD4⁺RA⁺, or CD8⁺RA⁺) or NK cells can be determined using standard techniques known to one of skill in the art such as FACS.

The subject's absolute neutrophil count (ANC) can be monitored/assessed using standard techniques known to one of skill in the art. In some embodiments, the regimen includes monitoring the patient's ANC in order to avoid the risk of the patient developing neutropenia.

The ANC can be calculated from measurements of the total number of white blood cells (WBC) and the numbers of neutrophils and bands (immature neutrophils). The ANC can be determined manually by trained medical technologists or by automated ANC results obtained from automated hematology analyzers.

### 6.7. BIOLOGICAL ASSAYS

### 6.7.1. IN VITRO ASSAYS

The compounds, pharmaceutical compositions and regimens of the invention can be tested in *vitro* and/or *in vivo* for their ability to reduce the number of cancer stem cells and/or cancer cells, or inhibit their proliferation. The ability of a compound or a regimen of the invention to reduce the number of cancer stem cells, cancer cells and/or immune cells (*e.g.,* lymphocytes) or inhibit their proliferation can be assessed by: detecting the expression of antigens on cancer stern cells, cancer cells, and immune cells; detecting the proliferation cancer stem cells, cancer cells and immune cells; detecting the effector function of cancer stem cells and cancer cells. Techniques known to those of skilled in the art can be used for measuring these activities. For example, cellular proliferation can be assayed by ³H-thymidine incorporation assays and trypan blue cell counts. Antigen expression can be assayed, for example, by immunoassays including, but are not limited to, competitive and non-competitive assay systems using techniques such as western blots, immunohistochemistry radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, flow cytometry and FACS analysis.

A compound, pharmaceutical composition, or regimen of the invention is preferably tested *in vitro* and then *in vivo* for the desired therapeutic or prophylactic activity prior to use in humans. For example, assays which can be used to determine whether administration of a specific compound is indicated include cell culture assays in which a patient tissue sample (e.g., a cancer stem cell or cancer cell) is grown in culture and exposed to, or otherwise contacted with, a compound of the invention, and the effect of such compound upon the tissue sample is observed. The tissue sample can be obtained by biopsy from the patient. This test allows the identification of the therapeutically most effective therapy (*e.g.,* prophylactic or therapeutic agent) for each individual patient.

In some embodiments, the effect of a compound, pharmaceutical composition, or regimen of the invention is assessed in a cell viability assay. In a specific embodiment, the determination of cell viability is assessed using the XTT assay.

By way of illustration, the effect of a compound of the invention can be determined on CD34⁺ cells which may be derived, for example, from normal stem cells (which can be isolated from human cord blood) or a leukemia cell line, such as HL-60. CD34⁺ cells are isolated using magnetic beads coated with anti-CD34 antibody. Isolated cells are then counted and aliquoted into 96-well plates and then incubated in the presence of varying concentrations of the test compound. Cell viability is measured by the addition of the XTT colorimetric reagent. Viability is determined by the absorbance of treated cultures at approximately 450-500 nm compared to untreated cultures. This assay can also be used to determine the time course of cell killing by various compounds by performing the XTT assay on cultures that are incubated with the compounds for varying periods of time.

In some embodiments, the effect of a compound, pharmaceutical composition, or regimen of the invention is assessed in a cobblestone area-forming cell (CAFC) assay. The cobblestone area-forming cell (CAFC) assay exploits a reproducible visual end point for the quantitation of cancer stem cells.

By way of illustration, leukemia samples are added to adherent cultures of stromal cells, which in some embodiments are MS-5 stromal cells. The cancer stem cells in the culture will migrate below the MS-5 stromal cells and form a colony of cells called a cobblestone that can be visual quantitated. To test the effect of a test compound on the cancer stem cell population using this assay, cells are first cultured in the presence of the compound. In some embodiements the cells are cultured for 16 hours. After this incubation, the cells are added to the stromal cultures. A reduction in the cobblestone area formation in cultures that were treated with the test compound compared to the untreated cells represents the cancer stem cell activity for the drug.

In some embodiments, the effects of a compound, pharmaceutical composition, or regimen of the invention is assessed in a neurosphere assay. The neurosphere assay exploits a reproducible visual end point for the quantitation of cancer stem cells.

By way of illustration, brain tumor specimens are dissociated and added to media optimized for the growth of neural tumor neurospheres. See, *e.g.,* Galli et al, Cancer Res 2004, 64:7011-21. The efficiency of sphere growth is assayed after 2 to 20 days in culture.

### 6.7.2. IN VIVO ASSAYS

The compounds, pharmaceutical compositions, and regimens of the invention can be tested in suitable animal model systems prior to use in humans. Such animal model systems include, but are not limited to, rats, mice, chicken, cows, monkeys, pigs, dogs, rabbits, etc. Any animal system well-known in the art may be used. Several aspects of the procedure may vary; said aspects include, but are not limited to, the temporal regime of administering the therapeutic modalities (*e*.*g*., prophylactic and/or therapeutic agents), whether such therapeutic modalities are administered separately or as an admixture, and the frequency of administration of the therapeutic modalities.

Animal models for cancer can be used to assess the efficacy of a compound or a combination therapy of the invention. Examples of animal models for lung cancer include, but are not limited to, lung cancer animal models described by Zhang & Roth (1994, In Vivo 8(5):755-69) and a transgenic mouse model with disrupted p53 function (*see, e.g.,* Morris et al. J. La. State Med. Soc. 1998, 150(4):179-85). An example of an animal model for breast cancer includes, but is not limited to, a transgenic mouse that overexpresses cyclin D1 (*see, e.g.,* Hosokawa et al., Transgenic Res. 2001, 10(5), 471-8. An example of an animal model for colon cancer includes, but is not limited to, a TCR b and p53 double knockout mouse (*see, e.g.,* Kado et al., Cancer Res. 2001, 61(6):2395-8). Examples of animal models for pancreatic cancer include, but are not limited to, a metastatic model of PancO2 murine pancreatic adenocarcinoma (*see, e.g.,* Wang et al., Int. J. Pancreatol. 2001, 29(1):37-46) and nu-nu mice generated in subcutaneous pancreatic tumours (*see, e.g.,* Ghaneh et al., Gene Ther. 2001, 8(3):199-208). Examples of animal models for non-Hodgkin's lymphoma include, but are not limited to, a severe combined immunodeficiency ("SCID") mouse (*see, e.g.,* Bryant et al., Lab Invest. 2000, 80(4), 553-73) and an JgHmu-HOX11 transgenic mouse (*see, e.g.,* Hough et al., Proc. Natl. Acad. Sci. USA 1998, 95(23), 13853-8. An example of an animal model for esophageal cancer includes, but is not limited to, a mouse transgenic for the human papillomavirus type 16 E7 oncogene (*see, e.g.,* Herber et al., J. Virol. 1996, 70(3):1873-81). Examples of animal models for colorectal carcinomas include, but are not limited to, Apc mouse models (*see, e*.*g*. Fodde & Smits, Trends Mol. Med. 2001, 7(8):369-73 and Kuraguchi et al., Oncogene 2000, 19(50), 5755-63).

In other embodiments, a sample (*e.g*., a tumor sample, blood sample or bone marrow sample) obtained from the patient is analyzed in *in vivo* systems to determine the cancer stem cell population. In certain embodiments, for example, *in vivo* engraftment is used to quantitate the number of cancer stem cells in a sample. *In vivo* engraftment is the formation of tumors in an animal such as in immunocompromised or immunodeficient mice (such as NOD/SCID mice). Typically, the patient sample is cultured or manipulated ***in*** vitro and then injected into the mice. In these assays, mice can be injected with decreasing numbers of cells from patient samples, and the frequency of tumor formation can be plotted vs. the number of cells injected to determine the frequency of the cancer stem cells in the sample. Alternatively, the rate of growth of the resulting tumor can be measured, with larger or more rapidly advancing tumors indicating a higher cancer stem cell frequency in the patient sample.

In some embodiments of the invention, the efficacy of the therapeutic regimen in reducing the number of cancer stem cells in animals (including humans) undergoing treatment can be evaluated using *in vivo* techniques. In these embodiments, an imaging agent is used which binds to biological moieties on cancer stem cells, *e.g*., cancer stem cell surface antigens or receptors. For instance, a fluorescent tag, photon emitting agent or radionuclide is covalently attached to an antibody or ligand that specifically binds to a cancer stem cell surface antigen or receptor. Section 6.4, *supra*, lists certain cancer stem cell surface antigens. The medical practitioner can infuse the labelled antibody or ligand into the patient either untreated or undergoing treatment, and then the practitioner can place the patient into a total body scanner/developer which can detect the attached label (*e.g*., fluorescent tag, photon emitting agent or radionuclide). The scanner/developer (*e.g*., CT or MRI scanner) records the presence, bodily location, and/or quantity of the bound antibody or ligand. In this manner, the mapping and quantitation of tag (*e.g*., fluorescence, radioactivity) in patterns (*i.e.*, different from patterns of normal stem cells within a tissue) within a tissue or tissues indicates the treatment efficacy within the patient's body. For example, a large signal (relative to a reference range or a prior treatment date) at a particular location indicates the presence of cancer stem cells. If this signal is increased relative to a prior treatment date it suggests a worsening of the disease and failure of therapy. Alternatively, a signal decrease indicates that therapy is working.

Similarly, in some embodiments of the invention, the efficacy of the therapeutic regimen in reducing the number of cancer cells in animals (including humans) undergoing treatment can be evaluated using *in vivo* techniques. In one embodiment, the medical practitioner performs the imaging technique with labelled molecule that specifically binds the surface of a cancer cell, *e*.*g*., a cancer cell surface antigen. See Section 6.4, *supra*, lists certain cancer cell surface antigens. In this manner, the mapping and quantitation of tag (*e.g*., fluorescence, radioactivity) in patterns within a tissue or tissues indicates the treatment efficacy within the body of the patient undergoing treatment.

Further, any assays known to those skilled in the art can be used to evaluate the prophylactic and/or therapeutic utility of a compound or pharmaceutical composition disclosed herein for cancer or one or more symptoms thereof.

### 6.7.3. ASSESSING TOXICITY

The toxicity and/or efficacy of compounds, pharmaceutical compositions, and regimens of the invention can be determined by standard pharmaceutical procedures in cell cultures or experimental animals, *e.g*., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Therapeutic regimens that exhibit large therapeutic indices are preferred. While therapeutic regimens that exhibit toxic side effects may be used, care should be taken to design a delivery system that targets such agents to the site of affected tissue in order to minimize potential damage to uninfected cells and, thereby, reduce side effects.

The data obtained from the cell culture assays and animal studies can be used in formulating a range of dosage of the therapies for use in humans. The dosage of such agents lies preferably within a range of circulating concentrations that include the ED₅₀ with little or no toxicity to normal tissues. The dosage may vary within this range depending upon the dosage form employed and the route of administration utilized. For any therapy used in the method of the invention, the therapeutically effective dose can be estimated initially from cell culture assays. A dose may be formulated in animal models to achieve a circulating plasma concentration range that includes the IC₅₀ (*i.e*., the concentration of the test compound that achieves a half-maximal inhibition of symptoms) as determined in cell culture. Such information can be used to more accurately determine useful doses in humans. Levels of compounds in plasma may be measured, for example, by high performance liquid chromatography.

### 6.8. ARTICLES OF MANUFACTURE

The present invention also encompasses a finished packaged and labeled pharmaceutical product. This article of manufacture includes the appropriate unit dosage form in an appropriate vessel or container such as a glass vial or other container that is hermetically sealed. The pharmaceutical product may contain, for example, a compound of the invention in a unit dosage form in a first container, and in a second container, sterile water for injection. Alternatively, the unit dosage form may be a solid suitable for oral, transdermal, intranasal, or topical delivery.

In a specific embodiment, the unit dosage form is suitable for intravenous, intramuscular, intranasal, oral, topical or subcutaneous delivery. Thus, the invention encompasses solutions, preferably sterile, suitable for each delivery route.

As with any pharmaceutical product, the packaging material and container are designed to protect the stability of the product during storage and shipment. Further, the products of the invention include instructions for use or other informational material that advise the physician, technician or patient on how to appropriately prevent or treat the disease or disorder in question. In other words, the article of manufacture includes instruction means indicating or suggesting a dosing regimen including, but not limited to, actual doses, monitoring procedures, cancer cell counts, cancer stem cell counts, and other monitoring information.

Specifically, the invention provides an article of manufacture comprising packaging material, such as a box, bottle, tube, vial, container, sprayer, insufflator, intravenous (i.v.) bag, envelope and the like; and at least one unit dosage form of a pharmaceutical agent contained within said packaging material, wherein said pharmaceutical agent comprises a compound of the invention, and wherein said packaging material includes instruction means which indicate that said compound can be used to prevent, manage, treat, and/or ameliorate one or more symptoms associated with cancer, or one or more symptoms thereof by administering specific doses and using specific dosing regimens as described herein.

In specific embodiments, the articles of manufacture include labelled antibodies that selectively or specifically bind to stem cells, and preferably, that selectively or specifically bind to cancer stem cells. As such, the article contains a method to adjust the dosages used in the therapeutic regimens, and to monitor the efficacy of the therapeutic regimen.

### 7. EXAMPLES

### 7.1. Example 1- Effects of Notch Activation and Notch Inhibition on Cancer Stem Cell Population in Medulloblastoma Cell Cultures

### ABSTRACT

Small subpopulations of stem-like cancer cells uniquely capable of supporting long-term neoplastic growth have been identified in both solid and hematopoetic tumors. Cancer stem cells, however, are relatively resistant to standard therapies, thus new strategies will be needed to remove this functionally critical cellular fraction in tumors. It was shown that cancer stem cells in the brain tumor medulloblastoma have elevated levels of Notch activity, and require this pathway for survival and proliferation. Activation of Notch2 in medulloblastoma cultures increased the cancer stem cell fraction as assessed by expression of CD133 and nestin, and by Hoechst dye exclusion (side population). In contrast, pharmacological inhibition ofNotch using gamma-secretase inhibitors depleted cancer stem cells via reduced proliferation, neuronal differentiation and Akt-dependent apoptosis. Notch blockade preferentially reduced the stem-like cell fraction, as viable populations of better-differentiated cells continued to slowly grow, but were unable to efficiently initiate soft agar colonies or tumor xenografts. Cancer stem cells in brain tumors thus appear selectively vulnerable to molecularly targeted agents inhibiting the Notch pathway.

### INTRODUCTION

The cancer stem cell (CSC) hypothesis postulates that not all neoplastic cells maintain long-term tumor growth, rather, a relatively small CSC subpopulation capable of ongoing self renewal is critical for tumor formation and expansion (1-4). The implications of the CSC hypothesis for the treatment of cancer patients are clear: only by targeting malignant stem cells can durable cures be achieved (2, 5). Two markers that identify stem-like cells in tumor specimens, CD133 and side population (SP), have been used to prospectively isolate a small percentage of cells in brain tumors uniquely able to generate xenografts (6-9).

The all-too-common recurrence of tumors that initially responded to therapy may be due to the persistence of CSC after treatment, as they appear to be resistant to many standard regimens (5, 10). Novel therapies that allow targeting CSC must be developed in order to prevent tumor recurrence in lesions perpetuated by this subpopulation. To date, no agent has been shown to target the CSC subpopulation in solid tumors. However, similarities in the growth characteristics and gene expression patterns of normal neural stem cells and brain tumor stem cells (11-13) suggest that the same signaling pathways may be required for survival and growth in both.

It has been previously shown that the Notch pathway, which plays an important role in neural stem cells, is also aberrantly activated in medulloblastoma (MB), the most common malignant brain tumor in children (14,15). Notch is known to promote the survival and proliferation of non-neoplastic neural stem cells, and to inhibit their differentiation (16, 17). Signaling is initiated by ligand binding, followed by intramembranous proteolytic cleavage of the Notch receptor by the gamma-secretase complex. Inhibitors of this complex block the growth of Notch-dependant tumors such as MB and T cell leukemia (14,15,19). Therefore, a gamma-secretase inhibitor (GSI) was used to investigate the role for Notch signaling in CSC. Here, it was demonstrated that survival, growth and differentiation of CSC in MB cultures are sensitive to Notch pathway blockade, and that pharmacological depletion of these stem-like cells blocks tumor initiation *in vitro* and *in vivo.* The data indicates that CSC may be especially vulnerable to attacks on molecular pathways such as Notch that are required in their non-neoplastic cognate cells.

### RESULTS

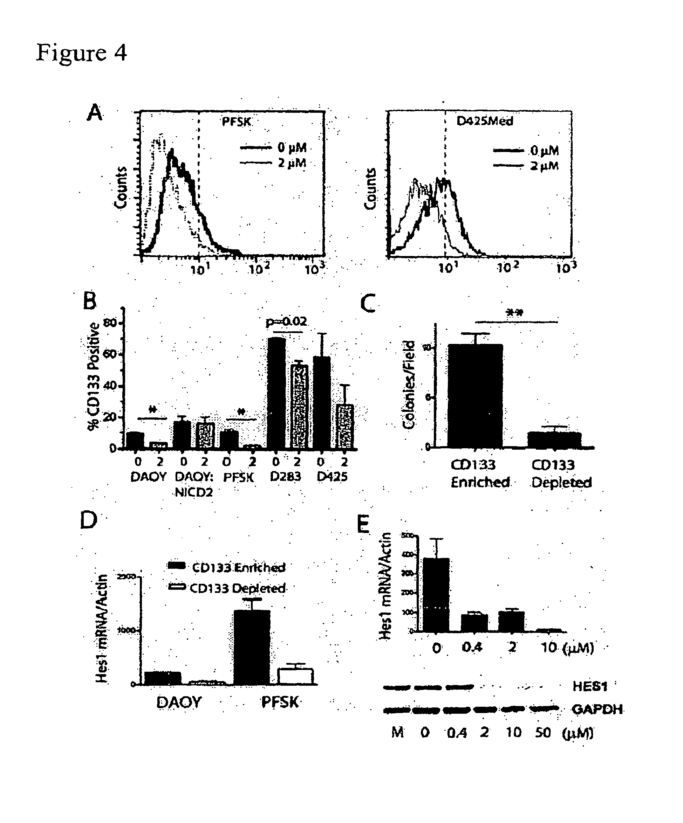

**Notch Regulates the Percentage of CSC in MB.** The effects of Notch on the CD133-positive CSC subpopulation were first examined. Singh and colleagues have shown that only CD133-positive MB and glioblastoma cells are able to form multipotent neurosphere clones or tumor xenografts (6, 12). Another type of brain tumor, ependymoma, also appears to contain a small fraction of CD133-positive cells uniquely capable of initiating tumor xenografts (9). In the adherent MB/primitive neuroectodermal tumor (PNET) cell lines DAOY and PFSK, flow cytometric analysis revealed that 9.9% and 10.8% of cells, respectively, expressed CD133 on their surface (Figures 4A, B), similar to the 6.1% to 45.4% (mean 19.7%) reported in primary MB (12). The D283Med and D425Med lines, which grow in suspension, contained higher percentages of CD133 positive cells. Sorting of DAOY cultures using magnetic beads coated with antibodies specific for CD133 generated an enriched population in which 30% of cells expressed the stem cell marker, and a negative population in which less than 1 % of cells were CD133-positive. CD133-enriched DAOY cultures formed 7-fold more small colonies after 4 weeks growth in soft agar than CD133-depleted ones did (Figure 4C), and 2-fold more large ones (data not shown), suggesting that as in primary human MB (6), tumor-initiating stem cells reside within the CD133-positive fraction. Interestingly, Hes1 mRNA, a marker of Notch pathway activity, was elevated 4-fold in the CD133-enriched fraction of DAOY, and 5-fold in the CD133-enriched fraction of PFSK (Figure 4D). Thus Notch signaling appears to be especially active in CSC, raising the possibility that inhibition of this pathway may target the CSC population.

Consistent with the hypothesis that Notch is important in CSC, gain or loss of pathway activity modulated the size of the CD133-expressing stem-like subpopulation (Figure 4B). This fraction was elevated to 17.2% in DAOY cells stably transfected with a plasmid encoding the constitutively active Notch2 intracellular domain (DAOY:NICD2). In contrast, Notch pathway inhibition using GSI-18, a potent sulfonamide GSI (21), reduced the CD133-positive stem-like fraction 3-fold to 3.3% (p < 0.01). DAOY:NICD2 cells expressing truncated Notch2 do not require gamma-secretase for activation of the receptor, and were insensitive to GSI-18, confirming the specificity of the pharmacological inhibition. The 4.8-fold reduction in the percentage of CD 133-positive cells seen in PFSK cultures was also statistically significant (p <0.01), while decreases in the D283 and D425 lines were less pronounced. 2 µM GSI-18 was used in these experiments, as both mRNA and protein levels of the Notch pathway target Hes1 are reduced over 70% after 48 hours by this concentration of the drug (Figure 4E). GSI-18 levels lower than 0.4 µM did not affect Hes1 mRNA levels (data not shown).

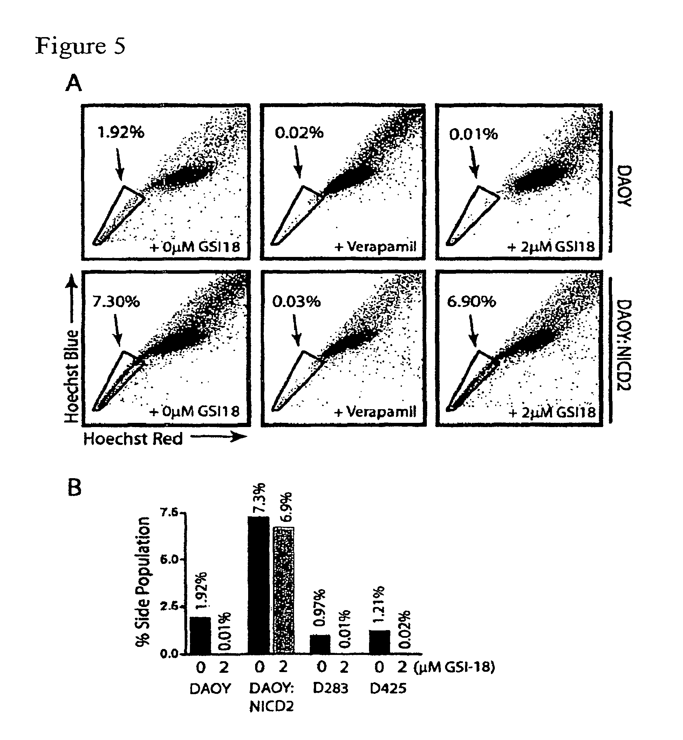

The ability to excrete Hoechst dye, which defines SP (also known as "side population") in flow cytometric analyses, is another marker of tumor-initiating CSC in the blood and brain (7, 8, 20). SP was therefore analyzed to obtain a second measure of the effects of Notch on CSC. DAOY cultures contained a Hoechst 33342-excreting SP of 1.9% (Figure 5A). The multiple drug resistance pumps mediating dye efflux are verapamil-sensitive (21), and adding verapamil to the MB culture abolished Hoechst excretion, verifying this was a bona fide SP. Consistent with the hypothesis that Notch activity regulates CSC, constitutive activation of Notch2 increased the SP almost 4-fold to 7.3%, while Notch inhibition essentially ablated it, with only 0.01% of remaining cells negative for dye (Figures 5A, B). NICD2 expression protected the SP from GSI-18 exposure, but not from verapamil, indicating that the effect of GSI-18 was specifically mediated through its effects on Notch signaling. Small side populations were also present in the D283Med and D425Med cell lines, and were ablated by both verapamil (data not shown) and 2 µM GSI-18 (Figure 5B). Thus Notch pathway blockade depletes the CSC subpopulation when either CD133 or SP is used to mark these cells.

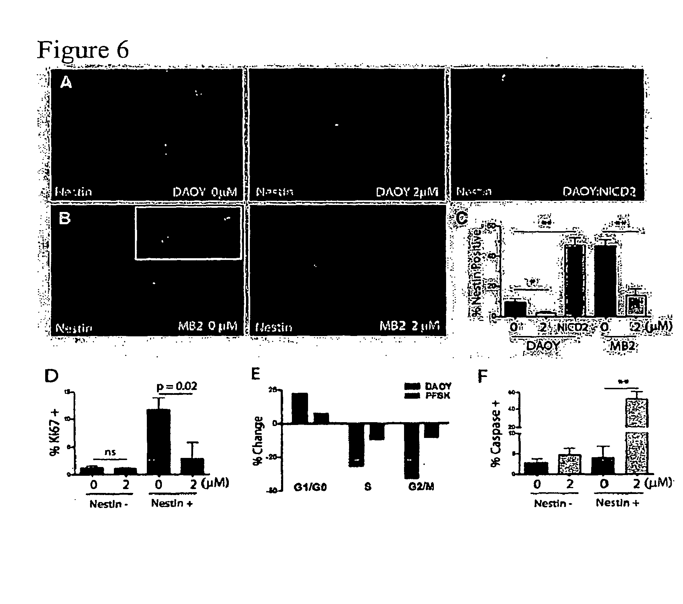

**Notch Blockade Suppresses Proliferation of Stem-like Cells.** The potential mechanisms by which CSC might be depleted following Notch pathway blockade were examined next, focusing initially on proliferation. Expression of the intermediate filament nestin was used to directly visualize stem-like cancer cells. Nestin was one of the first markers of neural stem cells to be identified (22), and its expression has been demonstrated in cells within neurospheres derived from primary human MB, suggesting it marks stem-like tumor cells (11, 12). Nestin staining was variable in intensity, with only 10% of DAOY cells expressing high levels, whereas almost half of MB2 cells were positive (Figures 6A, B). It was reasoned that MB cells expressing high levels of nestin might represent a stem-like subpopulation, and therefore correlated their percentage with varying levels of Notch activity. In DAOY cells, constitutive Notch2 activation unregulated the strongly nestn-positive fraction of cells almost 5-fold, to 47.7% (p<0.001), while Notch blockade using GSI-18 reduced this population 4-fold to 2.4% (p<0.01, Figure 6C). A significant reduction was also observed in MB2 cells following Notch blockade.

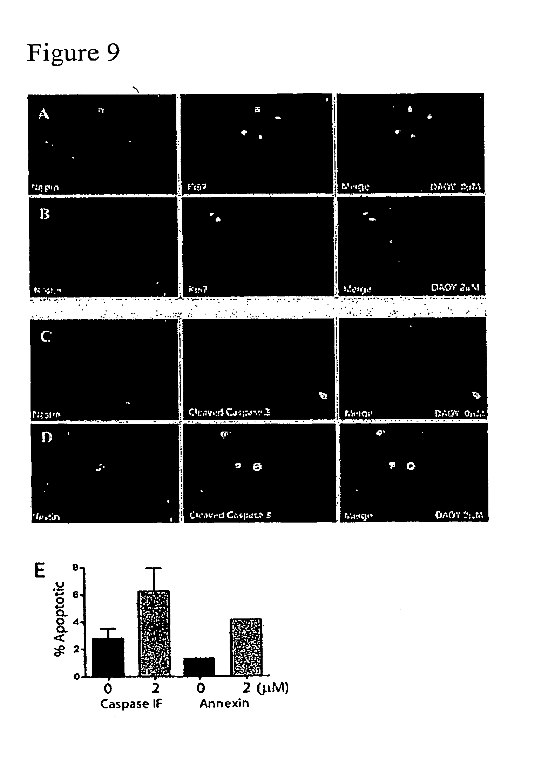

Double immunolabeling, using antibodies specific for nestin and for Ki67, a marker of proliferating cells, was used to determine effects of Notch pathway blockade on the cell cycle. The percentage of Ki67-positive cells was significantly higher in the nestin-positive subpopulation compared to the nestin-negative one, indicating the stem-like subpopulation was especially proliferative (Figure 6D and Supplementary Figure 9). While adult neural stem cells are generally thought to be relatively quiescent, during embryonic growth of the brain the stem cell compartment is highly proliferative. The high rate of division observed in stem-like MB cells may reflect their similarity to those in fetal germinal epithelium. A significant reduction in the proliferation ofnestin-positive DAOY cells was seen after 48 hours of Notch pathway inhibition with GSI-18, while the fraction of Ki67 positive cells changed only slightly in the nestin-negative subpopulation (Figure 6D). This suggests that the proliferation of stem-like cancer cells may be especially sensitive to Notch pathway inhibition. Flow cytometric analysis of the cell cycle in DAOY and PFSK cultures also supported a role for Notch in regulating proliferation, as 2 µM GSI-18 increased the G1/G4 cell fraction and decreased the S phase and G2/M fractions of both lines (Figure 6E).

**Apoptotic Induction and Neuronal Differentiation Following Notch Pathway Blockade.** The basal apoptotic rate was similar in both nestin-negative and nestin-positive DAOY cells, however, after 48 hours of treatment with 2 µM GSI-18, the apoptotic rate was increased over 10-fold in the putative CSC, while apoptotic induction in better-differentiated cells was less than 2-fold (Figures 6F and Figure 9). The assessment of overall apoptotic induction using cleaved caspase 3 corresponds relatively well to apoptotic induction measured by flow cytometric analysis of Annexin staining, suggesting that the immunofluoresecence-based counting procedure is accurate (Figure 9). Taken together, these data indicate the survival of stem-like tumor cells is more sensitive to Notch pathway inhibition than the survival of better-differentiated cells, and provides an additional mechanism for the depletion of CSC.

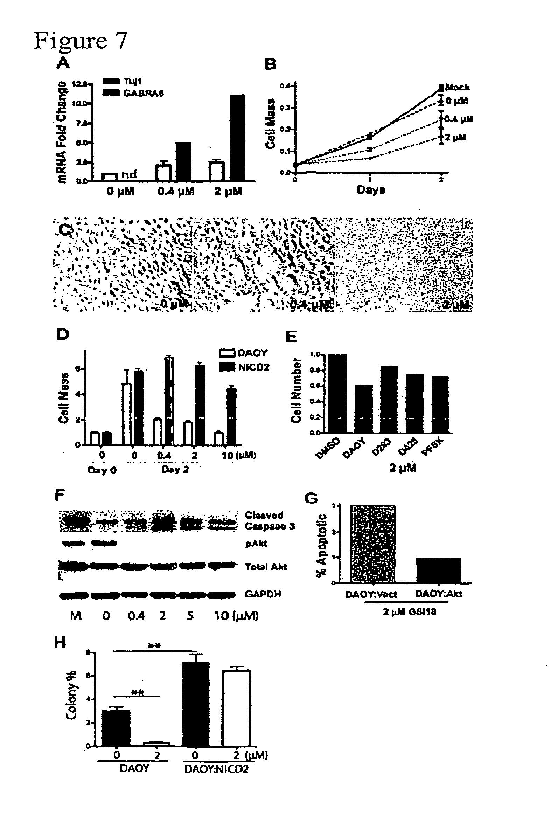

Notch pathway downregulation has been linked to cellular differentiation in both normal development and in neoplasms (23, 24), suggesting that cells surviving after Notch inhibition might be better-differentiated. It was found that gamma-secretase inhibition in DAOY cells increased RNA levels of two markers of cerebellar neuronal differentiation, Tuj1 and GABRA6, in a dose-dependant fashion (Figure 7A). One of these neuronal markers, the alpha6 subunit of the gamma-aninobutyric acid type A (GABRA6), is specifically expressed in the brain in cerebellar granule cells, where its expression is induced as they mature (25). Its induction after Notch pathway inhibition suggests the differentiation pathway being actuated resembles that in normal cerebellar granule neuron precursors, and highlights the similarities between the DAOY line and developing cerebellum.

**Growth of MB Cultures is Slowed, but not Arrested, by Notch Blockade.** Short-term increases in viable cell mass were reduced in a dose-dependent fashion by GSI-18 (Figures 7B, C), but many tumor cells survived Notch pathway inhibition and continued to proliferate over this period. NICD2 expression rescued growth, demonstrating that GSI-18 acts through Notch and not other pathways regulated by gamma-secretase (Figure 7D). Growth in the number of live cells in DAOY, D283Med, D425Med and PFSK cultures exposed to 2 µM GSI-18 were also reduced as compared to vehicle treated controls, but the reduction was less than that of cell mass (Figure 7E). The fact that cultures grew 2-fold or more during the same time period that side population was essentially ablated by Notch pathway blockade provides additional support for the notion that medulloblatoma CSC are especially vulnerable to Notch signaling inhibition.

**Notch-Dependant Tumor Growth is Mediated in Part by Akt.** It has previously been shown that Akt is a critical effector of Notch-regulated survival and growth outside the brain (26, 27). Therefore, it was examined whether Akt was also a mediator of the pro-survival and pro-growth effects of Notch in brain tumors. GSI-18 blocks the phosphorylation of Akt required for its activation at concentrations as low as 0.4 µM (Figure 7F). In addition, the basal apoptotic rate in MB cells stably transfected with constitutively active Akt was lower than in parental or vector transfected cells, and was not significantly increased by exposure to GSI (Figure 7G). GSI-18-mediated inhibition of cell growth was also partially rescued in two DAOY subclones stably transfected with constitutively active Akt (data not shown). Thus the pro-survival effects of Notch signaling in MB appear to be mediated at least in part by Akt.

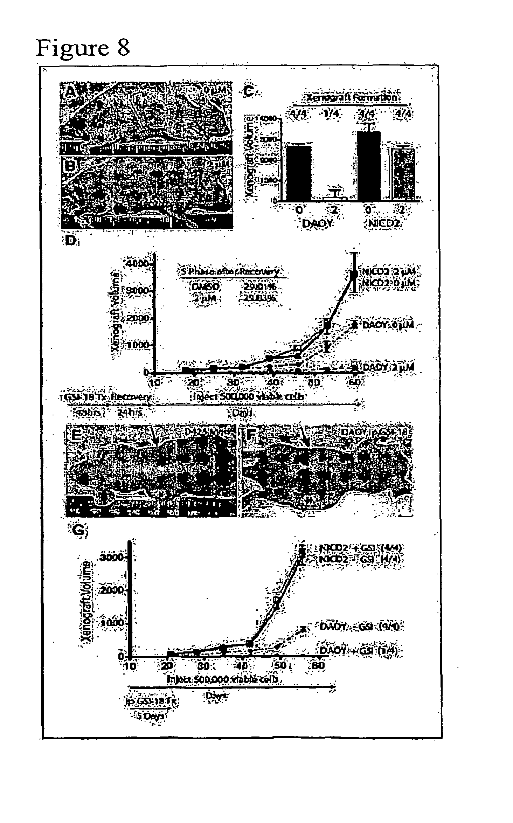

**Depletion of CSC By Gamma-Secretase Inhibition Blocks Tumor Initiation.** A clonogenic assay was used to determine whether cells capable of initiating anchorage-independent colonies were depleted by GSI-18. In vehicle-treated DAOY cultures, 2.9% of cells formed large colonies when seeded in soft agar (Figure 7H). This number dropped to 0.26% when an equal number ofviable cells were counted and seeded after 48 hours of treatment with GSI-18, and increased to 7.2% in the presence of constitutive Notch2 activation. Initiation of tumor xenografts was assayed next, a strategy used in several previous studies to examine the presence or absence of cancer stern cells (6-8). DAOY and DAOY:NICD2 cultures were treated with either 2 µM GSI-18 or vehicle alone, and the viable cells remaining after 48 hours counted using Trypan blue. 500,000 viable cells from each group were then injected subcutaneously in athymic (nude) mice. Large xenografts formed at the sites of all 12 control injections. In contrast, only one very small lesion formed among the four sites injected with cells that had been pretreated with 2 µM GSI-18, suggesting that while viable, they were no longer tumorigenic (Figures 8B, C). To rule out the possibility that cells were alive but moribund following gamma-secretase inhibition, the experiment was repeated with cells counted after a recovery period. After two days of treatment, followed by 24 hours of recovery in media containing 10% FBS and no gamma-secretase inhibitor, both GSI-18 and vehicle treated cells proliferated at similar rates, suggesting any overall effects on growth had normalized (Figure 8D). These "recovered" cells were still unable to form tumors when injected into mice if pretreated with GSI-18. D425Med cells also failed to engraft (n=2) after Notch pathway blockade, while vehicle treated cells always did (n=3; Figure 8E). These data strongly suggest that while viable and proliferative, MB cultures depleted of stem-like cells following Notch pathway blockade are no longer tumorigenic.

As a test of the requirement for ongoing Notch activity during tumor engraftment, animals were treated with GSI-18 after subcutaneously injecting DAOY and DAOY:NICD2 cells into their left and right flanks. A treatment group of four mice received intraperitoneal injections containing 0.5mg GSI-18 in DMSO for five consecutive days, beginning on the day of xenograft initiation, while the control group was treated with vehicle alone. DAOY cells failed to engraft in three of the four GSI-18 treated animals, while vehicle-treated animals all developed tumors (Figures 8F, G). Tumors also developed in both vehicle and GSI-18-treated mice at sites of DAOY:NICD2 injection. No behavioral or physical changes were noted in the animals treated with GSI-18.

### DISCUSSION

The CSC hypothesis postulates that a hierarchy exists within tumors. A relatively small population of stem-like cancer cells resides at the apex of this organization, and gives rise to a series of more numerous progeny cells which are better-differentiated and lack the ability to self-renew indefinitely. Recent reports indicate that such a hierarchy can persist in established cell lines derived from tumors of the brain or other organs (7,28). In this study, focus was on the Notch pathway, which promotes the specification, survival and growth of neural precursors in the cerebellum and elsewhere in the brain (16,17). It was found that Notch pathway inhibition depletes stem-like tumor cells, as demonstrated by the total loss of side population and significant reductions in the CD133 and nestin expressing cell fraction. Of these three markers, SP identified the smallest stem-like population, suggesting it might be a more specific marker of CSC. Interestingly, the SP fraction was also most dramatically reduced following Notch pathway blockade.

A hallmark finding is that Notch inhibition most potently affects stem-like cells, resulting in a still-viable culture depleted of CSC. This selective vulnerability may be due to the higher basal levels of Notch activity that were detected in the CSC subpopulation. Apoptosis was induced over 10-fold in nestin-positive cells by GSI-18, and less than 1-fold in nestin-negative ones. The suppression of proliferation was also more dramatic in the nestin-positive subpopulation. Most significantly, GSI-mediated CSC depletion occurred in the context of ongoing growth in overall viable tumor mass. Thus CSC are selectively targeted in a growing tumor by Notch pathway blockade.

The targeted depletion of stem-like cells observed following Notch pathway blockade contrasts sharply with prior reports of cancer stem cell survival following standard chemotherapies. Wulf and colleagues demonstrated higher efflux of chemotherapeutic agents from leukemic side population cells than from non-side population cells (20). Based on this, and on similar studies, it has been suggested that the efflux pumps which define side population also function to remove chemotherapy drugs from CSCs (reviewed in (5, 29)). In support of this concept, treatment of neuroblastoma cell lines with Mitoxantrone actually increases the side population, suggesting stern-like cancer cells are relatively resistant to this chemotherapeutic agent and accumulate as more differentiated cells are killed (30). In contrast, Notch blockade selectively depletes CSC, but leaves some better-differentiated cells capable of limited growth intact.

While MB/PNET cultures continued to grow in the face of Notch inhibition, the better-differentiated cells that remain viable are no longer able to form soft agar colonies or tumor xenografts, strongly suggesting that the ablated stem-like cell population is required for tumor initiation. The fact that overall culture growth was only slowed by Notch pathway blockade, while xenograft initiation was essentially ablated, illustrates how the short-term evaluation of tumor response might miss agents that selectively affect CSC but not the larger mass of better-differentiated tumor cells.

Only one type of malignant brain tumor was examined in this study, but these findings may be applicable to other neoplasms as well. The Notch pathway is known to regulate stem cells in a wide variety of tissues, and Notch blockade appears to affect survival and proliferation of multiple types of cancer (31, 32). For example, Notch is activated by translocation or mutation in over 50% of T cell ALL, and anti-Notch therapies have been shown to slow ALL growth in vitro (18). GSIs may therefore be useful in targeting CSCs in a wide range of neoplasms including T-ALL.

In summary, it was demonstrated that in the malignant brain tumor MB, Notch pathway blockade depletes a population of cells required for *in vivo* tumor initiation by suppressing proliferation and inducing apoptosis or differentiation in stem-like cells. The data suggest that certain Notch pathway blockers mayconstitute an efective new class of therapeutic oncology agents that target cancer stem cells.

### MATERIALS AND METHODS

### Cell culture

The DAOY, PFSK, D283 and D425 Med cell lines were obtained from the American Type Culture Collection. The low-passage MB line MB2 was derived from a tumor resected at Johns Hopkins Hospital. For treatment studies, cell were plated and allowed to grow overnight in MEM (Invitrogen, Carlsbad, CA) containing 10% FBS, then media was replaced the next morning with low serum (0.5% FBS) MEM containing GSI dissolved in DMSO. RNA and protein extractions, and all cell-based assays, were performed 48 hours after drug application unless otherwise noted. All experiments with error bars were performed in triplicate and shown as mean values with standard error unless otherwise noted. Cell mass was measured using CellTTTER assays according to the manufacturer's instructions (Promega, Madison, WI). Cell number and viability were assessed using the Guava PCA and Viacount reagent according to instructions (Guava Technologies, Heyward, CA). Soft agar assays were performed as previously described (15) and scored either manually after 4 weeks or using as automated reader after 6 weeks. The constitutively active Akt stable cell line was made by transfecting DAOY cells with myristolated Akt (pCB6-IMy-Akt-HA) or control (pCB6+) plasmids (kind gifts from Susan J. Baker) and selecting and maintaining clones in 0.8 mg/ml G418. Soft agar assays were performed as previously described, with colonies counted using an automated reader (15).

### GSI Synthesis

The potent GSI [11-*endo*]-*N*-(5,6,7,8,9,10-hexahydro-6,9-methanobenzo[α][8]annulen-11-yl)-thiophene-2-sulfonamide was listed as compound 18 in the recent report by Lewis *et al.* describing its synthesis and testing, and it is referred to as GSI-18 (19). It was synthesized as previously described (19), and its identity and quality were confirmed by NMR and mass spectral analysis. GSI-18 has the following structural formula:

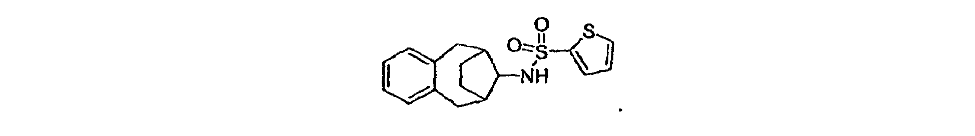

### Immunocytochemistry

The cell lines DAOY and MB2 were seeded in 12-well tissue culture plate and allowed to adhere overnight. After treatment with 2 µM GSI-18 or vehicle for 48 hours, they were washed and hybridized as previously described (15) using anti-Nestin antibody (1:1000, Chemicon, Temecula, CA), anti-Ki67 (1:1000, Novocastra, Newcastle-on-Tyne, UK), or anti-cleaved caspase 3 (1:500; Cell Signaling Technology, Beverly, MA) and Cy3-conjugated Goat-and-Mouse and Cy2-conjugated Goat-anti-Rabbit secondary antibodies (1:300, Jackson ImmunoResearch Laboratories, West Grove, PA). At least six high powered fields containing 150 to 350 cells were photographed and the percentage cells positive for nestin scored in a blinded fashion.

### Flow cytometric analyses

Flow cytometric analysis of S-phase fraction and cell cycle kinetics was performed using a FACSCalibur (Becton Dickinson, San Jose, CA) with CELL Quest, version 3.3 software (15). CD 133 studies were performed using the same instrument, with antibodies from Miltenyi (Miltenyi Biotec, Auburn, CA) according to the manufacturer's instructions. Cells expressing levels of CD133 higher than those seen in IgG controls were considered positive. For analyses of side population, cells (10⁶/ml) were incubated with Hoechst 33342 (3 µM, Molecular Probes, Carlsbad, CA) for 90 minutes at 37°C in DMEM containing 2% FBS. Prior to analysis, cells were washed and resuspended in 2 µg/ml PI (Sigma, St. Louis, MO). Cells were analyzed on a LSR flow cytometer equipped with 424/44 nm band pass and 670 nm long pass optical filters (Omega Optical, Brattleboro, VT). To ensure proper identification of SP cells, cells were incubated as above with the addition of 50 µM verapamil (Sigma).

### RNA and Protein Analyses

Quantitative RT PCR for Notch1, Notch2 and Hes1 was performed as previously described (29), with all reactions normalized to actin (Applied Biosystems, Foster City, CA). Commercially available Assay on Demand Taqman primers and probes were used to measure mRNA for Nestin, Bmi1, Tuj1 and GABRA6. Western blots contained 20 µg of protein per lane on a 10% Tris-glycine SDS-PAGE gel (Invitrogen). Blots were incubated overnight at 4 °C with antibodies directed against Hes1 (kind gift of Dr. Tetsuo Sudo, 1:2000) phospho-Akt (Cell Signaling, 1:1000), total Akt (Cell Signaling, 1:1000), Cleaved Caspase-3 (Cell Signaling, 1:1000) or GAPDH (Research Diagnostics, 1:20,000) and developed with enhanced chemiluminescence reagent (Pierce, Rockford, IL).

### Statistical Analysis

Statistical analyses were performed using GraphPad Prism 4 (GraphPad Software, San Diego, CA). Data graphed with error bars represent mean and standard error from experiments performed in triplicate unless otherwise noted. A two-sided Student's T test was used to determine the significance of any differences.

### 7.2. Example 2 - Notch pathway blockade inhibits glioblastoma growth by targeting cancer stern cells

### INTRODUCTION

There are more than 41,000 people diagnosed with primary brain tumors each year in the United States (33), and GBM are the most common malignant brain tumors in adults (34). More than 80% of GBM patients die within 1 year (33, 34). There is emerging evidence showing that a small population of cancer stem cells (CSCs) within neoplasms is responsible for long-term tumor propagation (35). Several groups also demonstrated that such stem-like cells exist in brain turnors, including GBMs (35-40). Two CSC markers, CD133 and side population, have been used to prospectively isolate a small percentage of cells in brain tumors uniquely able to generate tumor neurospheres and xenografts (36, 38, 39, 41). In addition, GBM propagated as neurosphere more accurately replicate the infiltrating growth patterns seen in primary tumors (40, 42).

Activation of the Notch signaling pathway has been shown to regulate stem cells in many different organs , including the CNS (43, 44). We and others have demonstrated that the Notch signaling pathway plays a very important role in the pathogenesis of MB and GBM (45-47), and that MB and GBM contain neural stem cell-like CSCs with higher Notch activity (40, 42, 48). We have recently demonstrated that Notch pathway blockade by gamma-secretase inhibitors (GSI) depletes CSCs in established MB cell lines through decreased proliferation and increased apoptosis (49). These results provide promising initial evidence that targeting the Notch signaling pathway could be an effective strategy to deplete CSCs in tumors. In the present study, we used GBM neurosphere lines as our model to examine the effects of Notch pathway blockade on CSCs in GBMs.

### RESULTS

It has been shown that the Notch signaling pathway regulates normal stem cells in the Central Neural System (CNS), and that GBMs contain cancer stem cells (CSCs) with higher Notch activity (Cancer Research, 64:7011-21, 2004; Cancer Cell, 9:391-403, 2006). However, it has not been known if Notch pathway blockade could target CSCs in GBM. Recent studies showed that GBM primary cultures propagated as neurosphere more accurately replicate the infiltrating growth patterns seen in primary tumors and contain stem-like cancer cells (Cancer Research, 64:7011-21, 2004). In the present study, we used GBM neurospheres to examine the effects of Notch pathway blockade in GBM. We found that Notch pathway blockade by 2 µM Gamma-Secretase Inhibitor (GSI-18) reduces expression of CD133 and inhibits tumor growth *in vitro.* Furthermore, overexpression of an active form of Notch2 (NICD2) increases tumor growth *in vitro.* When equal numbers of viable GBM neurosphere cells previously treated with either GSI-18 or vehicle (DMS-O) were injected subcutaneously into nude mice, DMSO treated cells always formed tumors whereas GSI-18 treated cells did not. Moreover, mice injected intracranially with GSI-18 treated GBM neurosphere cultures have longer survival compare with mice injected with DSMO treated cells. These results suggest that depleting CSCs by Notch pathway blockade inhibits tumor growth and propagation both in vitro and in vivo. Finally, we found a decrease in proliferation (Ki67 index) in the Nestin positive population. Increased expression of cleaved caspase 3 and reduced expression of phosphorelated Akt is also seen in GSI-18 treated GBM neurosphere lines, suggesting that Notch pathway inhibition depletes CSCs through Akt-mediated apoptosis. In summary, we demonstrate that Notch pathway blockade depletes stem-like cells in GBMs, suggesting that Gamma-secretase inhibitors can be used as chemotherapeutic reagents to target CSCs in malignant gliomas.

These results demonstrate that stem cell markers and Notch family genes are expressed in GBM cells, Notch Pathway blockade by gamma-secretase inhibitor reduces CD133 expression and inhibits tumor growth, whereas overexpression of active form of Notch2 (NICD2) increases tumor growth *in vitro,* that depleting CSCs by Notch pathway blockade inhibits tumor propagation *in vivo* and prolongs the survival in mice bearing intracranial xenografts, and that Notch pathway inhibition depletes CSCs through reducing proliferation and inducing Akt mediated apoptosis.

### REFERENCES

1. Park, C. H., Bergsagel, D. E. & McCulloch, E. A. (1971) JNatI Cancer Inst 46, 411-22.
2. Reya, T., Morrison, S. J., Clarke, M. F. & Weissman, L. L. (2001) Nature 414, 105-11.
3. Huntly, B. J. & Gilliland, D. G. (2005) Nat Rev Cancer 5,311-21.
4. Wang, J. C. & Dick, J. E. (2005) Trends Cell Biol.
5. Dean, M., Fojo, T. & Bates, S., (2005) Nat Rev Cancer 5, 275-84.
6. Singh S. K., Hawkins, C., Clarke, L. D., Squire, J. A., Bayani, J., Hide, T., Henkelman, R. M., Cusimano, M. D. & Dirks, P. B. (2004) Nature 432, 396-401.
7. Kondo, T., Setoguchi, T. & Taga, T. (2004) Proc Natl Acad Sci U S A 101, 781-6.
8. Patrawala, L., Calhoun, T., Schneider-Broussard, R., Zhou, J., Claypool, K. & Tang, D. G. (2005) Cancer Res 65, 6207-19.
9. Taylor, M. D., Poppleton, H., Fuller, C., Su, X., Liu, Y., Jensen, P., Magdaleno, S., Dalton, J., Calabrese, C., Board, J., Macdonald, T., Rutka, J., Goha, A., Gajjar, A., Curran, T. & Gilbertson, R. J. (2005) Cancer Cell 8, 323-335.
10. Costello, R. T., Mallet, F., Gangler, B., Sainty, D., Amoulet, C., Gastaut, J. A. & Olive, D. (2000) Cancer Res 60, 4403-1.
11. Hemmati, H. D., Nakano, I., Lazareff, J. A., Masterman-Smith, M., Geschwind, D. H., Brormer-Fraser, M. & Kornblum, H. I. (2003) Proc Natl Acad Sci USA 100, 15178-83.
12. Singh, S. K., Clarke, I. D., Terasaki, M., Bonn, V. E., Hawkins, C., Squire, J. & Dirks, P. B. (2003) Cancer Res 63, 5821-8.
13. Galli, R., Binda, E., Orfanelli, U., Cipeletti, B., Gritti, A., De Vitis, S., Fiocco, R., Foroni, C., Dimeco, F. & Vescovi, A. (2004) Cancer Res 64, 7011-21.
14. Hallahan, A. R., Pritchard, J. I., Hansen, S., Benson, M., Stoeck, J., Hatton, B. A., Russell, T. L., Ellenbogen, R. G., Bernstein, I. D., Beachy, P. A. & Olson, J. M- (2004) Cancer Res 64, 7794-800.
15. Fan, X., Mikolaenko, L, Elhassan, I., Ni, X., Wang, Y., Ball, D., Brat, D. J., Perry, A. & Eberhart, C. G. (2004) Cancer Res 64, 7787-93.
16. Solecki, D. J., Liu, X. L., Tomoda, T., Fang, Y. & Hatten, M. E. (2001) Neuron 31, 557-68.
17. Gaiano, N. & Fishell, G. (2002) Annu Rev Neurosci 25, 471-90.
18. Weng, A. P., Ferrando, A. A., Lee, W., Morris, J. P. t., Silverman, L. B., Sanchez-Irizarry, C., Blacklow, S. C, Look, A. T. & Aster, J. C. (2004) Science 306, 269-71
19. Lewis, S. J., Smith, A. L., Neduvelil, J. G., Stevenson, G. I., Lindon, M. J., Jones, A. B., Shearman, M. S., Beher, D., Clarke, E., Best, J. D., Peachey, J. E., Harrison, T., & Castro, J. L. (2005) Bioorg Med Chem Lett 15, 373-8.
20. Wulf, G. G., Wang, R. Y., Kuehnle, I., Weidner, D., Marini, F., Brenner, M. K., Andreeff, M. & Goodell, M. A., (2001) Blood 98, 1166-73.
21. Goodell, M, A., Brose, K., Paradis, G., Conner, A. S. & Mulligan, R. C. (1996) J. Exp Med 183, 1797-806.
22. Lendahl, U., Zinbnerman, L. B. & McKay, R D. (1990) Cell 60, 585-95.
23. Ishibashi, M., Ang, S. L., Shiota, K., Nakanishi, S., Kageyama, R & Guillemot, F. (1995) Genes Dev 9, 3136-48.
24. van Es, J. H., van Gijn, M. E., Riccio, O., van den Born, M., Vooijs, M., Begthel, H., Cozijnsen, M., Robine, S., Winton, D. J., Radtke, F. & Clevers, H. (2005) Nature 435, 959-63.
25. Kato, K. (1990) J Med Biol 214, 619-24.
26. Nair, P., Somasundaram, K. & Krishna, S. (2003) J. Virol 77, 7106-12.
27. Sade, H., Krishna, S. & Sarin, A. (2004) J Biol Chem 279, 2937-44.
28. Locke, M., Heywood, M., Fawell, S. & Mackenzie, I. C. (2005) Cuncer Res 65, 8944-50.
29. Donnenberg, V. S., & Donnenberg, A. D. (2005) J Clin Pharmacol 45, 872-7.
30. Hirschmann-Jax, C., Foster, A. E., Wulf, G. G., Nuchtern, J. G., Jax, T. W., Gobel, U., Goodell, M. A. & Brenner, M. K. (2004) Proc Natl Acad Sci USA 101,14228-33.
31. Weng, A. P. & Aster, J. C., (2004) Curr Opin Dev 14,48-54.
32. Radtke, F. & Raj, K. (2003) Nai Rev Cancer 3, 756-67.
33. Reardon, D. A., Rich, J. N., Friedman, H. S., and Bigner, D. D. Recent advances in the treatment of malignant astrocytoma. J Clin Oncol, 24: 1253-1265, 2006.
34. Kleihues, P. and Cavenee, W. K. Tumours of the Nervous System. Lyon (France): IARC Press, 2000.
35. Reya, T., Morrison, S. J., Clarke, M. F., and Weissman, I. L. Stem cells, cancer, and cancer stem cells. Nature, 414: 105-111, 2001.
36. Hemmati, H. D., Nakano, I., Lazareff, J. A., Masterman-Smith, M., Geschwind, D. H., Bronner-Fraser, M., and Kornblum, H. I. Cancerous stem cells can arise from pediatric brain tumors. Proc Natl Acad Sci U S A, 100: 15178-15183, 2003.
37. Sanai, N., Alvarez-Buylla, A., and Berger, M. S. Neural stem cells and the origin of gliomas. N Engl J Med, 353: 811-822, 2005.
38. Singh, S. K., Hawkins, C., Clarke, I. D., Squire, J. A., Bayani, J., Hide, T., Henkelman, R. M., Cusimano, M. D., and Dirks, P. B. Identification of human brain tumour initiating cells. Nature, 432: 396-401, 2004.
39. Kondo, T., Setoguchi, T., and Taga, T. Persistence of a small subpopulation of cancer stem-like cells in the C6 glioma cell line. Proc Natl Acad Sci U S A, 101: 781-786, 2004.
40. Galli, R., Binda, E., Orfanelli, U., Cipelletti, B., Gritti, A., De Vitis, S., Fiocco, R., Foroni, C., Dimeco, F., and Vescovi, A. Isolation and characterization of tumorigenic, stem-like neural precursors from human glioblastoma. Cancer Res, 64: 7011-7021, 2004.
41. Taylor, M. D., Poppleton, H., Fuller, C., Su, X., Liu, Y., Jensen, P., Magdaleno, S., Dalton, J., Calabrese, C., Board, J., Macdonald, T, Rutka, J., Guha, A., Gajjar, A-, Curran, T., and Gilbertson, R. J. Radial glia cells are candidate stem cells of ependymoma. Cancer Cell, 8: 323-335, 2005.
42. Lee, J., Kotliarova, S., Kotliarov, Y., Li, A., Su, Q., Donin, N. M., Pastorino, S., Purow, B. W., Christopher, N., Zhang, W., Park, J. K., and Fine, H. A. Tumor stem cells derived from glioblastoma cultured in bFGF and EGF more closely mirror the phenotype and genotype of primary tumors than do serum-cultured cell lines. Cancer Cell, 9: 391-403, 2006.
43. Shen, Q-, Goderie, S. K-, Jin, L-, Karanth, N., Sun, Y., Abramova, N., Vincent, P., Pumiglia, K., and Temple, S. Endothelial cells stimulate self-renewal and expand neurogenesis ofneural stem cells. Science, 304: 1338-1340, 2004.
44. Yoon, K. and Gaiano, N. Notch signaling in the mammalian central nervous system: insights from mouse mutants. Nat Neurosci, 8: 709-715, 2005.
45. Fan, X., Mikolaenko, I., Elhassan, I., Ni, X., Wang, Y., Ball, D., Brat, D. J., Perry, A., and Eberhart, C. G. Notch1 and notch2 have opposite effects on embryonal brain tumor growth. Cancer Res, 64: 7787-7793, 2004.
46. Purow, B. W., Haque, R. M., Noel, M. W., Su, Q., Burdick, M. J., Lee, J., Sundaresan, T., Pastorino, S., Park, J. K., Mikolaenko, I., Maric, D., Eberhart, C. G., and Fine, H. A. Expression of Notch-1 and its ligands, Delta-like-1 and Jagged-1, is critical for glioma cell survival and proliferation. Cancer Res, 65: 2353-2363, 2005.
47. Hallahan, A. R., Pritchard, J. I., Hansen, S., Benson, M., Stoeck, J., Hatton, B. A., Russell, T. L., Ellenbogen, R. G., Bernstein, I. D., Beachy, P. A., and Olson, J. M. The SmoA1 mouse model reveals that notch signaling is critical for the growth and survival of sonic hedgehog-induced medulloblastomas. Cancer Res, 64: 7794-7800,2004.

### EQUIVALENTS

The present invention is not to be limited in scope by the specific embodiments described which are intended as single illustrations of individual aspects of the invention, and functionally equivalent methods and components are within the scope of the invention. Indeed, various modifications of the invention, in addition to those shown and described herein, will become apparent to those skilled in the art from the foregoing description and accompanying drawings using no more than routine experimentation. Such modifications and equivalents are intended to fall within the scope of the appended claims.

All publications, patents and patent applications mentioned in this specification are herein incorporated by reference into the specification to the same extent as if each individual publication, patent or patent application was specifically and individually indicated to be incorporated herein by reference.

Citation or discussion of a reference herein shall not be construed as an admission that such is prior art to the present invention.

## Claims

1. Use of a compound for the manufacture of a medicament for treating cancer in a human patient, wherein the compound is any of the following: 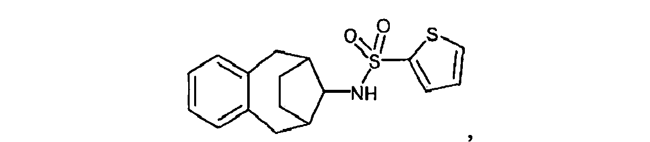 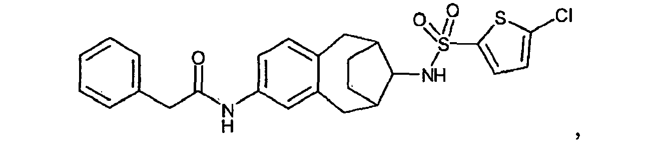 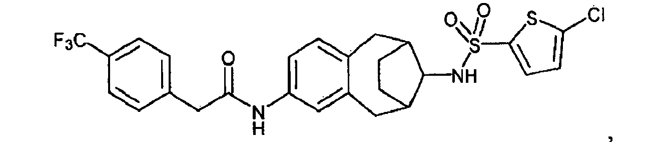 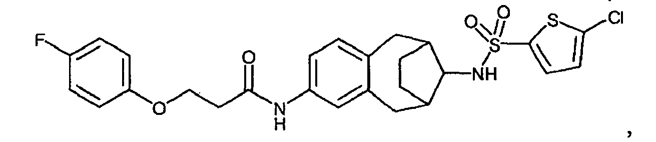 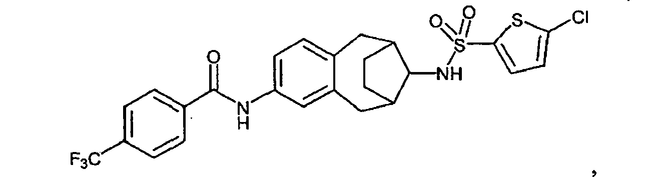 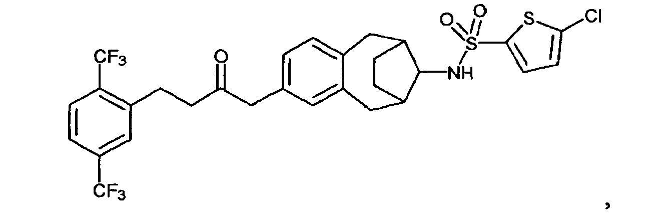 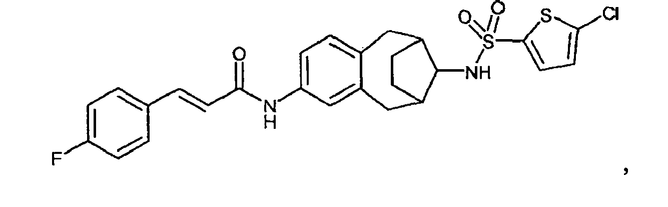 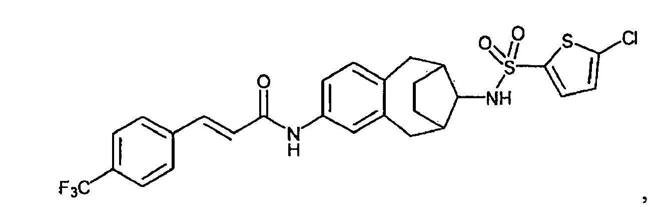 and 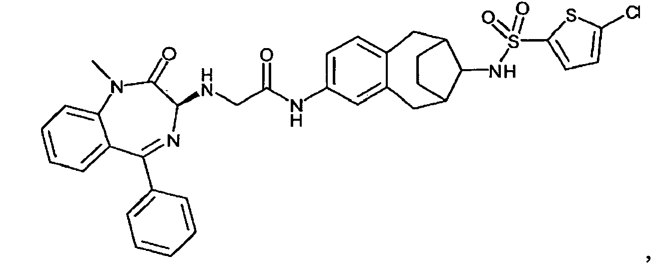 or a pharmaceutically acceptable salt thereof.

2. The use of claim 1, wherein the compound is 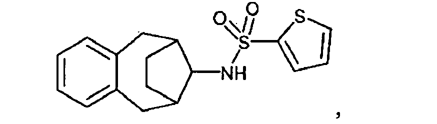 or a pharmaceutically acceptable salt thereof.

3. The use of claim 1, wherein the compound is administered to the human patient over a period of 1 day to 36 months.

4. The use of claim 1, wherein the compound is to be administered to the patient in a dose of 50 mg/kg or less.

5. The use of claim 1, wherein the cancer is pancreatic cancer, T-cell acute lymphoblastic leukemia, retinoblastoma, brain tumor, or breast cancer.

6. The use of claim 5, wherein the brain tumor is an embryonal brain tumor, a medulloblastoma, a PNET, an astrocytoma, a glioblastoma, an oligodendroglioma, an ependymoma, a choroids plexus tumor, or a meningioma.

7. The use of claim 1, wherein the cancer is a medullablastoma or a glioma.

8. The use of claim 1, wherein the compound is administered to a human patient in whom the cancer stem cell population is monitored.

9. The use of claim 8, wherein the cancer stem cell population is monitored by detecting the amount of cancer stem cells in a specimen from the human patient.
